(19) Europäisches Patentamt
European Patent Office
Office européen des brevets

(11) EP 2 906 662 B1

(12) **EUROPÄISCHE PATENTSCHRIFT**

(45) Veröffentlichungstag und Bekanntmachung des Hinweises auf die Patenterteilung:
**01.04.2020 Patentblatt 2020/14**

(21) Anmeldenummer: **13765649.2**

(22) Anmeldetag: **18.09.2013**

(51) Int Cl.:
***C09K 11/06*** (2006.01) ***H05B 33/10*** (2006.01)

(86) Internationale Anmeldenummer:
**PCT/EP2013/002806**

(87) Internationale Veröffentlichungsnummer:
**WO 2014/056573 (17.04.2014 Gazette 2014/16)**

(54) **EMITTER UND HOSTS MIT AROMATISCHEN EINHEITEN**

EMITTER AND HOSTS WITH AROMATIC UNITS

ÉMETTEURS ET HÔTES COMPORTANT DES MOTIFS AROMATIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.10.2012 EP 12007076**

(43) Veröffentlichungstag der Anmeldung:
**19.08.2015 Patentblatt 2015/34**

(73) Patentinhaber: **Merck Patent GmbH**
**64293 Darmstadt (DE)**

(72) Erfinder:
- **PAN, Junyou**
  **60320 Frankfurt am Main (DE)**
- **ENGEL, Martin**
  **64291 Darmstadt (DE)**
- **KOENEN, Nils**
  **64285 Darmstadt (DE)**
- **LUDEMANN, Aurélie**
  **60322 Frankfurt am Main (DE)**

(56) Entgegenhaltungen:
**EP-A1- 1 160 888**
**EP-A1- 1 223 209**
**WO-A2-2004/093207**
**WO-A2-2005/003253**
**JP-A- H03 162 484**
**US-A1- 2005 255 258**
**US-A1- 2010 308 313**

EP 2 906 662 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft organische Elektrolumineszenzvorrichtungen enthaltend aromatische, nicht kondensierte Emittermaterialien gemäß Anspruch 1 und eine Zusammensetzung gemäß Anspruch 8 sowie eine Formulierung gemäß Anspruch 9.

Der Aufbau organischer lichtemittierender Dioden (OLEDs), eine organischen Elektrolumineszenzvorrichtungen, in denen organische Halbleiter als funktionelle Materialien eingesetzt werden, ist beispielsweise in US 4539507, US 5151629, EP 0676461 und WO 98/27136 beschrieben. Als emittierende Materialien werden hierbei neben fluoreszierenden Emittern zunehmend metallorganische Komplexe eingesetzt, die Phosphoreszenz zeigen (M. A. Baldo et al., Appl. Phys. Lett. 1999, 75, 4-6). OLEDs stellen eine sehr vielversprechende Technologie für Bildschirm- und Beleuchtungsanwendungen dar. Dazu sind OLEDs erforderlich, die im sichtbaren Bereich des Spektrums Licht emittieren, also typsicherweise rotes, grünes und blaues Licht.

Weiterhin gibt es viele Anwendungen, die Licht bzw. Strahlung mit noch kürzeren Wellenlängen erfordern. So sind, z. B., im Bereich Life Science und Medizin 280 bis 400 nm für Cell Imaging oder für Biosensoren notwendig. Weiterhin werden in der Elektronikindustrie 300 bis 400 nm für Solid-State Lighting und 300 bis 365 nm , beispielsweise, für die Aushärtung von Polymeren und Druck-Tinte benötigt. Von großer Bedeutung sind auch phototherapeutische Anwendungen im medizinischen oder kosmetischen Bereich. Mittels Phototherapie können viele unerwünschte Hautveränderungen und Hautkrankheiten behandelt werden. Oftmals werden hierfür Wellenlängen im Bereich der ultravioletten (UV) Strahlung benötigt. Ein Beispiel hierfür ist die Behandlung der Haut psoriatischer Patienten, wofür typischerweise eine Strahlungsquelle eingesetzt wird, die UV-Strahlung einer Wellenlänge von 311 nm emittiert.

Quecksilber-, Deuterium-, Excimer- und Xenon-Lampen sind typische, konventionelle UV-Strahlungsquellen. Allerdings sind sie unhandlich und einige enthalten giftige Stoffe, die Verschmutzungen verursachen und Gesundheitsgefährdungen darstellen können. Die konventionellen Lampen haben daher Nachteile bezüglich Sicherheit, Anwendbarkeit, Handhabbarkeit und Portabilität, was wiederum zu begrenzten Anwendungsmöglichkeiten führt. Daneben sind auch UV-LEDs kommerziell erhältlich. Allerdings befinden sich die meisten dieser LEDs entweder im Forschungsstadium, emittieren nur Strahlung mit einer Wellenlänge größer 365 nm oder sind sehr teuer. LEDs haben zudem den Nachteil, dass es sich um Punktstrahler handelt, die relativ dicke und steife Vorrichtungen erfordern. Eine andere Klasse von Strahlungsquellen bzw. Lichtquellen sind die organischen Elektrolumineszenzvorrichtungen (z.B. OLEDs oder OLECsorganische lichtemittierende elektrochemische Zellen). Bei ihnen handelt es sich im Gegensatz zu den anderen Licht- und Strahlungsquellen um Flächenstrahler. Weiterhin gestatten die organischen Elektrolumineszenzvorrichtungen die Herstellung flexibler Geräte, wie Displays, Beleuchtungs- und Bestrahlungsvorrichtungen. Diese Vorrichtungen sind auch wegen ihrer Effizienz und des einfachen und platzsparenden Aufbaus für viele Anwendungen besonders gut geeignet.

Allerdings ist bisher nur sehr wenig über organische Elektrolumineszenzvorrichtungen bekannt, die Strahlung im UV-Bereich emittieren. Die Emission der meisten organischen Elektrolumineszenzvorrichtungen ist meist auf Wellenlängen größer als 350 nm begrenzt. Zudem sind die Leistungsdaten dieser Vorrichtungen sehr schlecht.

Chao et al. berichten (Adv.Mater. 17[8], 992-996. 2005.) von UV OLEDs basierend auf Fluoren Polymeren mit einer Elektrolumineszenz-Emissionswellenlänge größer als 360 nm;
Wong et al. berichten (Org.Lett. 7[23], 5131-5134. 2005) von UV OLEDs basierend auf Spiro-Bifluoren Polymeren mit einer Elektrolumineszenz-Emissionswellenlänge bei 360 nm oder größer;
Zhou et al. berichten (Macromolecules 2007, 40 (9), 3015-3020) von UV OLEDs mit emittierenden Polymeren basierend auf Fluoren und Tetraphenylsilan Derivaten mit einer Elektrolumineszenz-Emissionswellenlänge bei 350 nm;
Shinar et al. berichten (Applied Surface Science 2007, 254 (3), 749-756) von UV OLEDs unter Verwendung von Bu-PBD als Emitter mit einer Elektrolumineszenz-Emissionswellenlänge von 350 nm.

Burrows berichtet (Applied Physics Letters 2006, 88 (18), 183503) von einer OLED enthaltend 4,4 '-Bis (diphenylphosphinoxid) biphenyl als Emitter. Die Vorrichtung emittiert bei 337 nm.

Sharma et al., berichtet (Applied Physics Letters 2006, 88 (14), 143511-143513) von einer UV OLED, die bei 357 nm emittiert. Der verwendete Emitter basiert auf Polysilan.

In JP 403162484 werden Oligophenylene beschrieben, die in einer emittierenden Schicht einer OLED eingesetzt werden.

In US2010/0308313 werden Materialien beschrieben, die für organische Elektrolumineszenvorrichtungen geeignet sind, die mindestens drei Phenylgruppen haben, wobei mindestens eine Phenylengruppe mit zwei Oligophenylgruppen substituiert ist.

Es besteht daher ein sehr großer Bedarf organische Elektrolumineszenzvorrichtungen zu entwickeln, die Strahlung im UV Bereich, insbesondere im unteren UV-A Bereich (315 bis 380 nm) sowie im UV-B Bereich (280 bis 315 nm) emittieren. Eine besondere Herausforderung stellt dabei die Bereitstellung geeigneter organischer Emittermaterialien sowie die Bereitstellung organischer Elektrolumineszenzvorrichtungen enthaltend diese Emitter dar.

Aufgabe der vorliegenden Erfindung ist daher die Beseitigung der genannten Nachteile des Standes der Technik

durch Bereitstellung organischer elektrolumineszierender Vorrichtungen mit möglichst guten physikalischen Eigenschaften, die eine Emission im UV-Bereich zeigen.

Überraschend wurde gefunden, dass bestimmte, unten näher beschriebene Verbindungen diese Aufgaben lösen und zu organischen Elektrolumineszenzvorrichtung mit unerwartet guten Eigenschaften führen.

Organische Elektrolumineszenzvorrichtungen, welche derartige Verbindungen enthalten sind daher Gegenstand der vorliegenden Erfindung.

Die vorliegende Erfindung betrifft eine Elektrolumineszenzvorrichtung, vorzugsweise eine organische Elektrolumineszenzvorrichtung nach Anspruch 1, die Strah lung einer Wellenlänge von kleiner oder gleich 350 nm emittiert und bevorzugt UV-B Strahlung emittiert, d.h., Strahlung mit einer Wellenlänge im Bereich von 280 bis 315 nm. Die Vorrichtung enthält wenigstens zwei Elektroden und wenigstens eine emittierende Schicht, wobei die emittierende Schicht wenigstens eine Verbindung der unten genannten Formeln (89-1) und/oder (89-2) enthält.

Die vorliegende Erfindung liegt auf dem Gebiet einer Elektrolumineszenzvorrichtung enthaltend mindestens zwei Elektroden und wenigstens eine emittierende Schicht zwischen den Elektroden, wobei die Vorrichtung Strahlung mit einer Wellenlänge im Bereich von 280 nm und 380 nm emittiert.

Ein geeigneter Emitter ist wenigstens eine Verbindung der allgemeinen Formel (1),

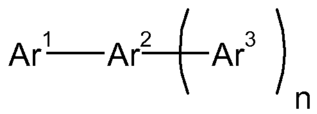

Formel (1)

wobei für die verwendeten Symbole und Indizes gilt:

$Ar^1$, $Ar^2$ und $Ar^3$ sind gleich oder verschieden fünf- oder sechsgliedrige aromatische und/oder heteroaromatische Ringe, die jeweils mit einem oder mehreren Resten $R^1$, die unabhängig voneinander sein können, substituiert sein können;

n ist 0 oder 1;

$R^1$ ist gleich oder verschieden bei jedem Auftreten H, D, F, $N(R^2)_2$, CN, $Si(R^2)_3$, $B(OR^2)_2$, $P(R^2)_2$, $S(=O)R^2$, eine geradkettige Alkyl-, Alkoxy- oder Thioalkoxygruppe mit 1 bis 40 C-Atomen oder eine verzweigte oder cyclische Alkyl-, Alkoxy-, Alkylalkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen, durch $Si(R^2)_2$, $Ge(R^2)_2$, $Sn(R^2)_2$, C=O, C=S, C=Se, $C=NR^2$, SO, $SO_2$, $NR^2$, O, S oder $CONR^2$, die nich direkt an Ring nach Formel (I) gebunden ist durch $R^2C=CR^2$, C=C oder $P(=O)(R^2)$ ersetzt sein können und wobei ein oder mehrere H-Atome durch D, F, Cl, oder CN ersetzt sein können, oder ein aromatischer oder hetero-aromatischer Ring mit 5 bis 18 aromatischen Ringatomen, der jeweils durch einen oder mehrere Reste $R^2$ substituiert sein kann, oder eine Kombination aus zwei oder mehr dieser Gruppen, dabei können zwei oder mehrere Substituenten $R^1$ auch miteinander ein nicht aromatisches Ringsystem bilden;

$R^2$ ist gleich oder verschieden bei jedem Auftreten H, D, F, Cl, $N(R^3)_2$, CN, $Si(R^3)_3$, $B(OR^3)_2$, $C(=O)R^3$, $P(=O)(R^3)_2$, $S(=O)R^3$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy-, Alkylalkoxy- oder Thioalkoxygruppe mit 3 bis 40 C-Atomen, die jeweils mit einem oder mehreren Resten $R^3$ substituiert sein kann, wobei eine oder mehrere nicht benachbarte $CH_2$-Gruppen durch $R^3C=CR^3$, C=C, $Si(R^3)_2$, $Ge(R^3)_2$, $Sn(R^3)_2$, C=O, C=S, C=Se, $C=NR^3$, $P(=O)(R^3)$, SO, $SO_2$, $NR^3$, O, S oder $CONR^3$ ersetzt sein können und wobei ein oder mehrere H-Atome durch D, F, Cl oder CN ersetzt sein können, oder ein aromatischer oder heteroaromatischer Ring mit 5 bis 18 aromatischen Ringatomen, der jeweils durch einen oder mehrere Reste $R^3$ substituiert sein kann, oder eine Kombination aus zwei oder mehr dieser Gruppen; dabei können zwei oder mehrere benachbarte Reste $R^2$ miteinander ein nicht aromatisches Ringsystem bilden;

$R^3$ ist gleich oder verschieden bei jedem Auftreten H, D, F oder ein aliphatischer, aromatischer und/oder heteroaromatischer Kohlenwasserstoffrest mit 1 bis 18 C-Atomen, in dem auch ein oder mehrere H-Atome durch F ersetzt sein können; dabei können zwei oder mehrere Substituenten $R^3$ auch

miteinander ein nicht aromatisches mono- oder polycyclisches, aliphatisches Ringsystem bilden.

mit der Maßgabe, dass die Verbindung der Formel (1) keine kondensierte aromatische oder kondensierte heteroaromatische Ringsysteme enthält, und mit der Maßgabe, dass die Verbindung der Formel (1) keine konjugierte Teilstruktur enthält, in der mehr als 18 konjugierte π(Pi)-Elektronen vorliegen.

Die Gruppen $Ar^1$, $Ar^2$ und $Ar^3$ sind beliebige aromatische und/oder heteroaromatische Ringe mit 5 oder 6 Ringatomen.

Es ist bevorzugt, wenn die Gruppen $Ar^1$, $Ar^2$ und $Ar^3$ in der Verbindung der allgemeinen Formel (1) gleich oder verschieden bei jedem Auftreten eine Verbindung der allgemeinen Formel (2) sind, wobei die Bindung zwischen $Ar^1$ und $Ar^2$ sowie zwischen $Ar^2$ und $Ar^3$ an jeder beliebigen Stelle der Verbindung der Formel (2) erfolgen kann;

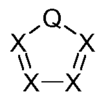

Formel (2)

wobei für die verwendeten Symbole gilt:

X Ist bei jedem Auftreten gleich oder verschieden $CR^1$ oder N;

Q ist bei jedem Auftreten gleich oder verschieden X=X, $NR^1$, O, S, Se, bevorzugt, X=X, $NR^1$ und S und ganz bevorzugt X=X und $NR^1$.

Weiterhin bevorzugt ist, wenn die Gruppen $Ar^1$, $Ar^2$ und $Ar^3$ in der Verbindung der Formel (1) gleich oder verschieden bei jedem Auftreten eine der folgenden Gruppen sind, wobei die Bindung zwischen den Gruppen an jeder beliebigen und chemisch möglichen Stelle erfolgen kann, und wobei die Gruppen mit einem oder mehreren voneinander unabhängigen Resten $R^1$ substituiert sein können, wobei die Reste $R^1$ wie oben angegeben definiert sind.

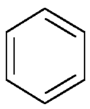

Formel (3)

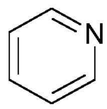

Formel (4)

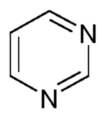

Formel (5)

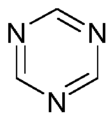

Formel (6)

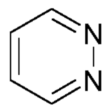

Formel (7)

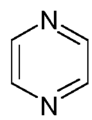

Formel (8)

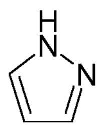

Formel (9)

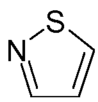

Formel (10)

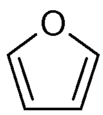

Formel (11)

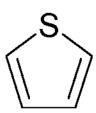

Formel (12)

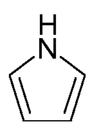

Formel (13)

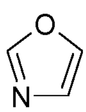

Formel (14)

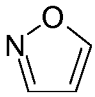

Formel (15)

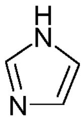

Formel (16)

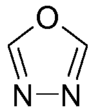

Formel (17)

Bevorzugt enthält die Elektrolumineszenzvorrichtung wenigstens eine Verbindung der Formel (18a) oder (18b) in der emittierenden Schicht

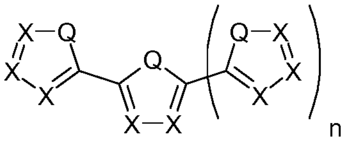

Formel (18a)

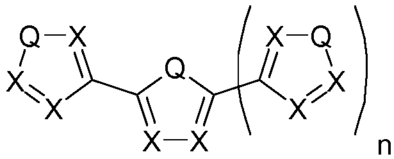

Formel (18b)

wobei obige Definitionen für die Symbole und den Index gelten.

Die Verbindungen der allgemeinen Formel (1) sind vorzugsweise fluoreszierende Emitter, d.h., die Verbindungen emittieren Strahlung aus einem elektronisch angeregten Singulett-Zustand.

Weiterhin bevorzugt ist, wenn die Verbindung der Formel (1) in der Elektrolumineszenzvorrichtung keine kondensierten Ringe enthält.

In einer weiteren geeigneten Ausführungsform enthält die genannte Elektrolumineszenzvorrichtung die Verbindung der allgemeinen Formel (1) in der emittierenden Schicht, wobei zwischen $Ar^1$, $Ar^2$ und $Ar^3$ wenigstens eine nichtaromatische Verbrückung vorkommt, vorzugsweise entsprechend den nachfolgenden allgemeinen Formeln (19) bis (26)

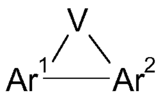

Formel (19)

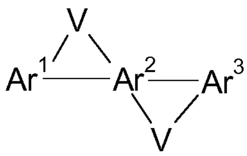

Formel (20)

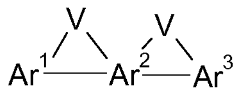

Formel (21)

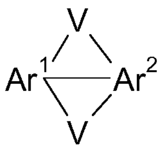

Formel (22)

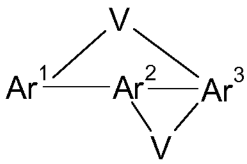

Formel (23)

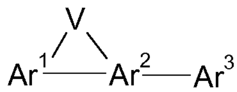

Formel (24)

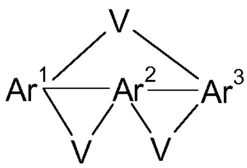

Formel (25)

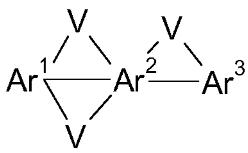

Formel (26)

wobei für $Ar^1$, $Ar^2$ und $Ar^3$ die obigen Definitionen gelten und wobei V gleich oder verschieden bei jedem Auftreten ist und für eine nichtaromatische Verbrückung der Aromaten $Ar^1$ bis $Ar^3$ steht und O, S, Se, N, Si, B, P und/oder wenigstens eine $C(R^2)_2$ Gruppe enthält.

Verbrückte Strukturen haben den Vorteil, dass die Effizienz und Stabilität der Vorrichtungen weiter gesteigert werden. In Hinblick auf die gewünschte UV-Emission ist jedoch zu beachten, dass die in den Verbindungen gemäß Formel (1) enthaltenen konjugierten Pi-Systeme, wie weiter oben angeben, ausreichend klein gehalten wird.

In einer bevorzugten Ausführungsform erfolgt die nichtaromatische Verbrückung durch V, wobei V gleich $C(R^2)_2$ oder $Si(R^2)_2$ ist.

Weitere ganz bevorzugte Ausführungsformen für V sind in der folgenden Übersicht zusammengefasst.

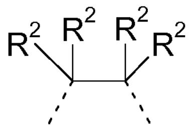
Formel (27)

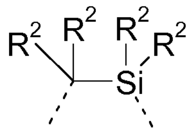
Formel (28)

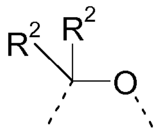
Formel (29)

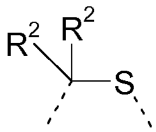
Formel (30)

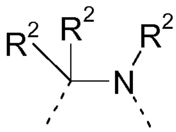
Formel (31)

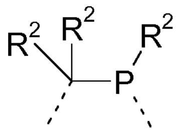
Formel (32)

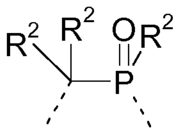
Formel (33)

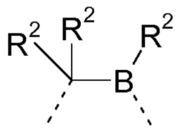
Formel (34)

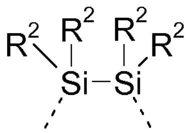
Formel (35)

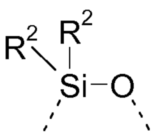
Formel (36)

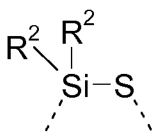
Formel (37)

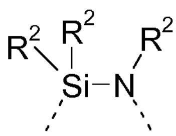
Formel (38)

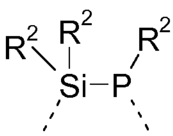
Formel (39)

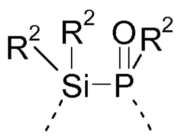
Formel (40)

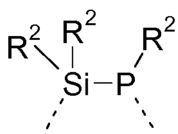
Formel (41)

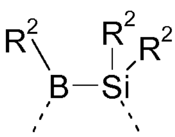
Formel (42)

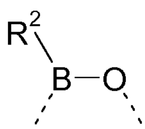
Formel (43)

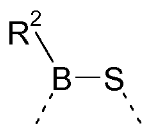
Formel (44)

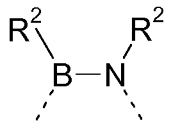

Formel (45)

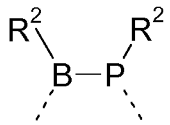

Formel (46)

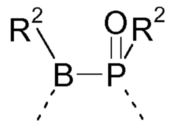

Formel (47)

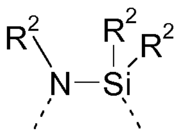

Formel (48)

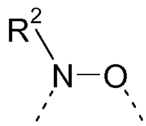

Formel (49)

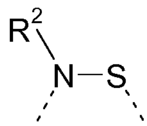

Formel (50)

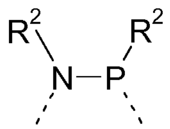

Formel (51)

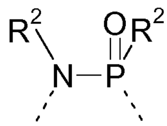

Formel (52)

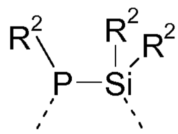

Formel (53)

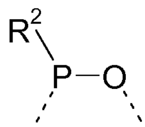

Formel (54)

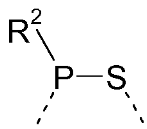

Formel (55)

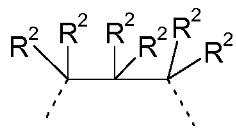

Formel (56)

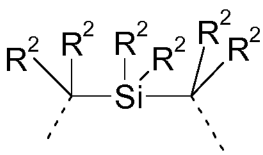

Formel (57)

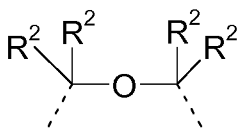

Formel (58)

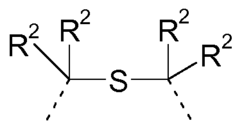

Formel (59)

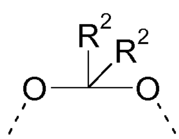

Formel (60)

wobei die Reste $R^2$ wie oben angegeben definiert sind und wobei die gestrichelten Linien die Bindungen zu den Gruppen $Ar^1$, $Ar^2$ oder $Ar^3$ kennzeichnen.

In einer geeigneten Ausführungsform enthält die Elektrolumineszenzvorrichtung in der emittierenden Schicht eine Verbindung ausgewählt aus den Verbindungen mit den Formeln (61) bis (68)

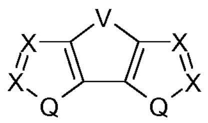

Formel (61)

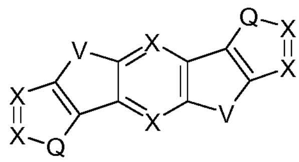

Formel (62)

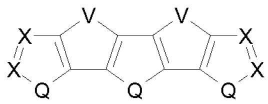

Formel (63)

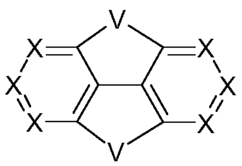

Formel (64)

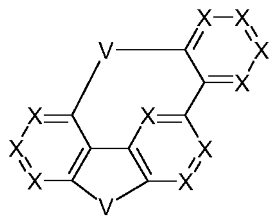

Formel (65)

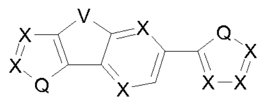

Formel (66)

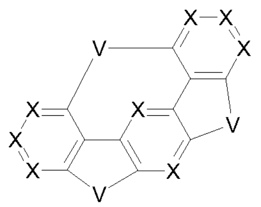

Formel (67)

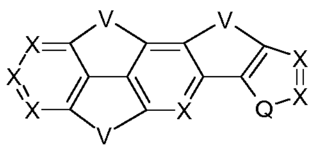

Formel (68),

wobei X, Q und V wie oben angegeben definiert sind.

Ganz besonders bevorzugt ist, dass Q gleich X=X ist.

Es ist weiterhin ganz besonders bevorzugt, wenn X = $CR^1$ ist.

Insbesondere bevorzugt ist, wenn $Ar^2$ in der Verbindung der Formel (1) ausgewählt ist aus den Formeln (69) bis (86)

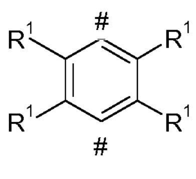

Formel (69)

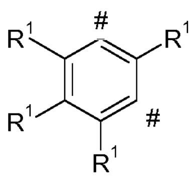

Formel (70)

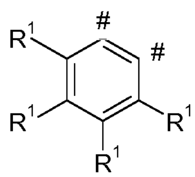

Formel (71)

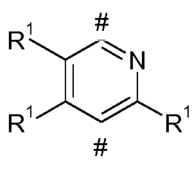

Formel (72)

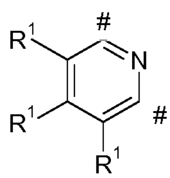

Formel (73)

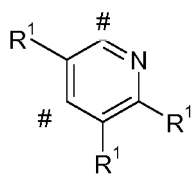

Formel (74)

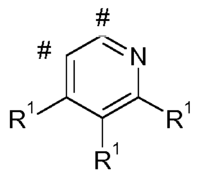
Formel (75)

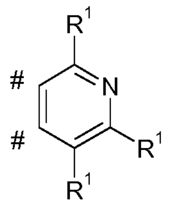
Formel (76)

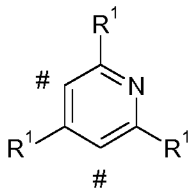
Formel (77)

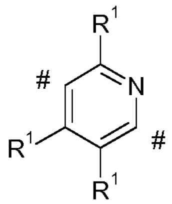
Formel (78)

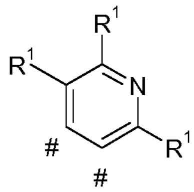
Formel (79)

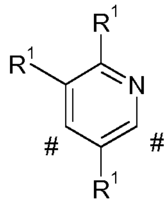
Formel (80)

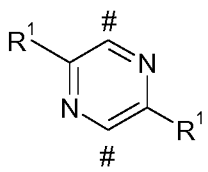
Formel (81)

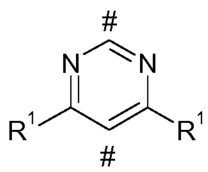
Formel (82)

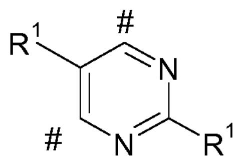
Formel (83)

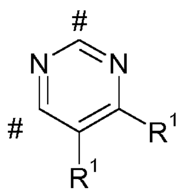
Formel (84)

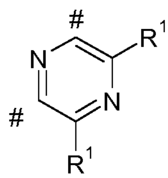
Formel (85)

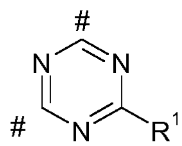
Formel (86),

wobei # für die Verknüpfungspositionen zu und $Ar^3$ steht und wobei für den Fall n = 0 gilt, dass eine der beiden mit # gekennzeichneten C-Atome mit dem Rest $R^1$ substituiert ist.

Noch mehr bevorzugt ist, wenn $Ar^2$ in der Verbindung der Formel (1) gleich der Formel (87) ist,

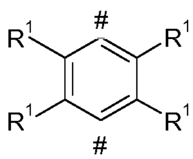
Formel (87),

wobei für die mit # gekennzeichneten Positionen obige Ausführungen gelten.

Weiterhin bevorzugt sind die Gruppen und $Ar^3$, die gleich oder verschieden voneinander sind können, gleich einer Verbindung der allgemeinen Formel (88),

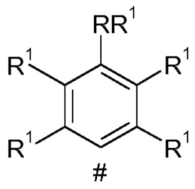

Formel (88),

wobei $RR^1$ entweder gleich $R^1$ oder aber gleich H, F, Cl, eine linear oder verzweigte oder cyclische Alkyl-, Alkoxy-, Alkylalkoxy- oder Thioalkoxygruppe mit 1 bis 40 C-Atomen ist, wobei die Gruppen unsubstituiert sein können oder mit $CF_3$, Halogen, CN einfach substituiert sein können und wobei eine oder mehrere $CH_2$-Gruppen substituiert sein können durch -O-, -S-, $-CF_2O-$, $-OCF_2-$, -OC-O- oder -O-CO- und zwar in der Art, dass die O-Atome nicht direkt miteinander verbunden sind.

In einer weiterhin insbesondere bevorzugten Ausführungsform hat die Verbindung in der emittierenden Schicht eine der folgende allgemeinen Formeln (89-1) bis (89-6)

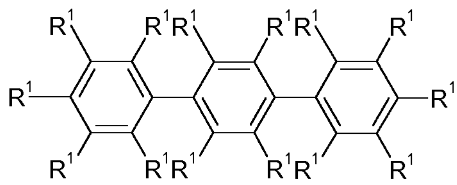

Formel (89-1)

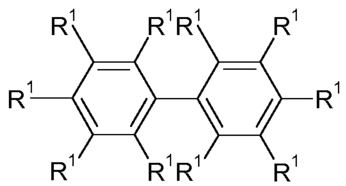

Formel (89-2)

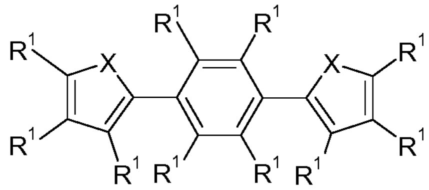

Formel (89-3)

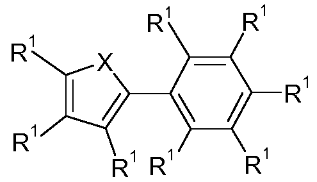

Formel (89-4)

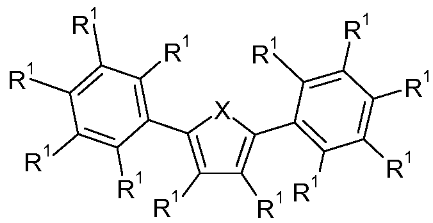

Formel (89-5)

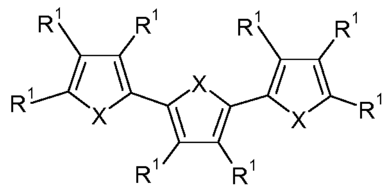

Formel (89-6),

wobei bevorzugt maximal 6 der Reste $R^1$ ungleich H sind und wobei die obigen Definitionen für die Reste $R^1$ gelten.

Ganz besonders bevorzugt als Emitter in der Emissionsschicht sind gemäß der Erfindung die Verbindungen der allgemeinen Formeln (89-1) und/oder (89-2).

In einer weiterhin bevorzugten Ausführungsform der Erfindung ist $R^1$ gleich oder verschieden bei jedem Auftreten ausgewählt aus der Gruppe bestehend aus H, F, CN, $CF_3$, $CHF_2$ und einer Alkyl-, Alkoxy-, Alkylalkoxygruppe mit 1 bis 15 C-Atomen.

In einer weiteren bevorzugten Ausführungsform ist mehr als ein Rest $R^1$ in den oben genannten Formeln, die $R^1$ enthalten, gleich F, ganz bevorzugt sind mehr als 2 der Reste $R^1$ gleich F und ganz besonders bevorzugt sind mehr als 3 Reste $R^1$ gleich F.

In einer weiteren geeigneten Ausführungsform ist die Verbindung in der emittierenden Schicht ausgewählt aus der Verbindung der Formel (89-1a)

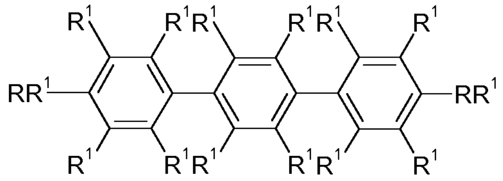

Formel (89-1a)

wobei bevorzugt maximal 4 der Reste $R^1$ ungleich H sind und RR1 wie oben angegeben definiert ist.

Beispiele für geeignete Verbindungen gemäß den oben aufgeführten Ausführungsformen sind die Emitter-Verbindungen der folgenden Strukturen.

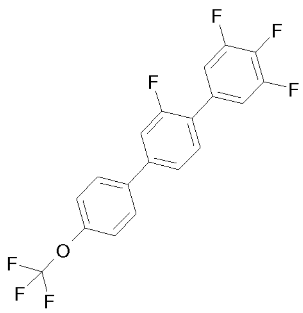

Formel (90)

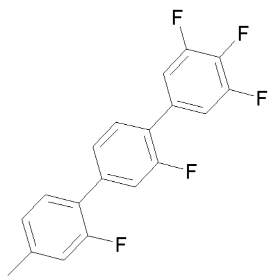

Formel (91)

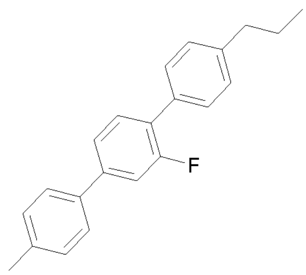

Formel (92)

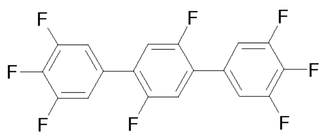

Formel (93)

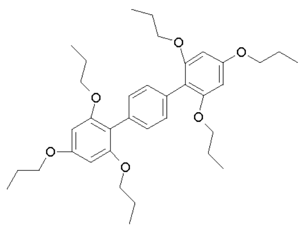

Formel (94)

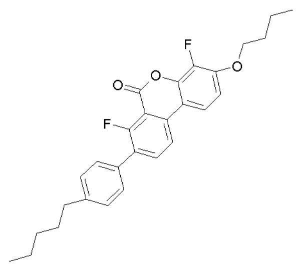

Formel (95)

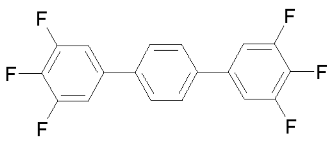

Formel (96)

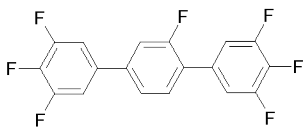

Formel (97)

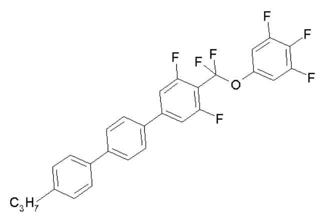

Formel (98)

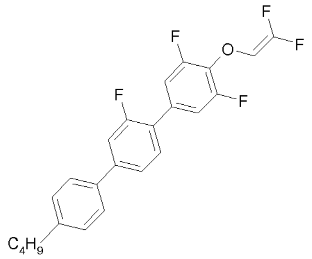

Formel (99)

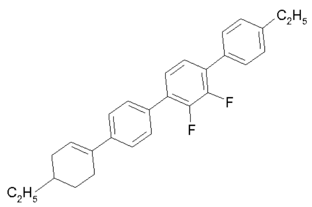

Formel (100)

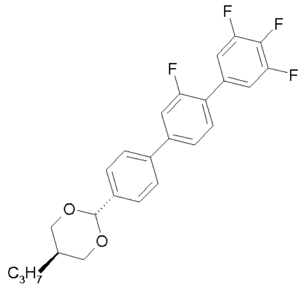

Formel (101)

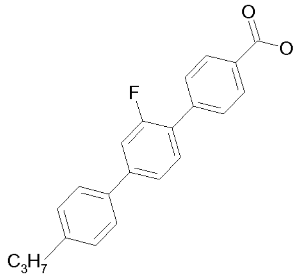

Formel (102)

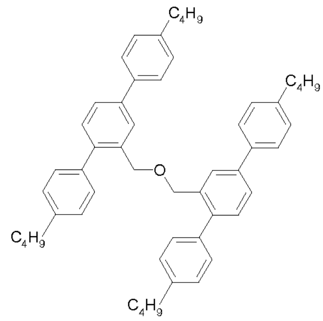

Formel (102a)

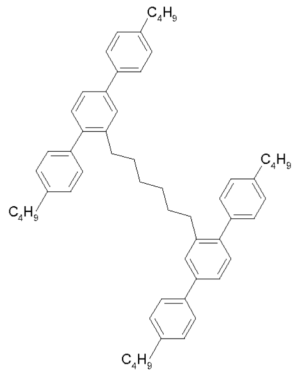

Formel (103)

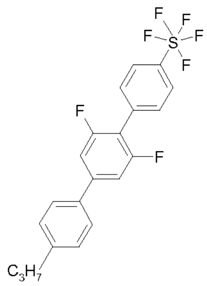

Formel (104)

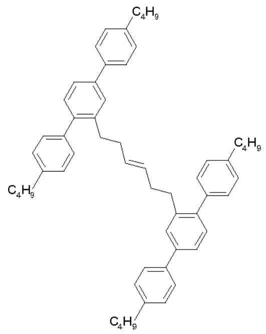

Formel (105)

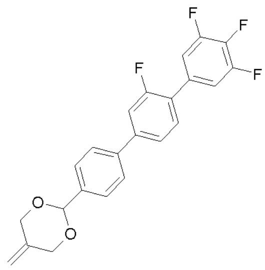

Formel (106)

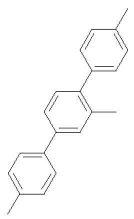

Formel (107)

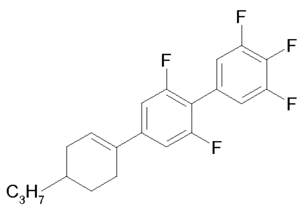

Formel (108)

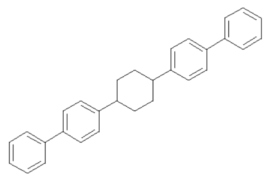

Formel (109)

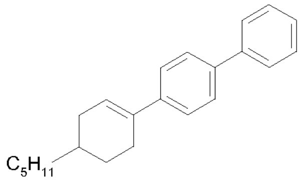

Formel (110)

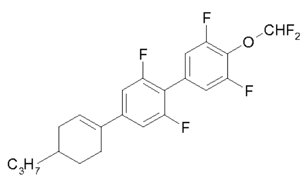

Formel (111)

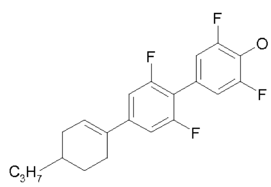

Formel (112)

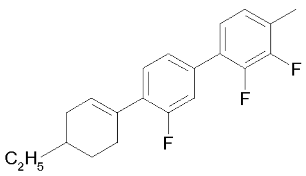

Formel (113)

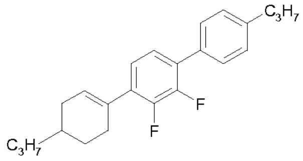

Formel (114)

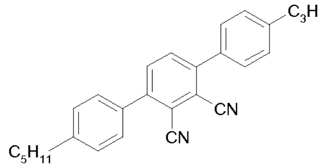

Formel (115)

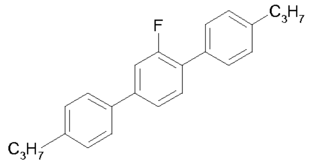

Formel (116)

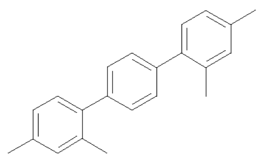

Formel (117)

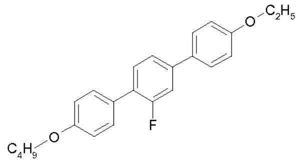

Formel (118)

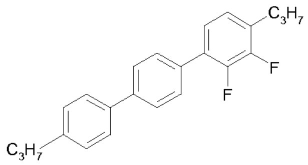

Formel (119)

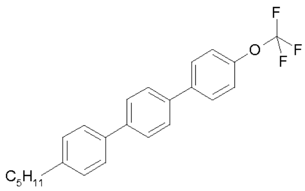

Formel (120)

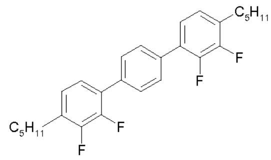

Formel (121)

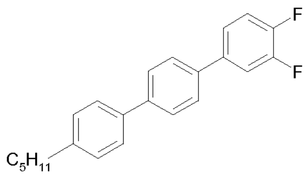

Formel (122)

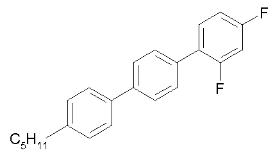

Formel (123)

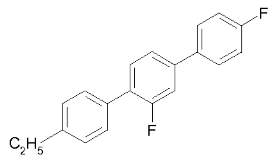

Formel (124)

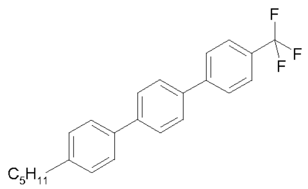

Formel (125)

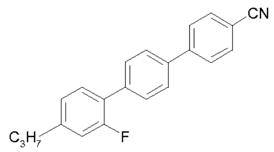

Formel (126)

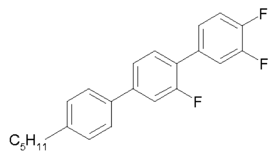

Formel (127)

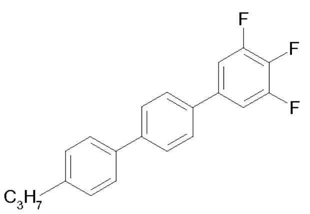

Formel (128)

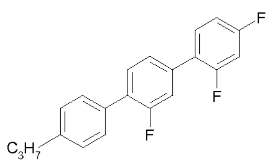

Formel (129)

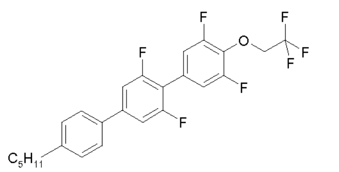

Formel (130)

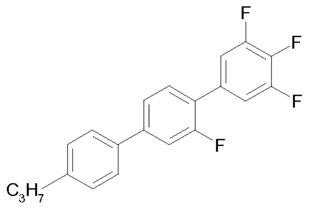
Formel (131)

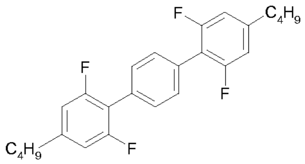
Formel (132)

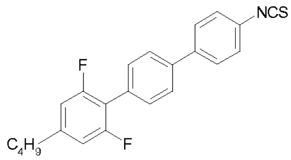
Formel (133)

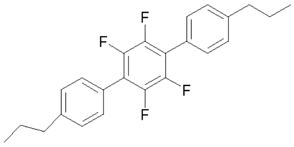
Formel (134)

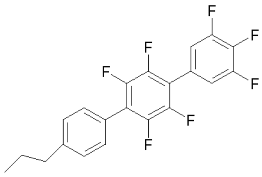
Formel (135)

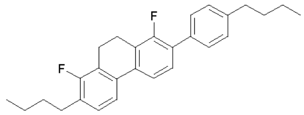
Formel (136)

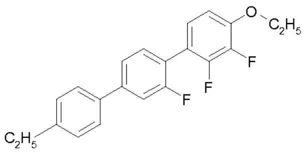
Formel (137)

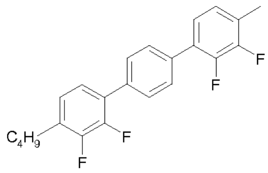
Formel (138)

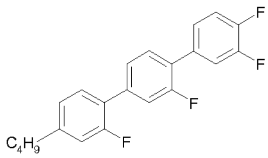
Formel (139)

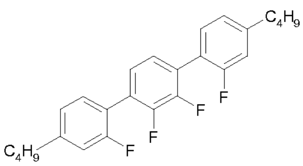
Formel (140)

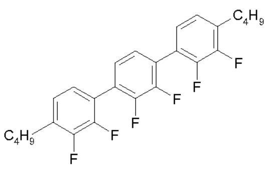
Formel (141)

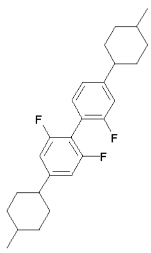
Formel (142)

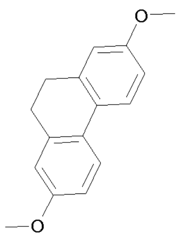
Formel (143)

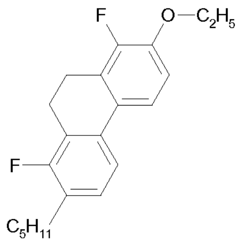
Formel (144)

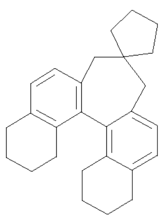
Formel (145)

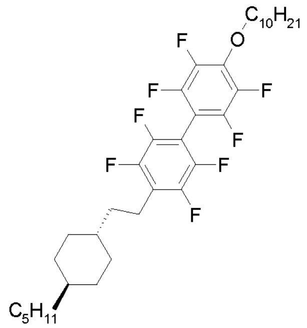
Formel (146)

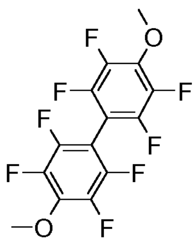
Formel (147)

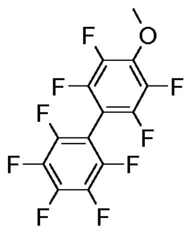
Formel (148)

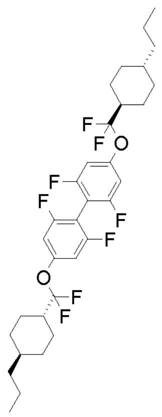
Formel (149)

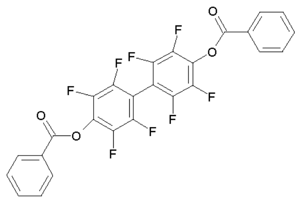
Formel (150)

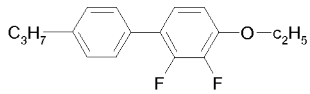
Formel (151)

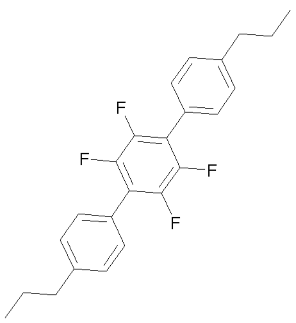
Formel (152)

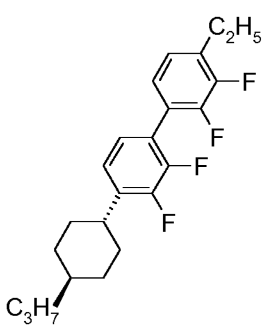
Formel (153)

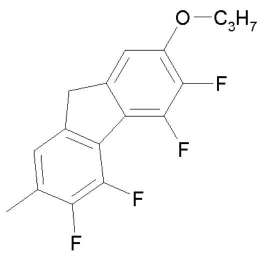
Formel (154)

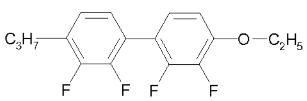
Formel (155)

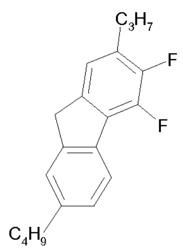
Formel (156)

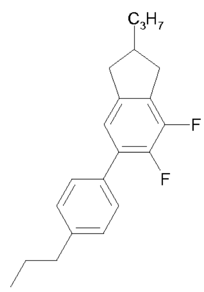
Formel (157)

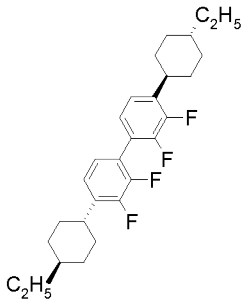
Formel (158)

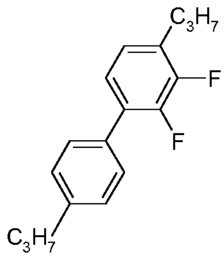
Formel (159)

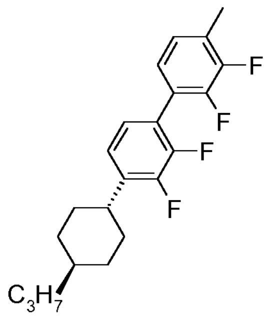
Formel (160)

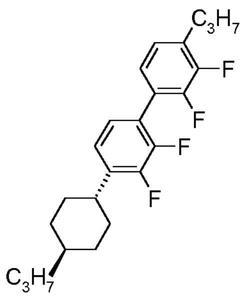
Formel (161)

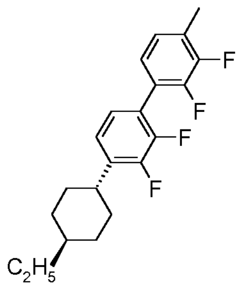
Formel (162)

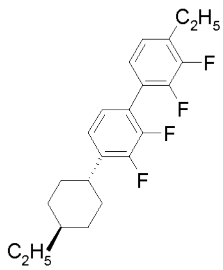
Formel (163)

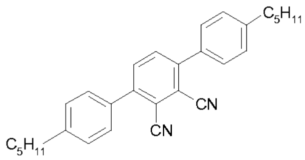
Formel (164)

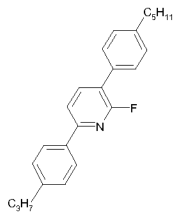
Formel (165)

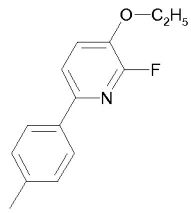
Formel (166)

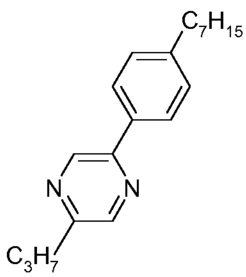
Formel (167)

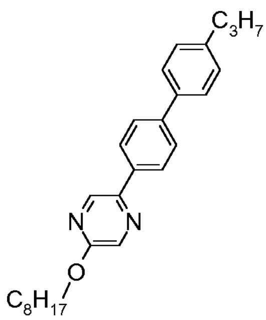
Formel (168)

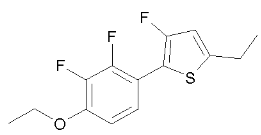
Formel (169)

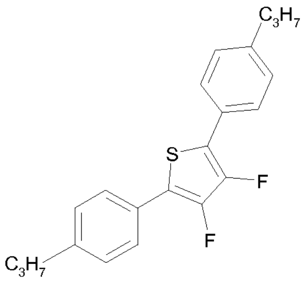
Formel (170)

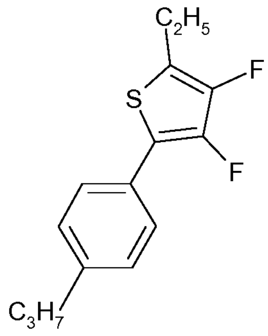
Formel (171)

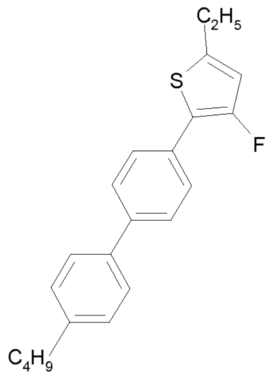
Formel (172)

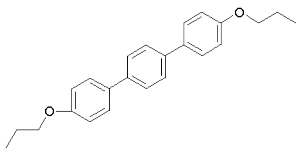
Formel (173)

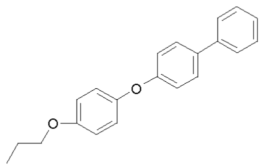
Formel (174)

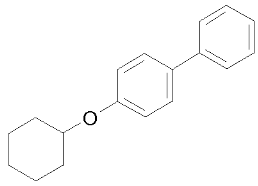
Formel (175)

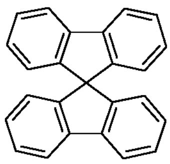
Formel (176)

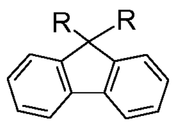
Formel (177)

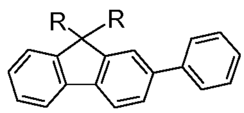
Formel (178)

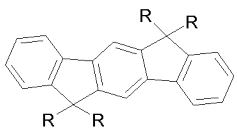
Formel (179)

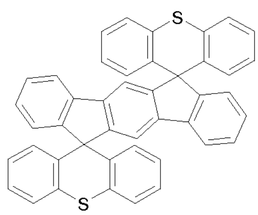
Formel (180)

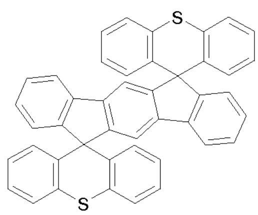
Formel (181)

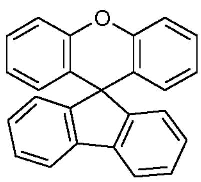
Formel (182)

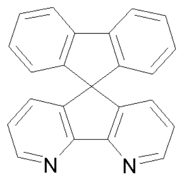
Formel (183)

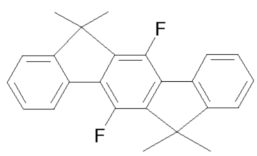
Formel (184)

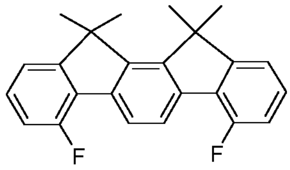

Formel (185)

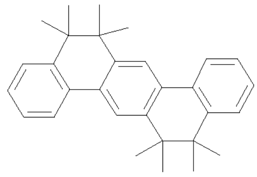

Formel (186)

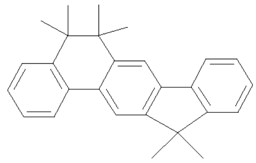

Formel (187)

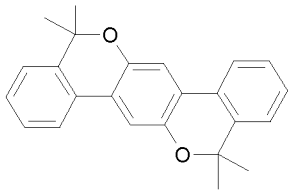

Formel (188)

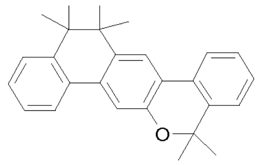

Formel (189)

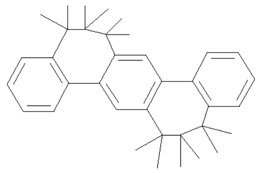

Formel (190)

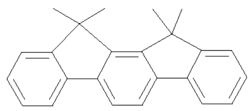

Formel (191)

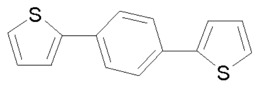

Formel (192)

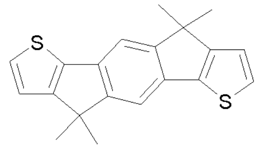

Formel (193)

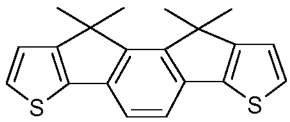

Formel (194)

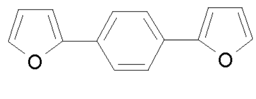

Formel (195)

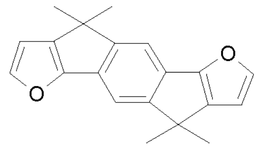

Formel (196)

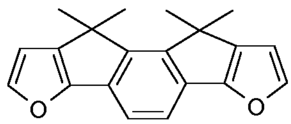

Formel (197)

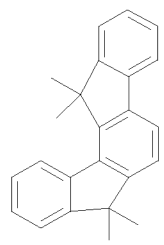

Formel (198)

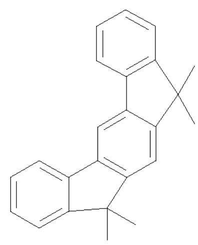

Formel (199)

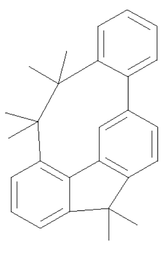

Formel (200)

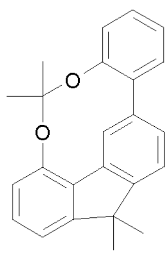

Formel (201)

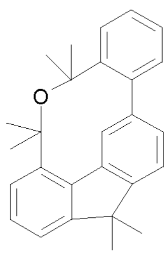

Formel (202),

wobei R wie $R^1$ definiert ist.

In einer Ausführungsform enthält die emittierende Schicht der erfindungsgemäßen Vorrichtung einen Emitter

gemäß Anspruch 1 und wenigstens ein Host Material. Dabei hat das Host Material eine größere Bandlücke (= Abstand zwischen Valenzband (LUMO - lowest unoccupied molecular orbital) und Leitungsband (HOMO- highest occupied molecular orbital)) oder einen höheren angeregten elektronischen Zustand. Das Host Material weist folglich ein höheres $S_1$- oder $T_1$-Niveau auf, bevorzugt ist das $S_1$-Niveau des Host Materials höher als das des Emitters. Hierbei ist $S_1$ das erste elektronisch angeregte Singulett-Niveau. $T_1$ ist das erste elektronisch angeregte Triplett-Niveau.

Die oben genannten Materialien können als Emitter in der Emissionsschicht eingesetzt werden. Die Materialien gemäß Formel (1) können aber auch als Hostmaterialien eingesetzt werden. Die Host-Verbindung gemäß Formel (1) kann entweder mit wenigstens einem beliebigen Dotanden (Emitter) oder mit wenigstens einem Emitter gemäß Formel (1) dotiert sein. Die vorliegende Erfindung betrifft daher auch eine Elektrolumineszenzvorrichtung, die dadurch charakterisiert ist, dass die Emissionsschicht wenigstens eine Verbindungen gemäß Anspruch 1 als Hostmaterial in der Emissionsschicht aufweist.

Die vorliegende Erfindung betrifft daher auch eine Elektrolumineszenzvorrichtung, die dadurch charakterisiert ist, dass die Emissionsschicht wenigstens eine Verbindung gemäß Anspruch 1 als Hostmaterial sowie wenigstens eine Verbindung gemäß Anspruch 1 als Emitter in der Emissionsschicht enthält.

Weiterhin bevorzugt enthält die emittierende Schicht ein Hostmaterial nach Formel (1) oder (18), wobei n gleich 0 ist.

Weiterhin bevorzugt im Sinne der vorliegenden Erfindung ist die Verwendung von Polystyrol oder von Derivaten von Polystyrol als Host Material.

In einer weiteren Ausführungsform enthält die emittierende Schicht wenigstens einen weiteren UV-Emitter und/oder wenigstens ein weiteres Host Material.

Geeignete Materialien für den emittierende Schicht, entweder als Emitter oder als Host, sind in der folgenden Tabelle mit den entsprechenden Referenzen exemplarisch zusammengestellt.

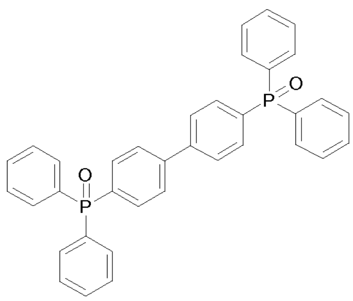

Formel (203)

Burrows et al., Appl. Phys. Lett., 88, 183503, 2006

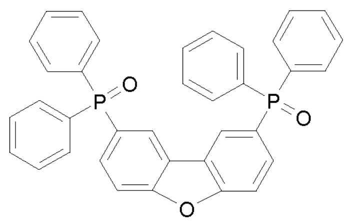

Formel (204)

Vecchi et al., Organic Letters, 8, 4211, 2006

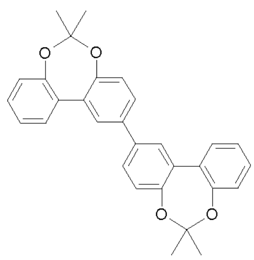

Formel (205)

Zhang et al., J. Phys. Chem. B, 108, 9571, 2004

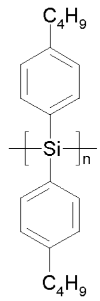

Formel (206)

Hoshino et al., J. Appl. Phys., 88, 2892, 2000

(fortgesetzt)

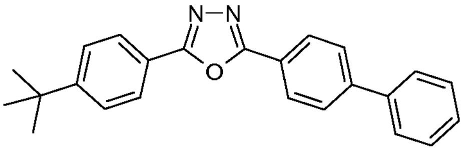

Formel (207)

Zou et al., Appl. Phys. Lett., 79, 2282, 2001

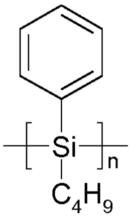

Formel (208)

Sharma et al., Appl. Phys. Lett., 88, 143511, 2006

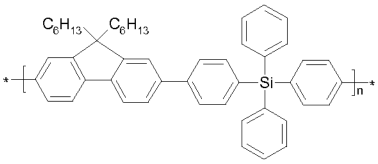

Formel (209)

Zhou et al., Macromolecules, 40, 3015, 2007

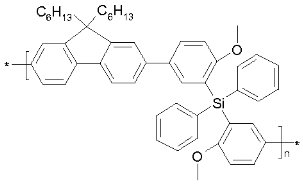

Formel (210)

Zhou et al., Macromolecules, 40, 3015, 2007

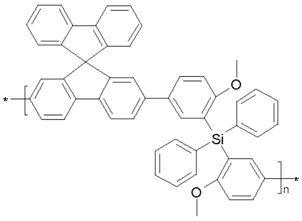

Formel (211)

Zhou et al., Macromolecules, 40, 3015, 2007

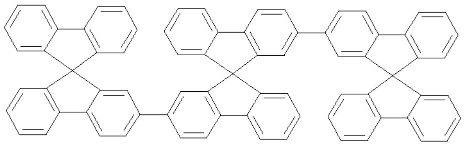

Formel (212)

Wong et al., Organic Letters, 7, 5131, 2005

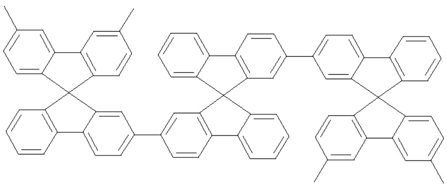

Formel (213)

Wong et al., Organic Letters, 7, 5131, 2005

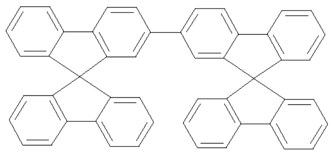

Formel (214)

Chao et al., Adv. Mater., 17, 992, 2005

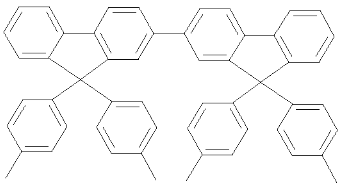

Formel (215)

Chao et al., Adv. Mater., 17, 992, 2005

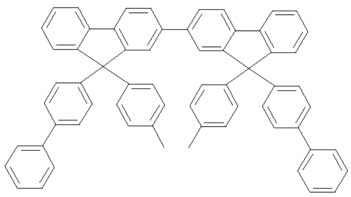

Formel (216)

Chao et al., Adv. Mater., 17, 992, 2005

(fortgesetzt)

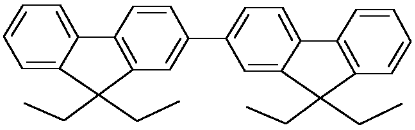

Formel (217)

Chao et al., Adv. Mater., 17, 992, 2005

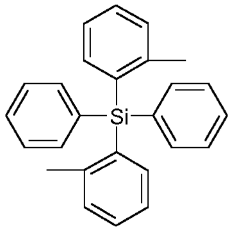

Formel (218)

Ren et al., Chem. Mater., 16, 4743, 2004

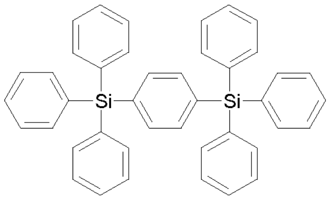

Formel (219)

Ren et al., Chem. Mater., 16, 4743, 2004

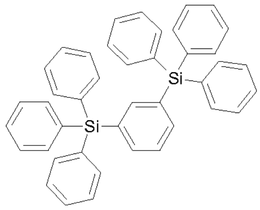

Formel (220)

Ren et al., Chem. Mater., 16, 4743, 2004

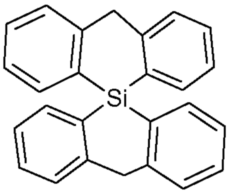

Formel (221)

Ren et al., Chem. Mater., 16, 4743, 2004

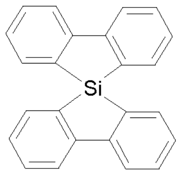

Formel (222)

Ren et al., Chem. Mater., 16, 4743, 2004

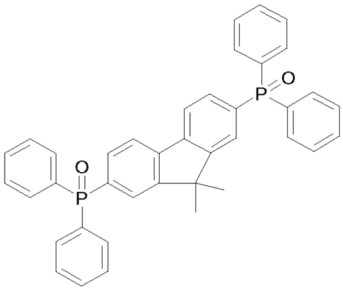

Formel (223)

Padmaperuma et al., Chem. Mater., 18, 2389, 2006

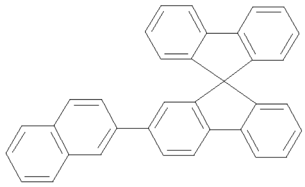

Formel (224)

Etori et al., Jpn. J. Appl. Phys., 46, 5071, 2007

(fortgesetzt)

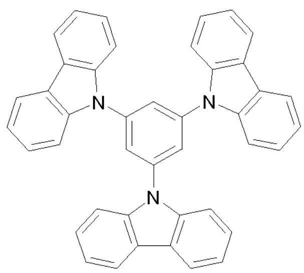

Formel (225)

Etori et al., Jpn. J. Appl. Phys., 46, 5071, 2007

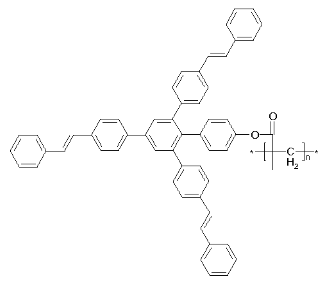

Formel (226)

Spiliopoulos et al., Macromolecules, 35, 7254, 2002

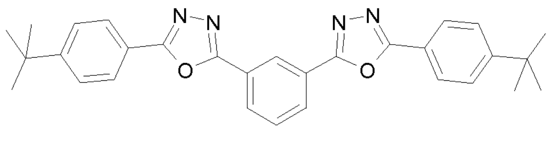

Formel (227)

Ichikawa et al., Thin Solid Films, 515, 3932, 2007

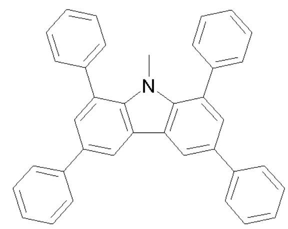

Formel (228)

Niu et al., RSC Adv., 1, 415, 2011

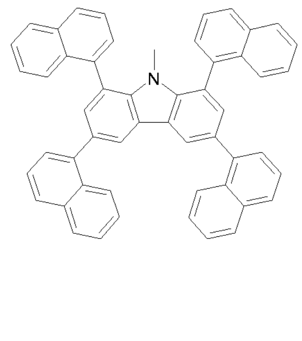

Formel (229)

Niu et al., RSC Adv., 1, 415, 2011

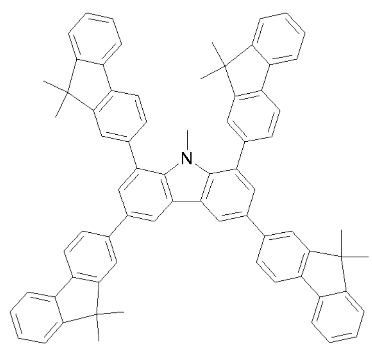

Formel (230)

Niu et al., RSC Adv., 1, 415, 2011

Die Leistungsdaten der erfindungsgemäßen Vorrichtungen können auf unterschiedliche Weise weiter verbessert werden.

Wie bereits erwähnt, wird in der Emissionsschicht organischer Elektrolumineszenzvorrichtungen neben dem Emitter oder den Emittern meist wenigstens ein Host Material verwendet. Bei Verwendung eines Mixed Host Systems in der erfindungsgemäßen Vorrichtung lassen sich aber besonders gute Ergebnisse erzielen.

In einer weiteren bevorzugten Ausführungsform wird in der Emissionsschicht der erfindungsgemäßen Vorrichtung ein Mixed-Host verwendet. Hierdurch lassen sich die Strahlungsintensitäten der Vorrichtungen signifikant erhöhen und die Betriebsspannungen deutlich verringern. Mixed-Host bedeutet, dass der Host aus mindestens 2 unterschiedlichen Verbindungen besteht. Vorzugsweise enthält der Mixed-Host mindestens eine Verbindung nach Anspruch 1. Zudem enthält der Mixed-Host eine weitere, beliebige Host Verbindung. Der Fachmann kann hierbei ohne Schwierigkeiten auf eine Vielzahl im Stand der Technik bekannter HostVerbindungen zurückgreifen.

In einer weiteren Ausführungsform werden weitere Schichten zwischen die emittierende Schicht und eine der beiden Elektroden eingeführt.

Es ist vorteilhaft, wenn wenigstens eine Sperrschicht zwischen EML und einer der Elektroden verwendet wird. Hierdurch können insbesondere die Betriebsspannung reduziert und die absoluten Strahlungsintensitäten erhöht werden. Geeignete Sperrschichten können Exzitonen, Elektronen oder Löcher blockieren.

Daher betrifft die vorliegende Erfindung auch eine Vorrichtung, wie hierin offenbart, die eine weitere Schicht zwischen der emittierenden Schicht und einer der zwei Elektroden enthält, dadurch charakterisiert, dass die zusätzliche Schicht ein Exzitonen-blockierendes Material (Sperrmaterial) mit einer Bandlücke von 3.4 eV oder höher, bevorzugt von 3.6 eV oder höher, ganz bevorzugt von 3.8 eV oder höher und ganz besonders bevorzugt von 4.0 eV oder höher enthält.

Die vorliegende Erfindung betrifft auch eine Vorrichtung, wie hierin offenbart, die eine weitere Schicht zwischen der emittierenden Schicht und einer der zwei Elektroden enthält, dadurch charakterisiert, dass die zusätzliche Schicht ein Loch-blockierendes Material (Sperrmaterial) mit einem HOMO von niedriger als -5.9 eV, bevorzugt niedriger als -6.0 eV, ganz bevorzugt niedriger als -6.2 eV und ganz besonders bevorzugt niedriger als -6.3 eV, enthält.

Die vorliegende Erfindung betrifft auch eine Vorrichtung, wie hierin offenbart, die eine weitere Schicht zwischen der emittierenden Schicht und einer der zwei Elektroden enthält, dadurch charakterisiert, dass die zusätzliche Schicht ein Elektronen-blockierendes Material (Sperrmaterial) mit einem LUMO von höher als -2.2 eV, vorzugsweise höher als -2.1 eV enthält.

In einer ganz bevorzugten Ausführungsform enthält die erfindungsgemäße Vorrichtung eine Sperrschicht, die sowohl Exzitonen als auch Löcher blockiert.

In einer weiteren ganz bevorzugten Ausführungsform enthält die erfindungsgemäße Vorrichtung eine Sperrschicht, die sowohl Exzitonen als auch Elektronen blockiert.

Als Sperrmaterial in der Sperrschicht können alle Materialien eingesetzt werden, die die oben genannten Kriterien erfüllen. Dazu gehören auch die Verbindungen der allgemeinen Formel (1).

Vorzugsweise ist das Sperrmaterial eine Verbindung der allgemeinen Formeln (19) oder (22).

In einer geeigneten Ausführungsform wird die Sperrschicht durch Vernetzung einer oder mehrerer Verbindungen enthaltend wenigstens 2 oder mehr vernetzbare Gruppe (hiernach Precursor), gebildet. Insbesondere bevorzugt ist eine Sperrschicht, die durch Precursor der Verbindung gemäß Formel (1) gebildet wird, wobei n = 0 gilt, oder Formel (19) oder Formel (22), die weiterhin wenigstens 2 oder mehr vernetzbare Gruppe enthalten.

Eine vernetzbare Gruppe ist eine Gruppe umfassend ein vernetzbares Reagenz, was zu einer Vernetzungsreaktion mit Hilfe von Wärme, Strahlung oder beidem führt. Die Strahlungsquelle kann ein Elektronenstrahl und UV-Strahlung sein. Die bevorzugte UV-Strahlungsquelle emittiert Strahlung einer Wellenlänge von 200 bis 400 nm, ganz bevorzugt ist eine Strahlung von 300 bis 400 nm. Geeignete Quellen für UV-Strahlung sind, beispielsweise, Quecksilber UV Fluoreszenz-Lampen, UV-LEDs und UV-Laserdioden.

Geeignete vernetzbare Gruppen sind beispielsweise die Acrylat-Gruppe (z.B. Scheler et al., In Macromol. Symp.. 254, 203-209 (2007)), die Vinyl- oder Styrolgruppe (z.B. WO 2006/043087) und die Oxetangruppe (z.B. Mueller et al., In Nature 421, 829-833 (2003)).

In einer bevorzugten Ausführungsform ist die Precursor Verbindung für die Sperrschicht eine Verbindung der allgemeinen Formel (231).

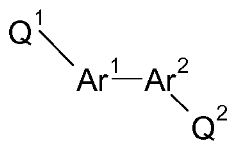

Formel (231)

wobei und $Ar^2$ wie oben definiert sind, und $Q^1$ und $Q^2$ unabhängig voneinander jeweils eine vernetzbare Gruppe ist, welche vorzugsweise aus den folgenden Formel (232) bis (255) ausgewählt wird:

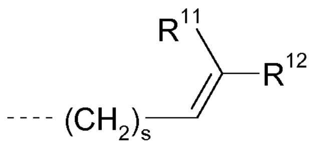

Formel (232)

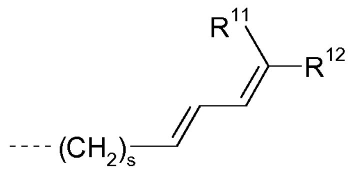

Formel (233)

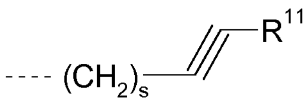

Formel (234)

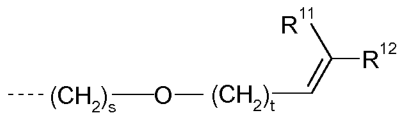

Formel (235)

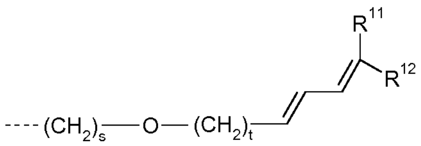

Formel (236)

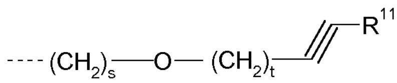

Formel (237)

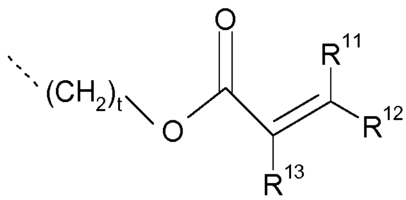

Formel (238)

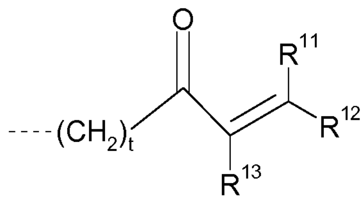

Formel (239)

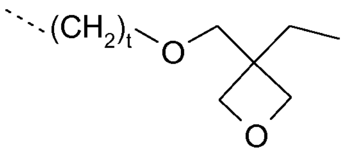

Formel (240)

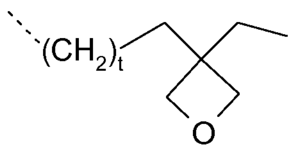

Formel (241)

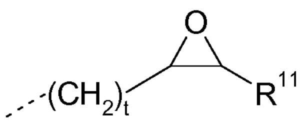

Formel (242)

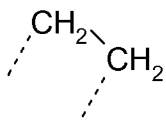

Formel (243)

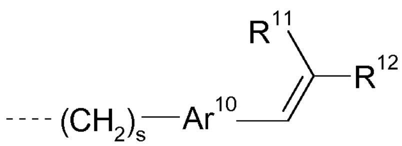

Formel (244)

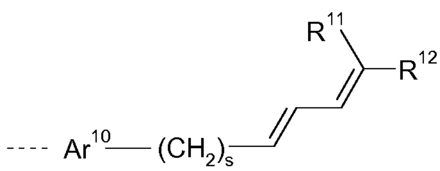

Formel (245)

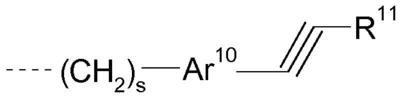

Formel (246)

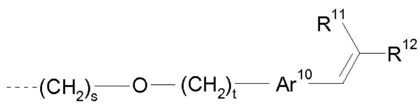

Formel (247)

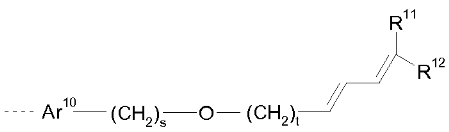

Formel (248)

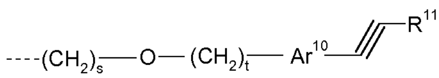

Formel (249)

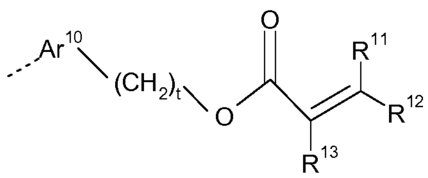

Formel (250)

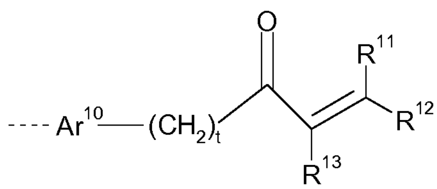

Formel (251)

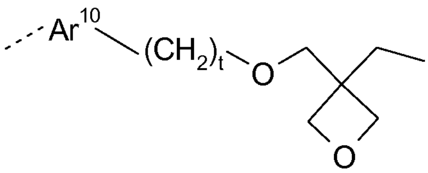

Formel (252)

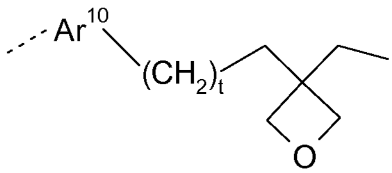

Formel (253)

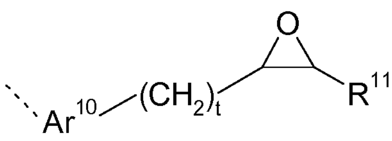

Formel (254)

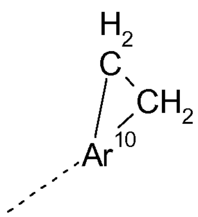

Formel (255),

wobei
die Reste $R^{11}$, $R^{12}$ und $R^{13}$ bei jedem Auftreten, gleich oder verschieden, H, eine geradkettige oder verzweigte Alkylgruppe mit 1 bis 6 C-Atomen sind;
$Ar^{10}$ in den Formeln (244) bis (255) ist ein mono- oder polycyclisches, aromatisches oder hetero-aromatisches Ringsystem mit 5 oder 6 Ringatomen, das mit einem oder mehreren Resten R substituiert sein kann, wobei R bei jedem Auftreten gleich oder verschieden H, D, F oder ein aliphatischer, aromatischer und/oder heteroaromatischer Kohlenwasserstoffrest mit 1 bis 20 C-Atomen ist, in dem auch ein oder mehrere H Atome durch F ersetzt sein können; wobei zwei oder mehrere Substituenten R auch miteinander ein mono- oder polycyclisches, aliphatisches oder aromatisches Ringsystem bilden können;
s ist eine ganze Zahl von 0 bis 8;
t ist eine ganze Zahl von 1 bis 8;
und wobei die gestrichelte Bindung die Anknüpfung der vernetzbaren Gruppe an eines der mono- oder polycyclischen, aromatischen oder heteroaromatischen Ringsysteme oder $Ar^2$ in Formel (231) darstellen.

In der Gruppe der Formel (243) bedeuten die beiden gestrichelten Linien, dass und/oder $Ar^2$ in der Verbindung der Formel (231) in ortho-Position mit den beiden Kohlestoffatomen der Ethylengruppe verbunden sind, so dass ein viergliedriger Ring entsteht. Analog ist $Ar^{10}$ in der Gruppe der Formel (255) mit den beiden Kohlenstoffatomen der Ethylengruppe in ortho-Position verbunden, so dass ein viergliedriger Ring entsteht.

Beispiele für bevorzugte Precursor Verbindungen für die Sperrschicht gemäß den oben aufgeführten Ausführungsformen sind die Verbindungen der folgenden Strukturen.

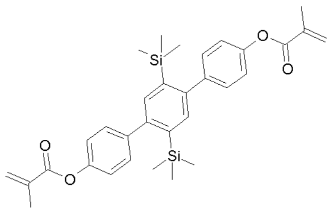
Formel (256)

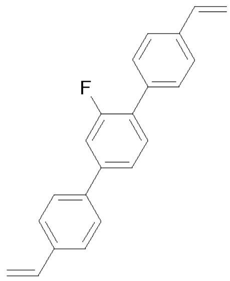
Formel (257)

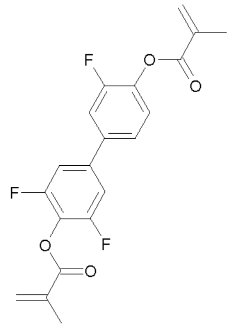
Formel (258)

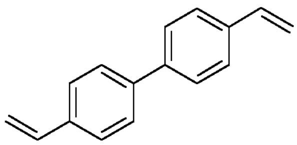
Formel (259)

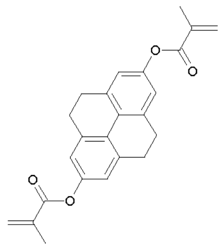
Formel (260)

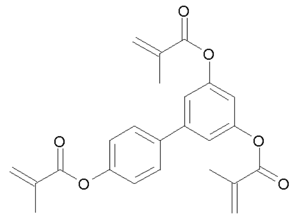
Formel (261)

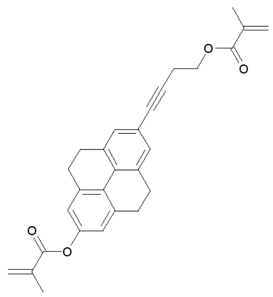
Formel (262)

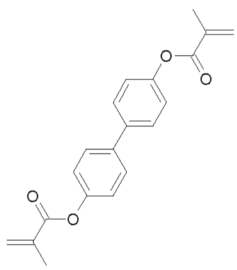
Formel (263)

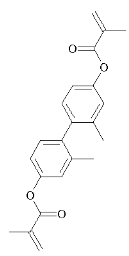
Formel (264)

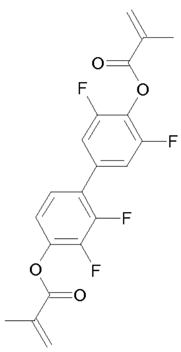
Formel (265)

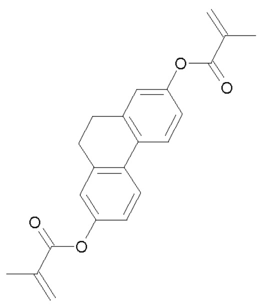
Formel (266)

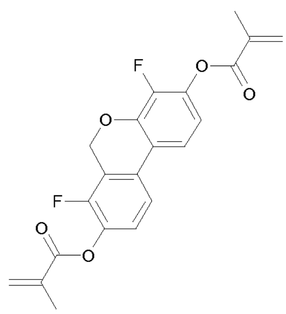
Formel (267)

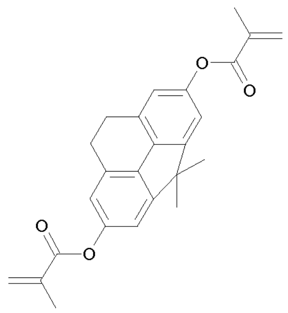

Formel (268)

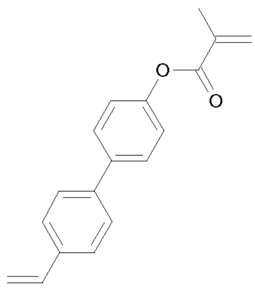

Formel (269)

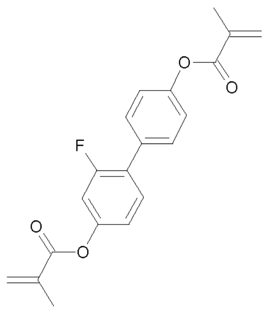

Formel (270)

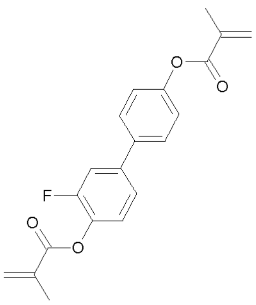

Formel (271)

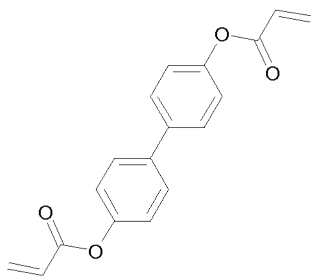

Formel (272)

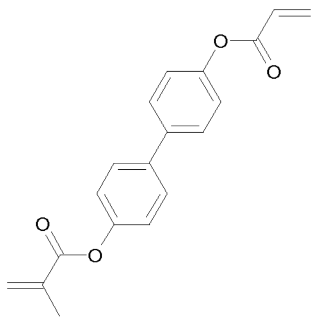

Formel (273)

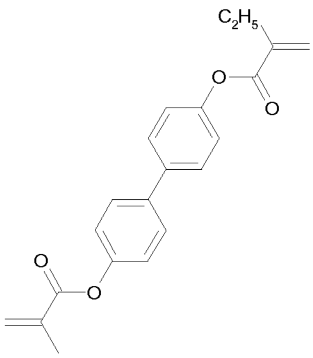

Formel (274)

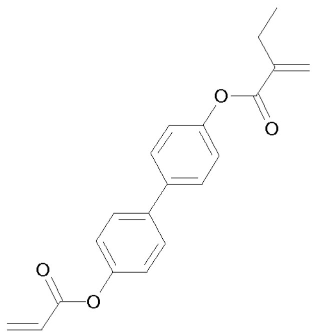

Formel (275)

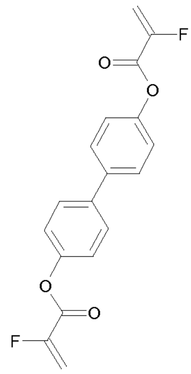

Formel (276)

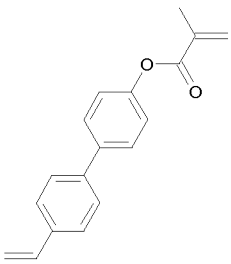

Formel (277)

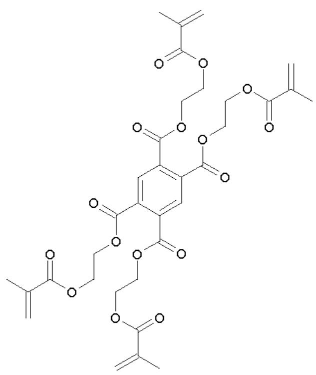

Formel (278).

Verfahren zur Herstellung der genannten Precursor Verbindungen sind dem Fachmann aus dem Stand der

Technik gut bekannt (z.B. WO 2010/133278 und US 7807068).

Die Elektrolumineszenzvorrichtung kann jede Elektrolumineszenzvorrichtung sein. Der Fachmann kann hierbei ohne Schwierigkeiten aus einer großen Anzahl ihm bekannter Vorrichtungen auswählen. Bevorzugt handelt es sich bei der Elektrolumineszenzvorrichtung um eine organische lichtemittierende Diode (OLED), polymere lichtemittierende Diode (PLED), organische lichtemittierende elektrochemische Zelle (OLEC, LEC oder LEEC), einen organischen lichtemittierenden Transistor (O-LETs) und einen organischen lichtemittierenden elektrochemischen Transistor. In einer ganz bevorzugten Ausführungsform betrifft die vorliegende Erfindung OLEDs oder PLEDs. In einer weiterhin ganz bevorzugten Ausführungsform betrifft die vorliegende Erfindung OLECs.

Die Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Die Elektrolumineszenzvorrichtung kann eine emittierende Schicht enthalten, oder sie kann mehrere emittierende Schichten enthalten, wobei bevorzugt ist, wenn sie eine emittierende Schicht enthält.

In einer bevorzugten Ausführungsform enthält die erfindungsgemäße Elektrolumineszenzvorrichtung eine Lochinjektionsschicht, die auch Pufferschicht (Buffer Layer) genannt wird. Die Austrittsarbeit der Lochinjektionsschicht ist größer als 5.0 eV, bevorzugt größer als 5.4 eV, ganz bevorzugt größer als 5.8eV und ganz besonders bevorzugt größer als 6.0 eV. In einer weiteren Ausführungsform enthält die Lochinjektionsschicht leitfähige, konjugierte Polymere, wie zum Beispiele Polythiophen, Polyanilin und Polypyrrol und deren Derivate. Solche Polymere werden teilweise auch kommerziell angeboten, wie beispielsweise CLEVIOS™ P VP Al 4083, CLEVIOS™ HIL 1.3, und CLEVIOS™ HIL 1.3N von Heraeus Precious Metals GmbH & Co. KG.

Die Verbindungen gemäß Formel (1) können auch in die Seitenkette von Polymeren eingebaut werden. Der Einbau der Verbindungen in die Seitenkette von Polymeren hat verschiedene Vorteile, die im Folgenden aufgeführt werden.

1) Die Polymere weisen eine verbesserte Löslichkeit in organischen Lösungsmitteln und somit auch eine verbesserte Prozessierbarkeit auf.
2) Die Polymere weisen verbesserte Schichtbildungseigenschaften auf.
3) Die Polymere haben höhere Glasübergangstemperaturen (Tg) im Vergleich zu kleinen Molekülen.
4) Die Polymere weisen ein breiteres Prozess-Fenster und verbesserte Leistungsdaten auf.

Geeignet ist ein Polymer der allgemeinen Formel (279),

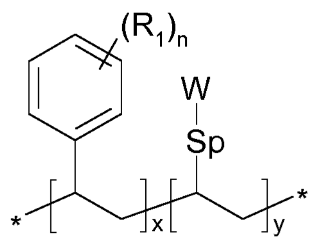

Formel (279),

wobei für die verwendeten Indizes und Symbole gilt:

Sp ist eine Einfachbindung oder ein nicht-konjugierter Spacer;

W ist gleich oder verschieden bei jedem Auftreten eine Struktureinheit gemäß Formel (1), wobei die Bindung zwischen Sp und der Verbindung der Formel (1) an jeder beliebigen und chemisch mögliche Position erfolgen kann;

x ist eine Zahl zwischen 0 und 80 und steht für mol% der jeweiligen Einheit;

y ist eine Zahl von 2 bis 100 und steht für mol% der jeweiligen Einheit, wobei x+y=100 mol% gilt;

n ist eine ganze Zahl von 0 bis 5;

$R^1$ ist wie $R^3$ in Formel (1) definiert;

wobei das Polymer bevorzugt eine Photolumineszenz- und/oder Elektrolumineszenzemission im Wellenlängenbereich von 280 und 380 nm aufweist.

In einer weiteren bevorzugten Ausführungsform emittiert das Polymer sowohl Strahlung im Wellenlängenbereich von 280 bis 380 nm als auch Strahlung im Wellenlängenbereich von 400 bis 500 nm.

In einer bevorzugten Ausführungsform ist der Spacer ein Kohlenwasserstoffrest mit 1 bis 20 C-Atomen, wobei Alkyl- und Alkylalkoxyreste bevorzugt sind.

In einer bevorzugten Ausführungsform hat das Polymer die generelle Formel (280),

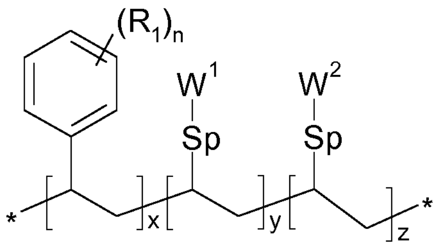

Formel (280),

wobei für die verwendeten Indizes und Symbole gilt:

x ist eine Zahl zwischen 0 und 80 mol%;

y ist eine Zahl von 19 bis 80 mol%;

z ist eine Zahl von 1 bis 20 mol%, wobei x+y+z=100 mol% gilt;

$W^1$ ist ausgewählt aus den Verbindungen der Formeln (19), (22), (61), (64), (89-2), (89-4);

$W^2$ ist ausgewählt aus den Verbindungen der Formeln (20), (21), (23), (24), (25), (26), (62), (63), (65), (66), (67), (68), (89-1), (89-3), (89-5) und (89-6);

und wobei die anderen Symbole und Indizes wie in Formel (279) angegeben definiert sind.

In einer bevorzugten Ausführungsform ist Sp eine Einfachbindung. Ganz bevorzugt ist, wenn die aromatischen oder heteroaromatischen Ringe von W, $W^1$ oder $W^2$ direkt an das Polymer-Rückgrat (Backbone) gebunden sind.

In einer ganz besonders bevorzugten Ausführungsform ist das Polymer eine der folgenden allgemeinen Verbindungen,

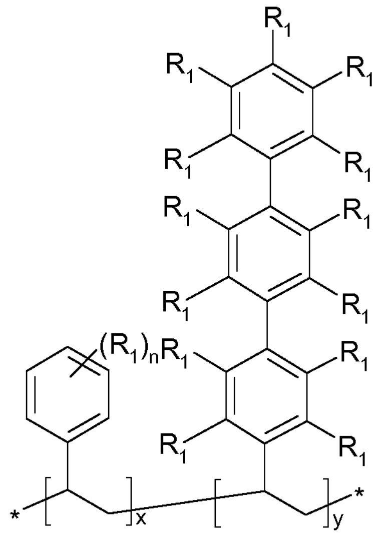

Formel (281)

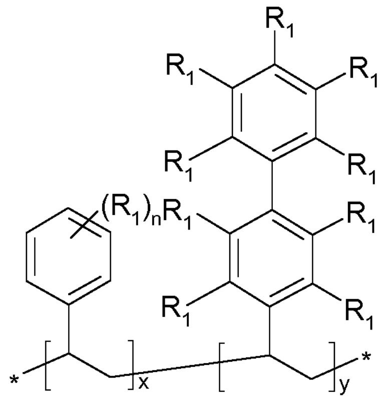

Formel (282)

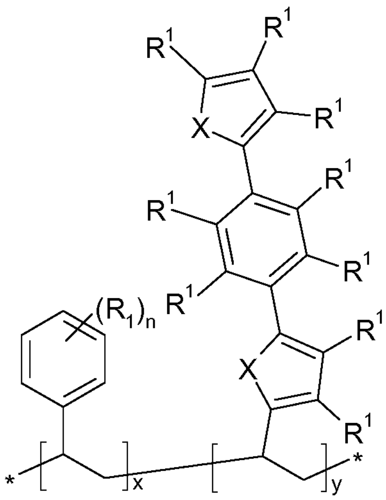

Formel (283)

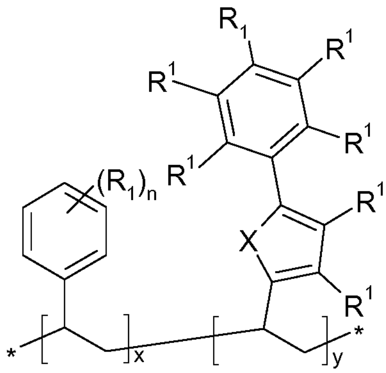

Formel (284)

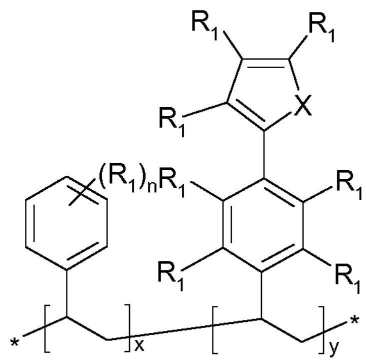

Formel (285)

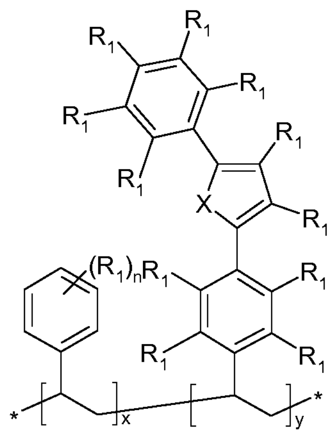

Formel (286)

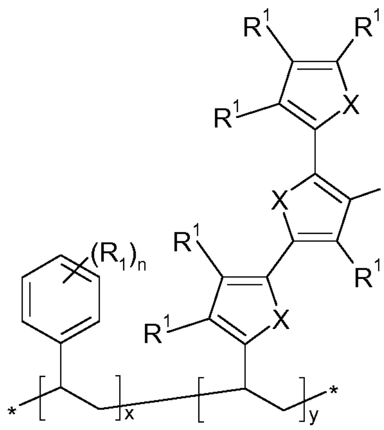

Formel (287).

wobei X und $R^1$ wie in Formel (89-1) bis (89-6) definiert sind und wobei die im Zusammenhang mit der Verbindung der Formel (1) aufgeführten bevorzugten Ausführungsformen für $R^1$ auch bevorzugte Ausführungsformen für $R^1$ im Zusammenhang mit dem Polymer darstellen.

Folgende allgemeine Synthesevorschriften können zur Herstellung von Polymeren der Formel (279) verwendet werden:

Syntheseroute 1:

Radikalische Polymerisation für Polymere der Formeln (281) bis (287).

Die Monomere werden im gewünschten Verhältnis in einen Kolben eingewogen und sorgfältig inertisiert. Es werden 10 Äquivalente der Gesamt-Monomer-Menge an Toluol zugegeben und die Lösung erneut inertisiert. In einem zweiten Kolben werden 0,01 Äquivalente der Gesamt-Monomer-Menge AIBN eingewogen und in der zehnfachen Molmenge Toluol unter leichtem Erwärmen gelöst. Die Monomer-Lösung wird auf 70°C erhitzt und ein Prozent der Toluol-AIBN-Lösung wird per Spritze schnell zugefügt. Unter Lichtausschluss wird die Lösung für 72 Stunden bei 70°C gerührt, dann auf Raumtemperatur abgekühlt und weitere 24 Stunden gerührt. Das Polymer wird zweimal aus Toluol in Ethanol gefällt, abfiltriert und im Hochvakuum für 24 Stunden getrocknet.

Syntheseroute 2:

Anionische Polymerisation für Polymere der Formeln (281) bis (287).

In einen getrockneten Kolben werden 32 Äquivalente der Gesamt-Monomer-Menge frisch getrocknetes und destilliertes Cyclohexan gegeben und mit 0,002 ÄEquivalenten der Gesamt-Monomer-Menge 2-Butyllithium in Hexan (1.4 M) versetzt. Die Lösung wird auf 45°C erwärmt und die Monomere im gewünschten Verhältnis schnell hinzugegeben. Die Reaktionsmischung wird bei der Temperatur zwischen 4 Minuten und 10 Stunden gerührt, danach in eine Lösung von entgastem Methanol getropft. Das Polymer wird zweimal aus Toluol in Ethanol gefällt, abfiltriert und im Hochvakuum für 24 Stunden getrocknet.

Syntheseroute 3:

Kationische Polymerisation für Polymere der Formeln (281) bis (287).

In einem Kolben werden die Monomere, 1-Phenylethylchlorid (0,043_Eq) und Dibutylether (0,34 Eq) vorgelegt und mit dem zehnfachen Volumen einer Mischung aus 1,2-Dichlorethan und n-Hexan (55:45 v/v) versetzt. Die Reaktionsmischung wird auf -15°C abgekühlt und eine Lösung von Titan(IV)chlorid in 1,2-Dichlorethan (4 M, 0.172 Eq) zugegeben. Die Reaktionsmischung wird bei der Temperatur zwischen 4 Minuten und 10 Stunden gerührt, danach in eine Lösung von entgastem Ethanol getropft. Der Feststoff wird mit 0,5 M Salpetersäure und deionisiertem Wasser mehrfach gewaschen. Das Polymer wird zweimal aus Toluol in Ethanol gefällt, abfiltriert und im Hochvakuum für 24 Stunden getrocknet.

Die vorliegende Erfindung betrifft auch Zusammensetzungen enthaltend wenigstens eine der Verbindungen nach Formel (89-1) und/oder (89-2) sowie wenigstens ein organisch funktionelles Material oder einen organischen Halbleiter ausgewählt aus der Gruppe der Emitter, Host Materialien, Matrix Materialien, Elektronentransportmaterialien (ETM), Elektroneninjektionsmaterialien (EIM), Lochtransportmaterialien (HTM), Lochinjektionsmaterialien (HIM), Elektronenblockiermaterialien (EBM), Lochblockiermaterialien (HBM), Exzitonenblockiermaterialien (ExBM), wobei für die angegebenen Symbole die Definitionen aus dem Anspruch 1 gelten. Die Emitter können dabei sowohl fluoreszierende als auch phosphoreszierende Emitter sein. Der Fachmann kann dabei ohne Schwierigkeiten aus einer Vielzahl bekannter organischer funktioneller Materialien mit den genannten Funktionen auswählen. Die Definitionen und Beispiele für unterschiedliche organische funktionelle Materialien kann man beispielsweise der Offenbarung von WO 2011/015265 entnommen werden.

Es ist bevorzugt im Sinne der vorliegenden Erfindung, wenn die erfindungsgemäße Zusammensetzung neben wenigstens einer Verbindung der Formel (89-1) und/oder (89-2) als Emitter wenigstens ein Host Material als organisch funktionelles Material enthält. Ganz bevorzugt enthält die Zusammensetzung neben der wenigstens einen Verbindung der Formel (89-1) und/oder (89-2) als Emitter zwei Host Materialien. Ganz besonders bevorzugt enthält die Zusammensetzung genau eine Verbindung nach Anspruch 1 als Emitter und zwei Host Materialien. Weiterhin ganz besonders bevorzugt enthält die Zusammensetzung genau eine Verbindung nach Anspruch 1 als Emitter sowie genau ein Host Material.

In einer weiterhin bevorzugten Ausführungsform enthält die erfindungsgemäße Zusammensetzung neben wenigstens einer Verbindung nach Anspruch 1 als Host wenigstens einen Emitter als organisch funktionelles Material. Ganz bevorzugt enthält die Zusammensetzung neben de wenigstens zwei Verbindungen nach Anspruch 1 als Host einen Emitter.

Die Konzentration von Emitter(n) in der Zusammensetzung beträgt 2 bis 50 wt% (Gewichtsprozent), bevorzugt 5 bis 40 wt% und ganz bevorzugt 7 bis 30 wt%. Die Gesamtkonzentration des Hosts bzw. der Hostmaterialien beträgt 50 bis 98 wt%, bevorzugt 95 bis 60wt% und ganz bevorzugt 93 bis 70 wt%.

Die erfindungsgemäßen Vorrichtungen können nach verschiedenen Verfahren hergestellt werden. Eine oder mehrere der Schichten der Elektrolumineszenzvorrichtung können_mit einem Sublimationsverfahren aufgetragen werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Es ist aber auch möglich, dass der Anfangsdruck noch geringer ist, beispielsweise kleiner $10^{-7}$ mbar.

Ein bevorzugtes Verfahren zur Auftragung einer oder mehrere Schichten der Elektrolumineszenzvorrichtung ist das OVPD-Verfahren (Organic Vapour Phase Deposition) oder ein Verfahren mit Hilfe einer Trägergassublimation. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Eine oder mehrere der Schichten der Elektrolumineszenzvorrichtung können auch aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, aufgetragen werden. Hierfür sind lösliche Verbindungen nötig, welche beispielsweise durch geeignete Substitution erhalten werden. Diese Verfahren eignen sich auch für die Auftragung von Schichten mit Oligomeren, Dendrimeren und Polymeren.

Ebenso sind Hybridverfahren möglich, bei denen beispielsweise eine oder mehrere Schichten aus Lösung aufgebracht werden und eine oder mehrere andere Schichten im Vakuum aufgedampft werden.

Die vorliegende Beschreibung betrifft daher auch ein Verfahren zur Herstellung der erfindungsgemäßen Elektrolumineszenzvorrichtungen mittels Sublimationsverfahren und/oder mittels Verfahren aus Lösung.

Die vorliegende Erfindung betrifft weiterhin eine Formulierungen enthaltend eine erfindungsgemäße Zusammensetzung nach Anspruch 8 sowie ein oder mehrere Lösungsmittel.

Geeignete und bevorzugte Lösungsmittel sind beispielsweise Toluol, Anisol, Xylole, Methylbenzoat, Dimethylanisole, Trimethylbenzole, Tetralin, Veratrole, Tetrahydrofuran, Chlorbenzol oder Dichlorbenzole sowie Gemische derselben.

Elektrolumineszierende Vorrichtungen, die blaues Licht und/oder UV Strahlung emittieren sind vielseitig einsetzbar. Anwendungen, die Licht bzw. Strahlung mit sehr kurzen Wellenlängen erfordern und somit Anwendungsgebiete für die erfindungsgemäßen Vorrichtungen darstellen findet man, zum Beispiel, im Bereich Life Science und Medizin (z.B. für Cell Imaging) oder im Bereich der Biosensoren. Weiterhin finden die erfindungsgemäßen Vorrichtungen in der Elektronikindustrie, dem Solid-State Lighting und für die Aushärtung von Polymeren und Druck-Tinte Anwendung. Die vorliegende Beschreibung betrifft daher auch die Verwendung der erfindungsgemäßen Elektrolumineszenzvorrichtungen in den genannten Bereichen.

Die erfindungsgemäßen Vorrichtungen können auch zur Lichttherapie (Phototherapie) von Menschen und/oder Tieren eingesetzt werden. Noch ein weiterer Gegenstand der vorliegenden Beschreibung bezieht sich auf die Verwendung, der erfindungsgemäßen Vorrichtungen zur Behandlung und Prophylaxe kosmetischen Umstände mittel Phototherapie.

Phototherapie oder Lichttherapie findet in vielen medizinischen und/oder kosmetischen Bereichen Anwendung. Die erfindungsgemäßen Vorrichtungen können daher zur Therapie und/oder Prophylaxe und/oder Diagnose von allen Erkrankungen und/oder in kosmetischen Anwendungen eingesetzt werden, für die der Fachmann die Anwendung von

Phototherapie in Betracht zieht. Der Begriff Phototherapie beinhaltet dabei neben der Bestrahlung auch die photodynamischen Therapie (PDT) sowie das Konservieren, Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser, Trinkwasser, Bestecke, medizinische Bestecke und Geräte sowie andere Vorrichtungen.

Der Begriff Phototherapie beinhaltet auch jede Art der Kombination von Lichttherapie und anderen Therapiearten, wie bspw. die Behandlung mit Wirkstoffen. Viele Lichttherapien haben zum Ziel, äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt sind die Behandlung und/oder Prophylaxe von Psoriasis, Akne, Cellulite, Hautfaltenbildung, Hautalterung, Ikterus und Vitiligo.

Weitere Anwendungsgebiete für die Vorrichtungen sind ausgewählt aus der Gruppe der Entzündungs-erkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. - Veränder ungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt sind die Behandlung und/oder Prophylaxe von rheumatoider Arthritis, viraler Infektionen, und Schmerzen.

Weitere Anwendungsgebiete für die Vorrichtungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere Anwendungsgebiete für die Vorrichtungen sind ausgewählt aus der Gruppe der Desinfektionen. Mit den erfindungsgemäßen Vorrichtungen können jegliche Art von Objekten (unbelebte Materie) oder Subjekten (lebende Materie wie bspw. Mensch und Tier) zum Zweck der Desinfektion, Sterilisation oder Konservierung behandelt werden. Hierzu zählt, zum Beispiel, die Desinfektion von Wunden, die Reduktion von Bakterien, das Desinfizieren chirurgischer Instrumente oder anderer Gegenstände, das Desinfizieren oder Konservieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Die erfindungsgemäßen Vorrichtungen emittieren insbesondere im UV- und Blau- Bereich des Spektrums. Die genaue Wellenlänge zu größeren Wellenlängen hin kann in Abhängigkeit von der jeweiligen Anwendung ohne Schwierigkeiten von dem Fachmann eingestellt werden.

In einer besonders bevorzugten Ausführungsform der vorliegenden Erfindung ist die Vorrichtung eine organische lichtemittierende Diode (OLED) oder eine organische lichtemittierende elektrochemische Zelle (OLEC), die zum Zwecke der Phototherapie eingesetzt werden. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und - wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken,Textilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen im energiereichen Blaubereich und/oder im UV Bereich geringerer Bestrahlungsintensitäten an nahezu jedem Ort und zu jeder Tageszeit möglich sind. Die Bestrahlungen können stationär, ambulant und/oder selbst, d.h., ohne Ein- und/oder Anleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder mehrmaligen Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Eine Arylgruppe im Sinne dieser Offenbarung enthält 6 bis 40 C-Atome; eine Heteroarylgruppe im Sinne dieser Offenbarung enthält 1 bis 39 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Offenbarung enthält 6 bis 60 C-Atome im Ringsystem. Ein heteroaromatisches Ringsystem im Sinne dieser Offenbarung enthält 1 bis 59 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Offenbarung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verknüpft sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Offenbarung verstanden werden, und ebenso Systeme, in denen zwei oder mehrere Arylgruppen beispielsweise durch eine kurze Alkylgruppe unterbrochen sind. Weiterhin sollen Systeme, in denen mehrere Aryl- und/oder Heteroarylgruppen durch eine Einfachbindung miteinander verknüpft sind, wie z. B. Biphenyl, Terphenyl oder Bipyridin, als aromatisches bzw. heteroaromatisches Ringsystem verstanden werden.

Im Rahmen der vorliegenden Offenbarung werden unter einem aliphatischen Kohlenwasserstoffrest bzw. einer Alkylgruppe bzw. einer Alkenyl- oder Alkinylgruppe, die typischerweise 1 bis 40 oder auch 1 bis 20 C-Atome enthalten kann, und in der auch einzelne H-Atome oder $CH_2$-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Offenbarung geradkettig, verzweigt oder cyclisch sein, wobei eine oder mehrere nicht-benachbarte $CH_2$-Gruppen durch $R^1C=CR^1$, C=C, $Si(R^1)_2$, $Ge(R^1)_2$, $Sn(R^1)_2$, C=O, C=S, C=Se, $C=NR^1$, $P(=O)(R^1)$, SO, $SO_2$, $NR^1$, O, S oder $CONR^1$ ersetzt sein können; weiterhin können auch ein oder mehrere H-Atome durch D, F, Cl, Br, I, CN oder $NO_2$, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten $R^1$ oder einem Kohlenwasserstoffrest substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einer Aryloxygruppe gemäß der Definition der vorliegenden Offenbarung wird eine Arylgruppe, wie oben definiert, verstanden, welche über ein Sauerstoffatom gebunden ist. Eine analoge Definition gilt für Heteroaryloxygruppen.

Ein aromatisches Ringsystem im Sinne dieser Offenbarung enthält 6 bis 60 C-Atome im Ringsystem. Ein heteroaromatisches Ringsystem im Sinne dieser Offenbarung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Offenbarung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein $sp^3$-hybridisiertes C-, Si-, N- oder O-Atom, ein $sp^2$-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Offenbarung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Offenbarung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio,

n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Die erfindungsgemäßen Vorrichtungen, Zusammensetzungen und Formulierungen zeichnen sich durch folgende überraschende Vorteile gegenüber dem Stand der Technik aus:

1. Die erfindungsgemäßen Vorrichtungen emittieren im UV-A und UV-B Bereich.

2. Die für die bevorzugte Emission benötigten Emitterverbindungen sind leicht zugänglich.

3. Durch die Verwendung von Mixed Hosts kann die Betriebsspannung erniedrigt und die Strahlungsintensität erhöht werden.

4. Durch die Verwendung von Sperrschichten kann die Betriebsspannung deutlich erniedrigt und die Strahlungsintensität erhöht werden.

5. Die erfindungsgemäßen Vorrichtungen lassen sich leicht aus Lösungen prozessieren.

Diese oben genannten Vorteile gehen nicht mit einer Verschlechterung der weiteren elektronischen Eigenschaften einher.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit anderen Beispielen kombiniert werden.

Die Erfindung wird durch die nachfolgenden Beispiele und die Abbildungen 1 bis 20 näher erläutert, ohne sie dadurch einschränken zu wollen.

Kurzbeschreibung der Abbildungen:

Abbildungen 1 bis 16 zeigen die Elektrolumineszenz- (EL) sowie die entsprechenden Photolumineszenzspektren (PL) der OLEDs 1 bis 16.

Abbildung 17 zeigt die EL Spektren und Betriebsspannungen von OELD1 und OLED17 im Vergleich

Abbildung 18 zeigt Ergebnisse aus quantenchemische Berechnungen an BM1 und BM1b.Abb

Abbildung 19 zeigt die EL-Spektren und Betriebsspannungen von OLED17 (ohne Sperrschicht) und OLED18 (mit Sperrschicht)

Abbildung 20 zeigt das EL- und PL-Spektrum von OLED20.

Abbildung 21 zeigt die Absorptions- und Photolumineszenzspektren von Polymer P1.

Abbildung 22 zeigt das Elektrolumineszenzspektrum von OLED21.

Abbildung 23 zeigt das Elektrolumineszenzspektrum von OLED22.

Beispiele

Beispiel 1

Materialien

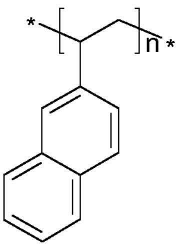

Ref1

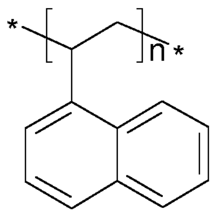

Ref2

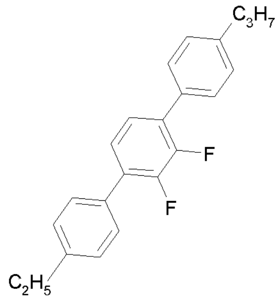

E1

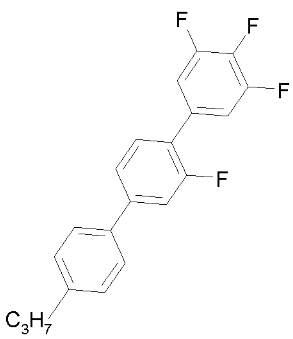

E2

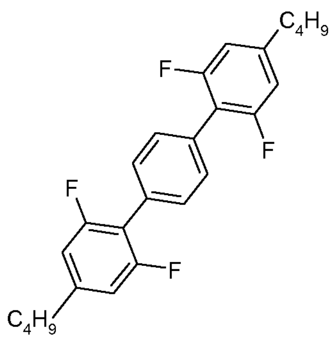

E3

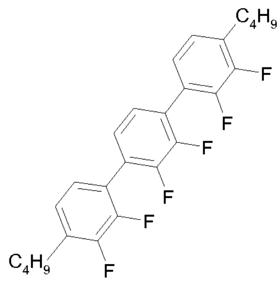

E4

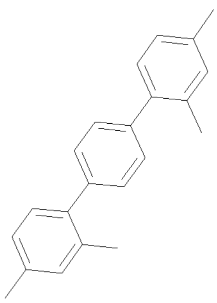

E6*

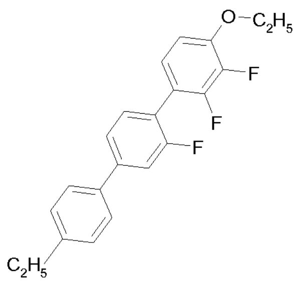

E7

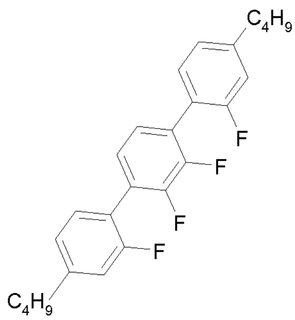

E8

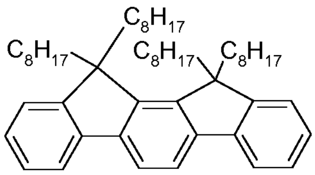

E9*

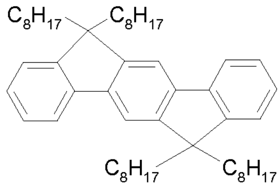

E10*

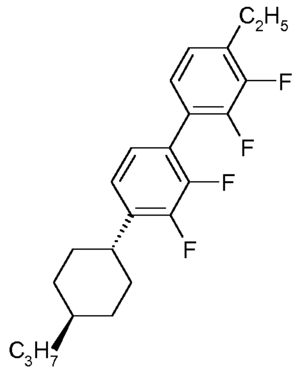

E11*

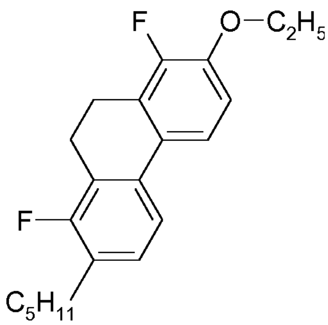

E12*

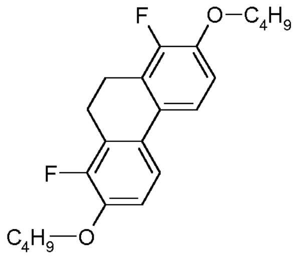

E13*

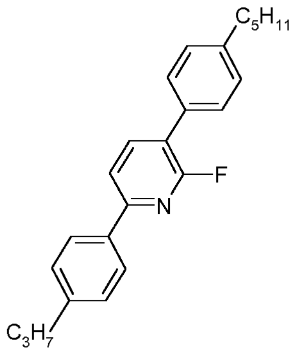

E14*

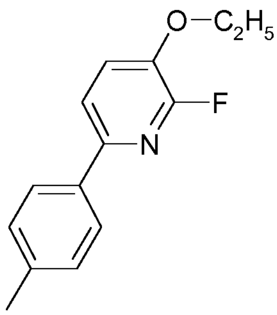

E15*

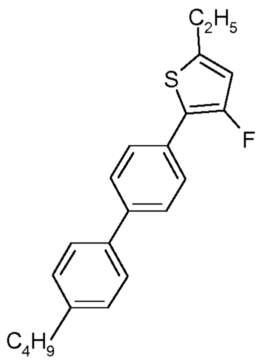

E16*

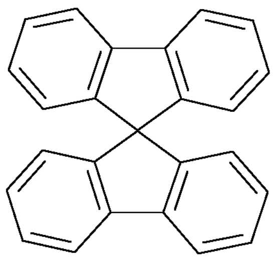

E17*

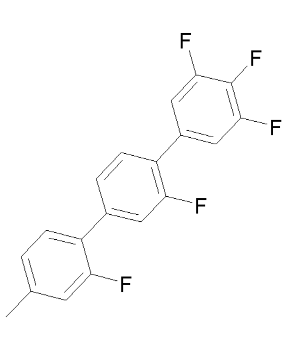

E20

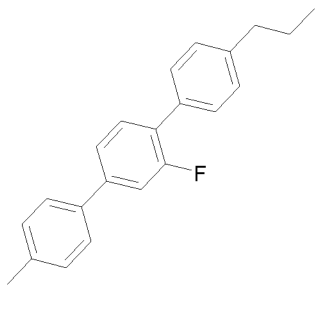

E21

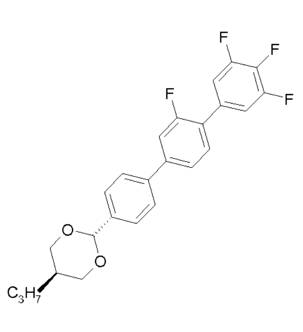

E22*

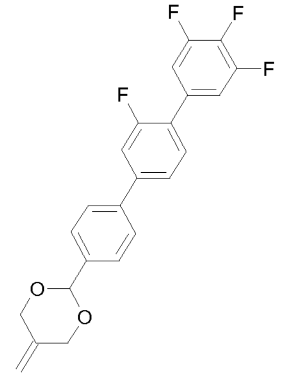
E23*

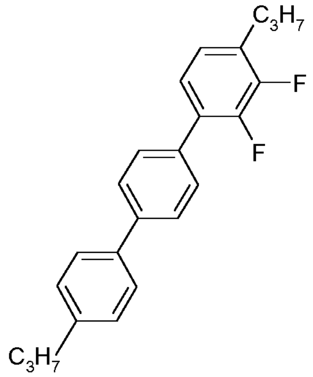
E24

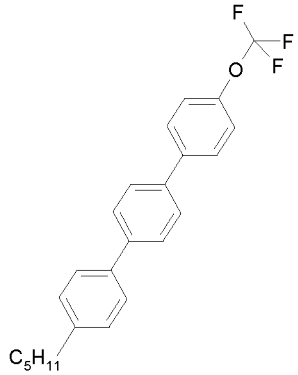
E25*

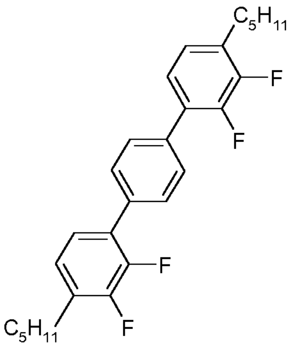
E26

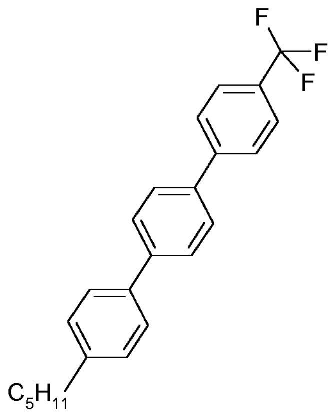
E27

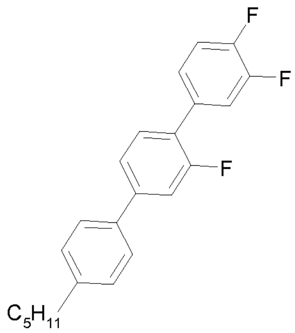
E28

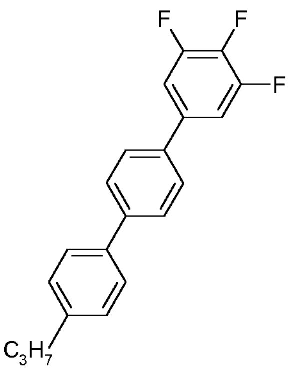
E29

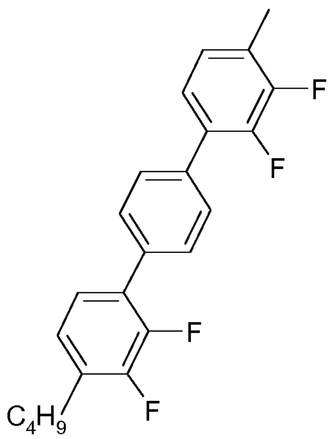
E30

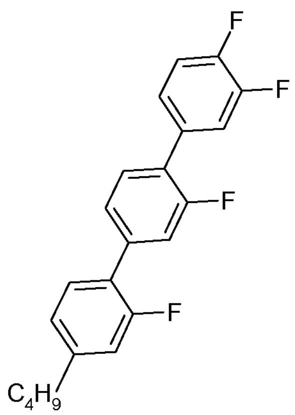
E31

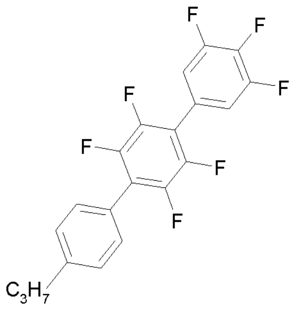

E32

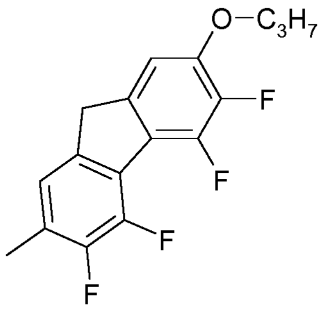

E33*

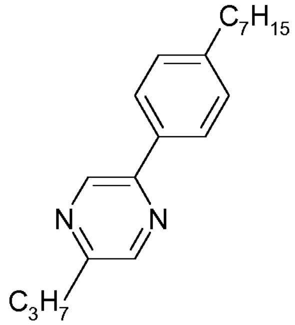

E34*

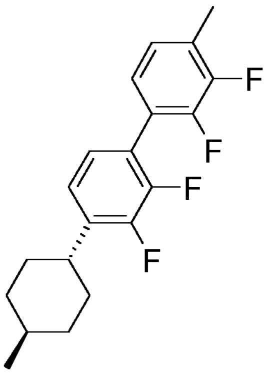

H1

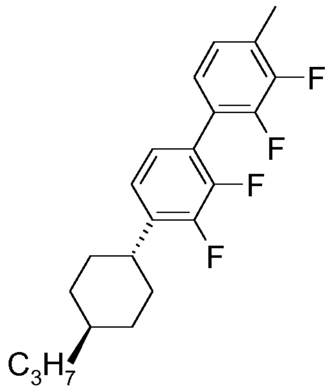

H2

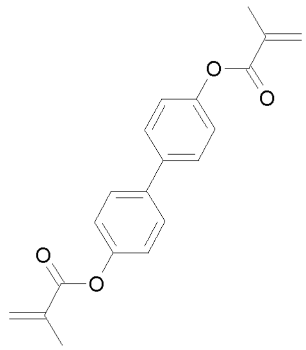

BM1

* Nicht erfindungsgemäße Emitter

E1 bis E34 sind Verbindungen gemäß Formel (1). Ref1 (Poly(2-vinylnaphthalene)) und Ref2 (Poly(1-vinylnaphthalene)) sind Referenzverbindungen und können von Sigma-Aldrich erworben werden. H1 und H2 sind Hostverbindungen. BM1 ist ein Sperrmaterial.

Zusätzlich wird Polystyrol (PS) von Fulka mit einem Molekulargewicht Mw von 200k Dalton verwendet.

Die Verbindungen E9, E10, E12, E13, E17, E33 und BM1 können gemäß den folgenden Offenlegungen hergestellt werden.

| | |
|---|---|
| E9 | Setayesh et al., Macromolecules, 33, 2016 (2000) und WO 2004/041901 |
| E10 | WO 2004/113412 |
| E12 | DE 19549741 |
| E13 | DE19549741 |
| E33 | EP 1223209 , DE 10200040223914, US 7,297,379,EP 1223210 |
| BM1 | EP 2033707 |
| | Wesslau, Makromolekulare Chemie 93, 55(1966) |

Die Synthese anderer Terphenyle oder Biphenyle sind dem Fachmann aus dem Stand der Technik geläufig. Verbindungen gemäß Formel (89-1) bis (89-6) können, beispielsweise, durch Suzuki-Kupplung wie folgt synthetisiert werden:

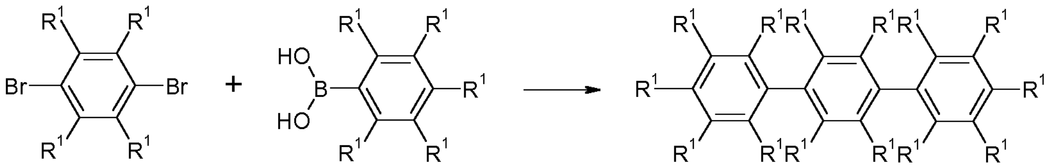

(89-1)

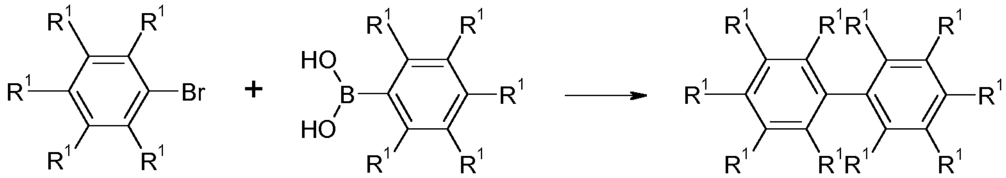

(89-2)

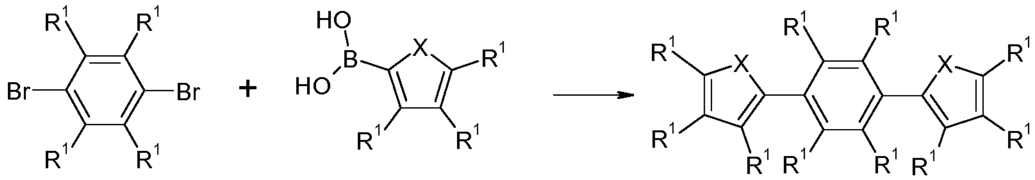

(89-3)

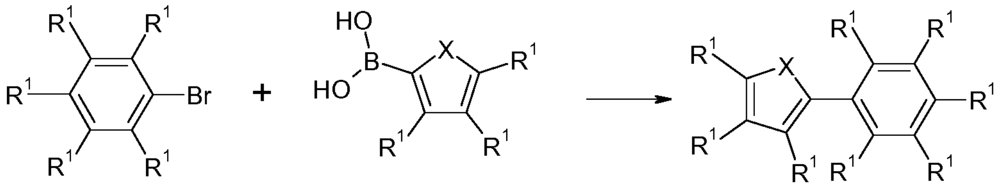

(89-4)

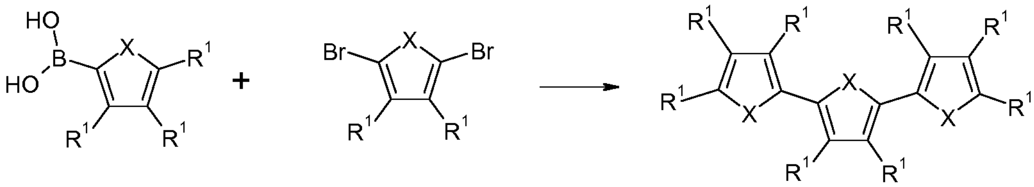

(89-6)

wobei $R^1$ und X die gleiche Bedeutung haben, wie oben angegeben wurde. Die folgende Übersicht enthält weitere Vorschriften für die genannten Verbindungen.

| | |
|---|---|
| E1 | EP 329752 |
| E2 | EP 4419932 B1 oder WO 9103450 A1 |
| E17 | EP 1053578 |
| E20 | EP 440082 |
| E24 | EP 329752 |
| E25 | DE 3878450 und EP 334911 |
| E28 | JP H 06-264059 (A) |
| E29 | EP 441932 |
| E31 | JP H 06-264059 (A) |

(fortgesetzt)

| | |
|---|---|
| H1 | DE 19927627 |
| H2 | DE 19927627 |

## Beispiel 2

### Quantenchemische Berechnungen

Die HOMO- (highest occupied molecular orbital) und LUMO-Lagen (lowest unoccupied molecular orbital) sowie das Triplett/Singulett Niveau und die Oszillatorstärke organischen Verbindungen werden über quantenchemische Rechnungen bestimmt. Hierzu wird das Programmpaket "Gaussian03W" (Gaussian Inc.) verwendet. Zur Berechnung organischer Substanzen ohne Metalle wird zunächst eine Geometrieoptimierung mittels der semiempirischen Methode "Ground State/Semi-empirical/ Default Spin/AM1" (Charge 0/Spin Singlet) durchgeführt. Im Anschluss daran erfolgt auf Basis der optimierten Geometrie eine Energierechnung. Hierbei wird die Methode "TD-SCF/DFT/ Default Spin/B3PW91" mit dem Basissatz "6-31 G(d)" verwendet (Charge 0/Spin Singlet). Die wichtigsten Ergebnisse sind HOMO / LUMO-Niveaus, Energien für die Triplett-und Singulett ($S_1$)-angeregten Zuständen sowie die Oszillatorstärke (f). Die ersten angeregten Zustände ($S_1$ und $T_1$) sind dabei am wichtigsten. $S_1$ steht für das erste angeregte Singulett Niveau und $T_1$ steht für das erste angeregte Triplett-Niveau. Aus der Energierechnung erhält man das HOMO HEh bzw. LUMO LEh in Hartree-Einheiten. Daraus werden die HOMO- und LUMO-Werte in Elektronenvolt (eV) wie folgt bestimmt, wobei sich diese Beziehungen aus der Kalibrierung anhand von Cyclovoltammetriemessungen (CV) ergeben:

$$HOMO(eV) = ((HEh * 27.212) - 0.9899) / 1.1206$$

$$LUMO(eV) = ((LEh * 27.212) - 2.0041) / 1.385$$

Diese Werte sind im Sinne dieser Anmeldung als energetische Lage des HOMO-Niveaus bzw. des LUMO-Niveaus der Materialien anzusehen. Als Beispiel erhält man für die Verbindung Ref1 (Siehe auch Tabelle 1) aus der Rechnung ein HOMO von -0,21401 Hartrees und ein LUMO von - 0,03463 Hartrees, was einem kalibrierten HOMO von -6,06 eV, einem kalibrierten LUMO von -2.19 eV.

Dem Fachmann ist bekannt, dass die quantenchemischen Berechnungen, wie hier beschrieben, sehr gut für die genannten Zwecke einsetzbar sind. Die Berechnungen liefern Ergebnisse, die sehr gut mit experimentell ermittelten Daten korrelieren.

**Tabelle 1:** Energieniveaus von Ref1, Ref2, E1-E34 und H1-H2

| Material | HOMO [eV] | LUMO [eV] | S1 [eV] | f |
|---|---|---|---|---|
| Ref1 | -6,06 | -2,19 | 4,31 | 0,08 |
| Ref2 | -6,08 | -2,13 | 4,24 | 0,03 |
| E1 | -6,15 | -2,27 | 4,11 | 1,00 |
| E2 | -6,38 | -2,49 | 4,20 | 0,90 |
| E3 | -6.21 | -2.24 | 4.30 | 1.05 |
| E4 | -6.47 | -2.48 | 4.04 | 0.93 |
| E6 | -6.01 | -2.00 | 4.24 | 0.73 |
| E7 | -6.30 | -2.25 | 4.26 | 0.82 |
| E8 | -6.48 | -2.14 | 4.22 | 0.56 |
| E9 | -5.73 | -2.32 | 3.91 | 0.74 |
| E10 | -5.73 | -2.34 | 3.90 | 0.82 |
| E11 | -6.51 | -2.20 | 4.24 | 0.35 |
| E12 | -6.04 | -2.17 | 4.18 | 0.58 |
| E13 | -5.81 | -2.05 | 4.17 | 0.65 |
| E14 | -6.17 | -2.42 | 3.92 | 0.92 |
| E15 | -6.09 | -2.12 | 4.42 | 0.35 |
| E16 | -5.73 | -2.27 | 3.98 | 1.14 |

(fortgesetzt)

| Material | HOMO [eV] | LUMO [eV] | S1 [eV] | f |
|---|---|---|---|---|
| E17 | -5.99 | -2.11 | 4.06 | 0.05 |
| E20 | -6.46 | -2.52 | 4.19 | 0.80 |
| E21 | -6.04 | -2.21 | 4.20 | 1.00 |
| E22 | -6.47 | -2.53 | 4.19 | 0.94 |
| E23 | -6.49 | -2.55 | 4.18 | 0.94 |
| E24 | -6.09 | -2.25 | 4.23 | 1.08 |
| E25 | -6.15 | -2.33 | 4.20 | 1.04 |
| E26 | -6.25 | -2.34 | 4.21 | 1.11 |
| E27 | -6.27 | -2.52 | 4.17 | 0.92 |
| E28 | -6.26 | -2.38 | 4.21 | 0.94 |
| E29 | -6.27 | -2.42 | 4.20 | 0.93 |
| E30 | -6.28 | -2.35 | 4.21 | 1.00 |
| E31 | -6.33 | -2.41 | 4.20 | 0.88 |
| E32 | -6.68 | -2.68 | 3.82 | 0.61 |
| E33 | -6.01 | -2.18 | 4.14 | 0.23 |
| E34 | -6.39 | -2.49 | 3.88 | 0.30 |
| H1 | -6.51 | -2.19 | 4.26 | 0.02 |
| H2 | -6.51 | -2.19 | 4.26 | 0.02 |

## Beispiel 3

### Lösungen und Zusammensetzungen

Lösungen, wie sie in Tabelle 2 zusammengefasst sind, werden wie folgt hergestellt: Zunächst werden die Mischungen von Host und Emitter in 10 mL Toluol gelöst und so lange gerührt, bis die Lösung klar ist. Die Lösung wird unter Verwendung eines Filters Millipore Millex LS, Hydrophobic PTFE 5.0 μm filtriert.

**Tabelle 2:** Zusammensetzung der Lösungen

| | Zusammensetzung | Verhältnis (bezogen auf Gewicht) | Konzentration |
|---|---|---|---|
| Lösung-Ref1 | Ref1 | 100% | 16 mg/ml |
| Lösung-Ref2 | Ref2 | 100% | 16 mg/ml |
| Lösung x* | PS:Ex | 70% : 30% | 16 mg/ml |
| *x ist 1, 2, 3, ... 34; PS steht für Polystyrol;* | | | |

Die Lösungen werden verwendet, um die emittierende Schicht von OLEDs zu beschichten. Die entsprechende Feststoffzusammensetzung kann erhalten werden, indem das Lösemittel der Lösungen verdampft wird. Diese kann für die Herstellung weiterer Formulierungen verwendet werden.

## Beispiel 4

### Herstellung der OLEDs

OLED-Ref1, OLED-Ref2, OLED1 - OLED16 weisen die folgende Struktur auf: ITO/PEDOT/EML/Kathode, wobei EML für die Emissionsschicht und ITO für die Anode (Indium-Zinnoxid) steht.

Die OLEDs werden unter Verwendung der entsprechenden Lösungen, wie in Tabelle 2 zusammengefasst, gemäß der folgenden Vorschrift hergestellt:

1) Beschichtung von 80 nm PEDOT (Clevios™ P VP Al 4083) auf ein ITObeschichtetet Glassubstrat durch Spin-Coating; Ausheizen für 10 min. bei 180°C.
2) Beschichtung einer 80 nm emittierenden Schicht durch Spin-Coating einer der Lösung gemäß Tabelle 2.

3) Ausheizen der Vorrichtung: 10 min bei 180°C für OLED-Ref1 und OLEd-Ref2; für OLED1-16 30 min bei 50°C und dann 30 min unter Vakuum.
4) Aufdampfen einer Ba/Al-Kathode (3 nm / 150 nm).
5) Verkapselung der Vorrichtung.

**Beispiel 5**

**Charakterisierung der OLEDs**

Von den so erhaltenen OLEDs werden zunächst Photolumineszenspektren von der Emissionsschicht (EML) und anschließend Elektrolumineszenzspektren (EL) gemessen, weil das EL-Spektrum die wichtigsten Indizien für ein funktionierendes Elektrolumineszenzvorrichtung liefert.

Die EL-Spektren werden mittels Ocean Optics UBS2000 gemessen.

Die EL-Spektren von OLED1-16 sind in Abbildung 1-16 zusammengefasst. Für die OLEDs mit Ref1 und Ref2 als Emitter sind keine EL Spektren messbar, selbst wenn die Spannung auf bis zu 40 V erhöht wird. Die OLEDs mit den Emittern E1 bis E16 zeigen aber ein deutliches EL-Spektrum mit einem wesentlichen Anteil zwischen 280 bis 380 nm.

Es ist in der Tat sehr überraschend, dass die erfindungsgemäßen Emitter in einer OLD mit dem angegebenen, einfachen Schichtaufbau und mit Polystyrol als Host in UV-Bereich emittiert haben. Es ist offensichtlich, dass der Fachmann auf der Grundlage der vorliegenden Erfindung und ohne erfinderisches Handeln weitere Optimierungen vornehmen kann.

Weitere erfindungsgemäße Verbesserungen werden in den folgenden Beispielen offenbart.

**Beispiel 6**

**OLEDs mit Mixed Host**

Durch die Verwendung eines Mixed-Hosts können die erfindungsgemäßen OLED-Vorrichtungen hinsichtlich absoluter Intensität und Betriebsspannung weiter verbessert werden. Hierzu wird OLED17 analog zu dem in Beispiel 4 beschriebenen Verfahren hergestellt. Im Unterscheid zu OLED1 wird für OLED17 nicht die Zusammensetzung PS(70 wt%):E1 (30 wt%) für die EML, sondern die Zusammensetzung PS(30 wt%):E4(60 wt%):E1 (10wt%) verwendet. Hier wird E1 als Emitter und E4 als Host eingesetzt.
Abbildung 17 zeigt die EL Spektren und Betriebsspannungen von OELD1 und OLED17 im Vergleich, wobei die absoluten Intensitäten auf das Maximum von OLED17 normiert sind. Es ist deutlich zu erkennen, dass durch die Verwendung eines Mixed-Hosts bestehend aus PS und E4 die Intensität von die UV OELD1 etwa um den Faktor 4 erhöht werden kann, wobei gleichzeitig die Betriebsspannung reduziert werden kann.

**Beispiel 7**

**OLEDs mit Sperrschicht**

Eine weitere Optimierung kann durch Verwendung einer Sperrschicht erhalten werden. Hierzu wird das Sperrmaterial BM1 eingesetzt. BM1 ist ein reaktives Mesogen, das mit Hilfe von Ausheizen oder UV-Strahlung vernetzt werden und dadurch ein unlösliches Netzwerk bilden kann.

Zunächst werden quantenchemische Berechnungen an BM1 und BM1b (das ist die nach der Vernetzungsreaktion im Netzwerk effektive Komponente; Abbildung 18) durchgeführt. Das $S_1$ Niveau von BM1 Liegt bei 3.6 eV und das von BM1b bei 4.29 eV. Die HOMOs und LUMOs von BM1 und BM1b sind in Abbildung 18 dargestellt. BM1b weist ein relativ hohes LUMO Niveau von -2.14 eV auf. Das Material eignet sich daher als Excitonen- und/oder als Elektronen-Blockiermaterial.

Zur Überprüfung dieser theoretischen Betrachtungen wird folgender Versuch durchgeführt.

1) 20 nm einer Schicht bestehend aus BM1 wird durch Spin-Coating aus einer Lösung von BM1 in Toluol mit einer Konzentration von 20 mg/ml auf ein Glas-Substrat aufgebracht;

2) die Schicht wird bei 180°C für 1 h in einer Glove-Box ausgeheizt;

3) die Schicht wird mit Toluol durch Spin-Coating gewaschen;

4) die Schichtdicke wird nochmals gemessen. Nach dem Waschen verbleibt eine etwa 15 nm dicke Schicht auf dem

Substrat.

Anschließend wird OLED18 wie folgt hergestellt:

1) Auftragung einer Schicht von 80 nm PEDOT (Clevios™ P VP AI 4083) auf einem ITO-beschichteteten Glassubstrat durch Spin-Coating, und Ausheizen für 10 min. bei 180°C;
2) Auftragung einer 20 nm Sperrschicht durch Spin-Coating einer Toluol Lösung von BM1 mit einer Konzentration von 20 g/ml;
3) Ausheizen der Sperrschicht: 60 min. bei 180°C;
4) Waschen der Sperrschicht mit Toluol durch Spin-Coating;
5) Auftragung einer 80 nm dünnen emittierenden Schicht durch Spin-Coating einer Toluol Lösung enthaltend eine Zusammensetzung (PS(30wt%):E4(60wt%):E1 (10wt%)) mit einer Konzentration von 16 mg/ml;
6) Ausheizen der Vorrichtung: 30 min. bei 50 °C und anschließend 30 min. unter Vakuum;
7) Aufdampfen einer Ba/Al-Kathode (3 nm / 150 nm);
8) Verkapselung der Vorrichtung.

Abbildung 19 zeigt die EL Spektren und Betriebsspannungen von OLED17 (ohne Sperrschicht) und OLED18 (mit Sperrschicht), wobei die absoluten Intensitäten auf das Maximum von OLED18 normiert werden. Die Abbildung zeigt eine deutliche Verbesserung der Eigenschaften von OLED18 gegenüber OLED17 hinsichtlich Intensität und Betriebsspannung.

**Beispiel 8 nicht gemäß der Erfindung**

**OLEDs mit Pufferschicht mit einer hoch Austrittsarbeit**

Basierend auf der technischen Lehre der vorliegenden Beschreibung können auch OLEDs hergestellt werden, die UV-B Strahlung emittieren (OLED20). Hierzu wird eine Pufferschicht mit einer höheren Austrittsarbeit verwendet (Information von Hersteller "Heraeus Precious Metals GmbH & Co. KG", bzw. Siehe auch Youn et al., J. Electrochem. Soc. 158, J321 (2011).

Als Vergleich zu OLED20 wird OLED19 analog zu dem in Beispiel 4 beschriebenen Verfahren hergestellt, wobei die EML die Zusammensetzung PS(70wt%):E17(30wt%) hat

OLED20 wird wie folgt hergestellt:

1) Auftragung von 80 nm CLEVIOS™ HIL 1.3 auf ein ITO-beschichtetes Glassubstrat durch Spin-Coating mit anschließendem Ausheizen für 10 min. bei 180°C;
2) Auftragung einer 80 nm Schicht als EML durch Spin-Coating einer Toluol Lösung enthaltend die Zusammensetzung (PS(30wt%):H1(30wt%):H2(30wt%):E17(10wt%)) in einer Konzentration von 16 mg/ml;
3) Ausheizen der Vorrichtung: 30 min. bei 50°C und anschließend 30 min. unter Vakuum;
4) Aufdampfen einer Ba/Al-Kathode (3 nm / 150 nm).
5) Verkapselung der Vorrichtung.

In Vergleich zum Clevios™ P VP AI 4083 hat die CLEVIOS™ HIL 1.3 eine deutlich hohe Austrittsarbeit, sodass die Injektion von Löchern aus der Anode in die EML erleichtert werden kann.

Für die OLED19 mit PS als Host, ist das EL Spektrum nicht messbar, selbst bei einer angelegten Spannung von bis zu 40 V.

Abbildung 20 zeigt, dass OLED20 in UV-B Bereich bei einer niedrigen Betriebsspannung von 16 V emittiert. Das Emissionsmaximum liegt bei etwa 312 nm. Andere organische Elektrolumineszenzvorrichtungen, die UV-B Starhlung emittieren, sind nicht bekannt.

**Beispiel 9**

**OLEDs mit Emittern E18 bis E34**

Für die Emitter E18-E34 können die PL-Spektren gemessen werden. Hierzu werden sie in einer dünnen Schicht auf Quarz-Glas gebracht, wobei die dünne Schicht durch Spin-Coating einer entsprechenden Lösung gemäß Tabelle 2 hergestellt wird. Alle PL-Spektren zeigen eine Emission zwischen 280 und 380 nm. Auf der Grundlage der oben beschrieben technischen Lehre kann Fachmann ohne erfinderisches Zutun Elektrolumineszenzvorrichtungen herstellen, die Strahlung im UV Bereich emittieren. Des Weiteren kann der Fachmann ohne Schwierigkeiten weitere Verbesserungen

durch Routineexperimente an der erfindungsgemäßen technischen Lehre vornehmen. So kann er, beispielsweise, eine andere Co-Matrix oder eine Exzitonen-Blockierschicht oder eine Anode oder ein Substrat mit verbesserter UV-Transparenz verwenden.

## Beispiel 10

### Synthese von Polymer P1

Das Polymer P1 wird aus den Monomereinheiten M1 und M2 hergestellt.

P1

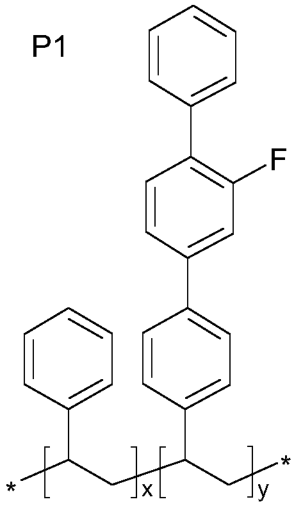

x=0.5, y=0.5

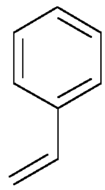

M1

CAS 100-42-5

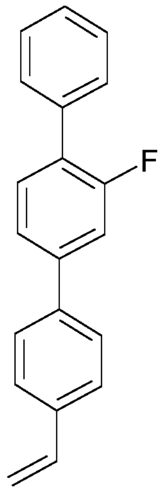

M2

### Synthese von M2:

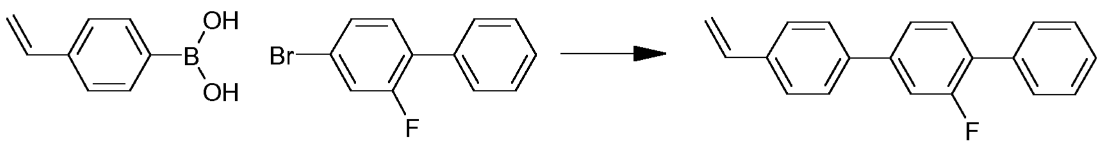

1 2 M2

In einen Kolben werden Verbindung **1** (7.0 g, 47.3 mmol) und Verbindung **2** (11.88 g, 47, 3 mmol) in 140 ml trockenem 1,4-Dioxan unter Rühren gelöst, dann $PdCl_2(PCy)_3$ (1.711 g, 2.3 mmol) zugesetzt und das Reaktionsgefäß mit Argon gespült. Cäsiumfluorid (Aldrich 19,832-3(14.4 g, 94.7 mmol)) wird zugegeben und die Reaktionsmischung über 12 Stunden bei 100°C gerührt. Die Reaktionsmischung wird mit Wasser und Dichlormethan versetzt und die Phasen getrennt. Die wässrige Phase wird mit Dichlormethan nachgewaschen. Die vereinten organischen Phasen werden mit gesättigter NaCl Lösung gewaschen, anschließend über Natriumsulfat getrocknet, filtriert und einrotiert.

### Synthesis von P1:

0.597 g (2.175 mmol) 2'-Fluor-4"-vinyl-[1,1',4',1"]-terphenyl (M2) und 0.228 g (2.175 mmol) Styrol (M1) werden in einen getrockneten Kolben überführt und inertisiert. Es werden 2.3 ml entgastes, trockenes Toluol hinzugegeben und die Lösung mehrfach inertisiert. In einem separaten Schlenkgefäß werden 0.7 mg (0.004 mmol) α,α'-Azoisobutyronitril in 10 ml trockenem, entgastem Toluol gelöst und inertisiert. Die Monomerlösung wird auf 70°C erwärmt und 0.1 ml der AIBN-Lösung zur Reaktionslösung per Spritze zugegeben. Die Reaktionsmischung wird für 72 h bei 70°C gerührt, dann auf Raumtemperatur abgekühlt und weitere 24 h gerührt. Die Reaktionsmischung wird in entgastes Ethanol getropft

und der entstehende Feststoff abfiltriert. Der Feststoff wird erneut in Toluol gelöst und in Ethanol ausgefällt. Der Feststoff wird abfiltriert und im Hochvakuum bei 40°C für 24h getrocknet.

GPC-Messung werden mit THF als Eluent und o-Dichlorbenzol als internem Standard (1.2 ml/l) gemessen. Die Konzentration des Analyten beträgt 1.000 g/l. Die Detektion erfolgte sowohl durch einen Dioden-Array-Detektor (250 nm), als auch durch einen Refractive-Index-Detektor.

GPC Daten:

Mn: 150.000 g/mol
Mw: 385.000 g/mol
PD: 2,6

Elementaranalyse:

C 88,0 % ±0,2 (88.63% berechnet)

Die Absorptions- und Photolumineszenzspektren von Polymer P1 werden mittels einer 80 nm Schicht gemessen und in Abbildung 21 gezeigt. P1 zeigt eine Emission im UV Bereich (Peak um 350 nm) sowie einen weiteren Anteil im blauen Bereich des Spektrums.

**Beispiel 11: OLEDs mit Polymer P1, nicht erfindungsgemäß**

OLED21 und OELD22 werden analog zu dem Verfahren in Beispiel 4 herstellt, wobei die EML für OLED21 aus 100% P1, und für OLED22 aus einer Mischung von 90% P1:10% E9 besteht. Beide EMLs werden für 10 min bei 180°C ausgeheizt..

Abbildung 22 zeigt das Elektrolumineszenzspektrum von OLED21. Abbildung 23 zeigt das Elektrolumineszenzspektrum von OLED22.

In Vergleich zum OLEDs mit kleinen Molekülen zeigt die P1-enthaltende EML Zusammensetzungen von OLED21 und OELD22 eine verbesserte Löslichkeit und eine verbesserte Schichtbildungseigenschaft. Weiterhin sind die Leistungsdaten OLED21 und OLED22, insbesondere bezüglich Intensität, Betriebsspannung und Stabilität verbessert.

## Patentansprüche

1. Organische Elektrolumineszenzvorrichtung enthaltend in der lichtemittierenden Schicht wenigstens eine organische Verbindung der Formeln (89-1) und/oder (89-2),

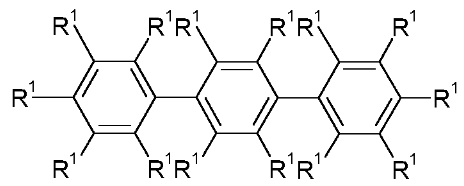

Formel (89-1)

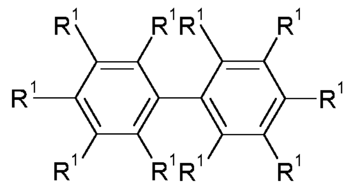

Formel (89-2)

wobei für die verwendeten Symbole und Indizes gilt:

$R^1$ ist gleich oder verschieden bei jedem Auftreten H, F, CN, $CF_3$, $CHF_2$ oder eine Alkyl-, Alkoxy- oder Alkylalkoxygruppe mit 1 bis 15 C-Atomen;

mit der Maßgabe, dass mehr als einer der Reste $R^1$ in der Verbindung der Formeln (89-1) und/oder (89-2) gleich F sind.

2. Vorrichtung gemäß Anspruch 1, **dadurch gekennzeichnet, dass** die Verbindung in der emittierenden Schicht die allgemeine Formel (89-1) hat,

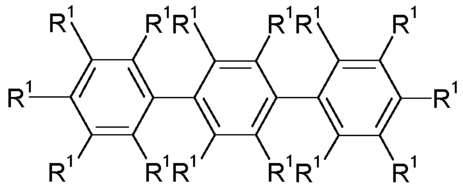

Formel (89-1),

wobei bevorzugt maximal 4 der Reste $R^1$ ungleich H sind.

3. Vorrichtung gemäß Anspruch 1, **dadurch gekennzeichnet, dass** die Verbindung in der emittierenden Schicht ausgewählt wird aus den Verbindungen

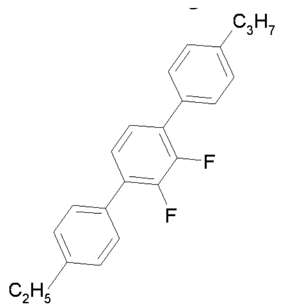

E1

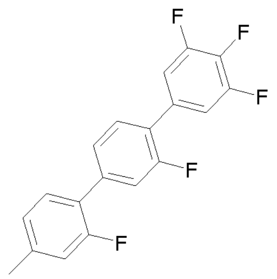

Formel (91)

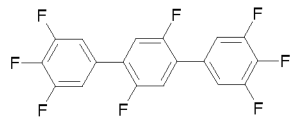

Formel (93)

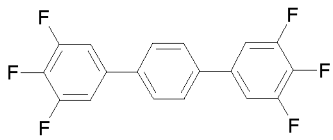

Formel (96)

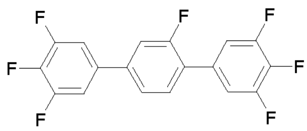

Formel (97)

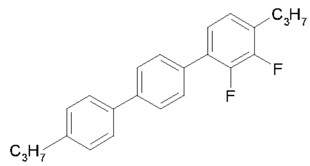

Formel (119)

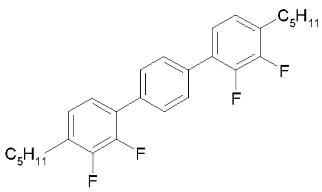

Formel (121)

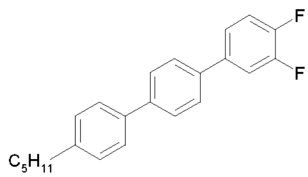

Formel (122)

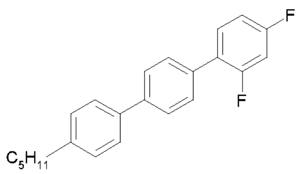

Formel (123)

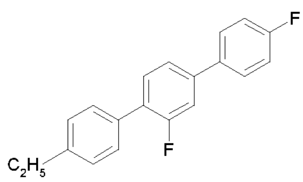

Formel (124)

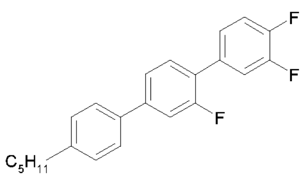

Formel (127)

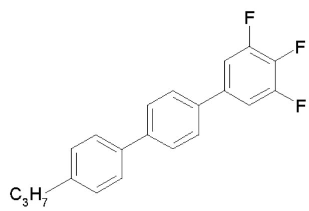

Formel (128)

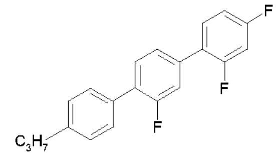

Formel (129)

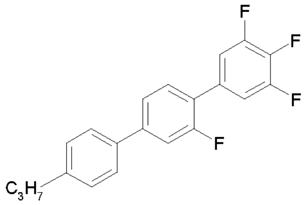

Formel (131)

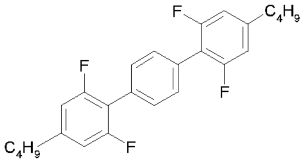

Formel (132)

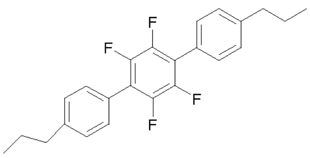

Formel (134)

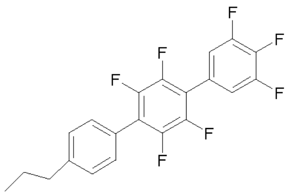

Formel (135)

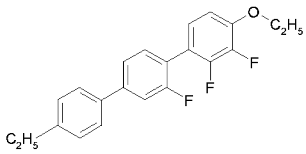

Formel (137)

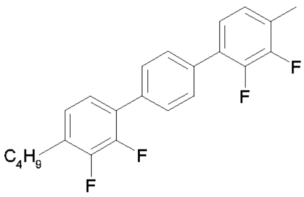

Formel (138)

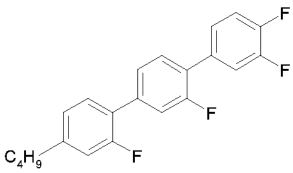

Formel (139)

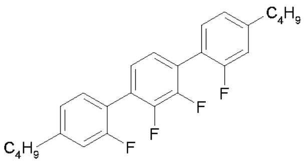

Formel (140)

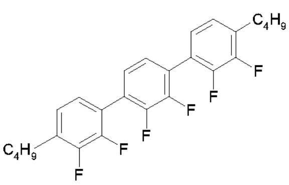

Formel (141)

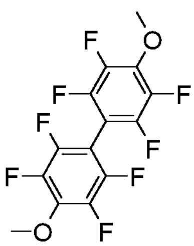

Formel (147)

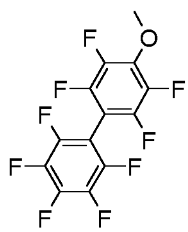

Formel (148)

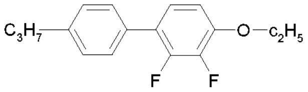

Formel (151)

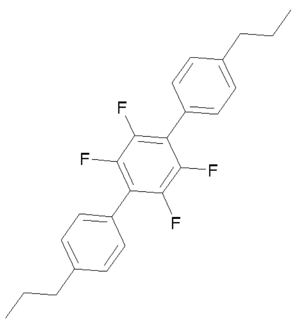

Formel (152)

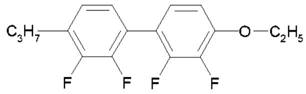

Formel (155)

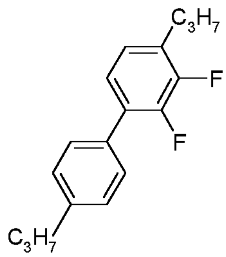

Formel (159).

4. Vorrichtung gemäß einem oder mehreren der Ansprüche 1 bis 3, **dadurch gekennzeichnet, dass** die organische Verbindung in der lichtemittierenden Schicht als Emitter und/oder als Host eingesetzt wird.

5. Vorrichtung gemäß Anspruch 1, **dadurch gekennzeichnet, dass** der Host aus mindestens 2 unterschiedlichen Verbindungen besteht, wobei mindestens ein Host der Formel (89-1) und/oder (89-2) entspricht.

6. Vorrichtung gemäß Anspruch 1, dadurch charakterisiert, dass die Vorrichtung wenigstens eine Sperrschicht zwischen der lichtemittierenden Schicht und einer der Elektroden enthält, wobei die weitere Sperrschicht ausgewählt ist aus Sperrschichten, die Exzitonen, Elektronen oder Löcher blockieren.

7. Vorrichtung gemäß einem oder mehreren der Ansprüche 1 bis 6, **dadurch gekennzeichnet, dass** es sich bei der Vorrichtung um eine Vorrichtung aus der Gruppe bestehend aus den organischen lichtemittierenden Dioden (OLEDs), polymeren lichtemittierenden Dioden (PLEDs), organischen lichtemittierenden elektrochemischen Zellen (OLECs), organischen lichtemittierenden Transistoren (O-LETs) und organischen lichtemittierenden elektrochemischen Transistoren handelt.

8. Zusammensetzung enthaltend wenigstens eine der Verbindungen nach Formel (89-1) und/oder (89-2) sowie wenigstens ein organisches Material oder organischen Halbleiter, ausgewählt aus der Gruppe der Emitter, Host Materialien, Matrix Materialien, Elektronentransportmaterialien (ETM), Elektroneninjektionsmaterialien (EIM), Lochtransportmaterialien (HTM), Lochinjektionsmaterialien (HIM), Elektronenblockiermaterialien (EBM), Lochblockiermaterialien (HBM) und Exzitonenblockiermaterialien (ExBM),

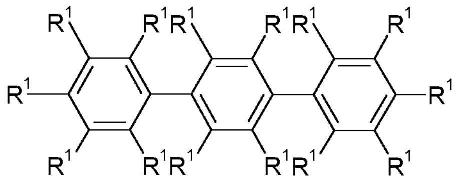

Formel (89-1)

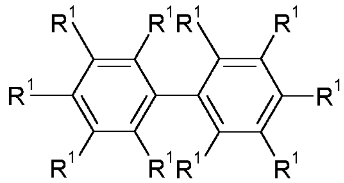

Formel (89-2)

wobei für die verwendeten Symbole und Indizes gilt:

$R^1$ ist gleich oder verschieden bei jedem Auftreten H, F, CN, $CF_3$, $CHF_2$ oder eine Alkyl-, Alkoxy- oder Alkylalkoxygruppe mit 1 bis 15 C-Atomen;

mit der Maßgabe, dass mehr als einer der Reste $R^1$ in der Verbindung der Formeln (89-1) und/oder (89-2) gleich F sind.

9. Formulierung enthaltend die Zusammensetzung nach Anspruch 8 und wenigstens ein Lösungsmittel.

## Claims

1. Organic electroluminescent device comprising, in the light-emitting layer, at least one organic compound of the formulae (89-1) and/or (89-2),

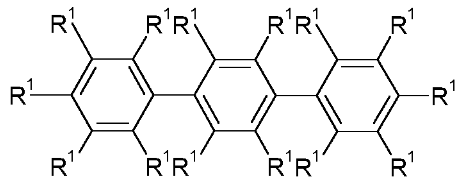

formula (89-1)

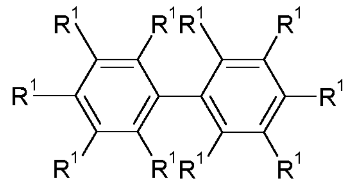

formula (89-2)

where the following applies to the symbols and indices used:

$R^1$ is, identically or differently on each occurrence, H, F, CN, $CF_3$, $CHF_2$ or an alkyl, alkoxy or alkylalkoxy group having 1 to 15 C atoms;

with the proviso that more than one of the radicals $R^1$ in the compound of the formulae (89-1) and/or (89-2) are equal to F.

**2.** Device according to Claim 1, **characterised in that** the compound in the emitting layer has the general formula (89-1),

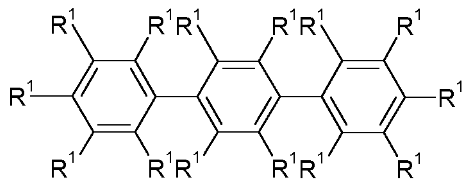

formula (89-1)

where preferably a maximum of 4 of the radicals $R^1$ are not equal to H.

**3.** Device according to Claim 1, **characterised in that** the compound in the emitting layer is selected from the compounds

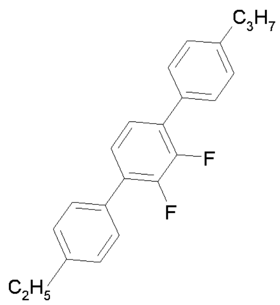

E1

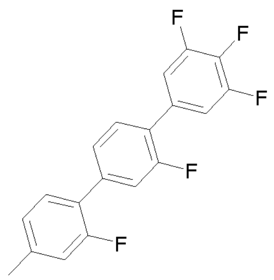

formula (91)

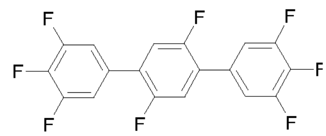

formula (93)

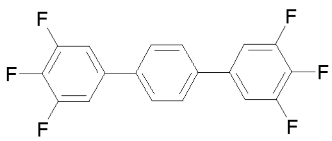

formula (96)

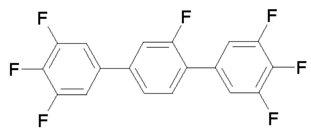

formula (97)

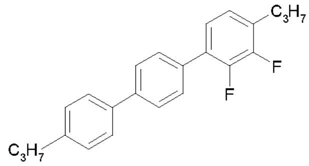

formula (119)

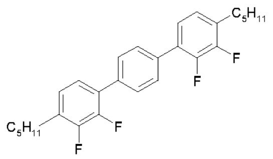
formula (121)

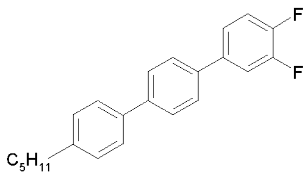
formula (122)

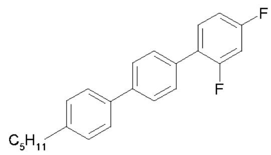
formula (123)

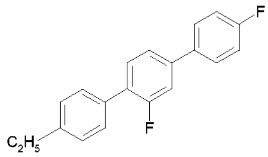
formula (124)

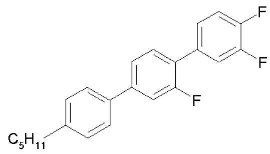
formula (127)

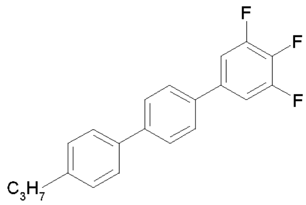
formula (128)

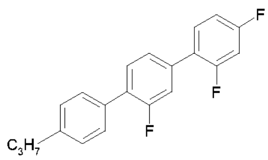
formula (129)

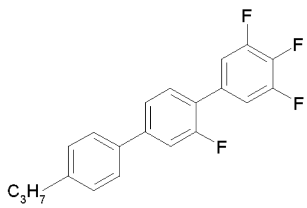
formula (131)

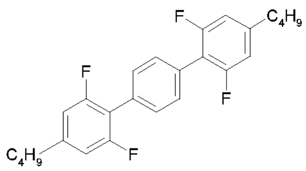
formula (132)

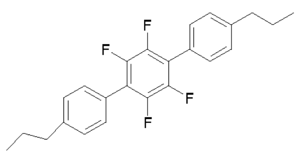
formula (134)

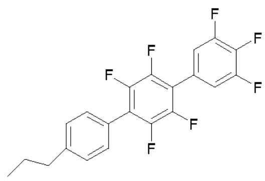
formula (135)

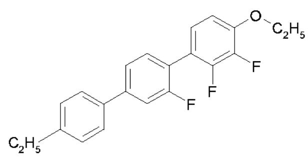
formula (137)

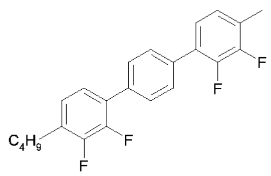
formula (138)

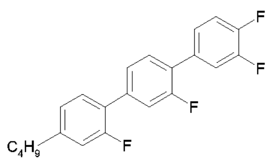
formula (139)

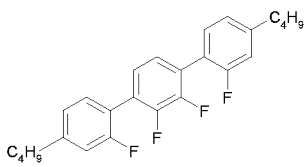
formula (140)

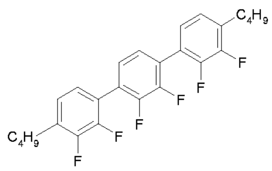
formula (141)

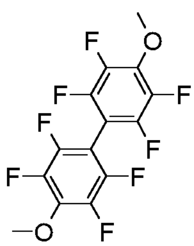
formula (147)

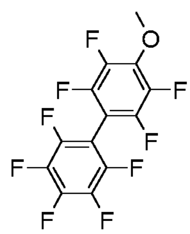
formula (148)

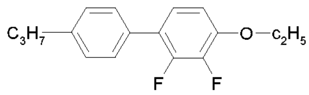

formula (151)

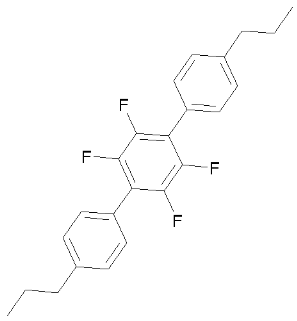

formula (152)

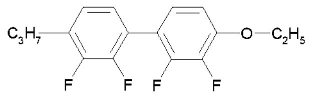

formula (155)

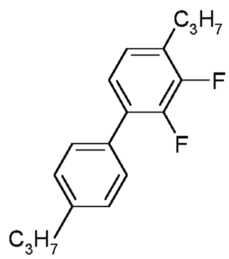

formula (159).

4. Device according to one or more of Claims 1 to 3, **characterised in that** the organic compound in the light-emitting layer is employed as emitter and/or as host.

5. Device according to Claim 1, **characterised in that** the host consists of at least 2 different compounds, where at least one host conforms to the formula (89-1) and/or (89-2).

6. Device according to Claim 1, **characterised in that** the device contains at least one barrier layer between the light-emitting layer and one of the electrodes, where the further barrier layer is selected from barrier layers which block excitons, electrons or holes.

7. Device according to one or more of Claims 1 to 6, **characterised in that** the device is a device from the group consisting of the organic light-emitting diodes (OLEDs), polymeric light-emitting diodes (PLEDs), organic light-emitting electrochemical cells (OLECs), organic light-emitting transistors (O-LETs) and organic light-emitting electrochemical transistors.

8. Composition comprising at least one of the compounds of the formula (89-1) and/or (89-2) and at least one organic material or organic semiconductor selected from the group of the emitters, host materials, matrix materials, electron-transport materials (ETMs), electron-injection materials (EIMs), hole-transport materials (HTMs), hole-injection materials (HIMs), electron-blocking materials (EBMs), hole-blocking materials (HBMs) and exciton-blocking materials (ExBMs),

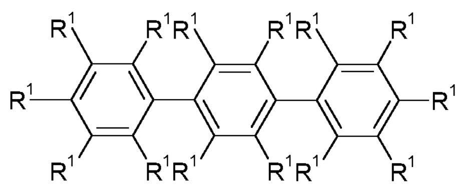

formula (89-1)

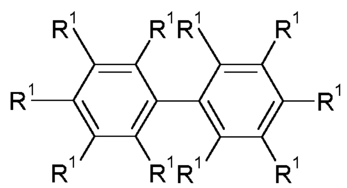

formula (89-2)

where the following applies to the symbols and indices used:

$R^1$ is, identically or differently on each occurrence, H, F, CN, $CF_3$, $CHF_2$ or an alkyl, alkoxy or alkylalkoxy group having 1 to 15 C atoms;

with the proviso that more than one of the radicals $R^1$ in the compound of the formulae (89-1) and/or (89-2) are equal to F.

**9.** Formulation comprising the composition according to Claim 8 and at least one solvent.

## Revendications

**1.** Dispositif électroluminescent organique comprenant, à l'intérieur de la couche d'émission de lumière, au moins un composé organique des formules (89-1) et/ou (89-2),

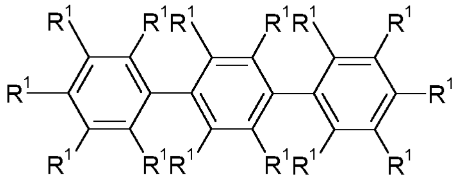

formule (89-1)

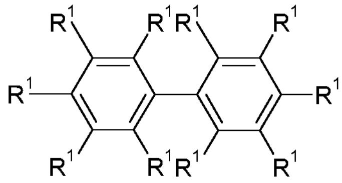

formule (89-2)

dans lesquelles ce qui suit s'applique aux symboles et aux indices qui sont utilisés :

$R^1$ est, de manière identique ou différente pour chaque occurrence, H, F, CN, $CF_3$, $CHF_2$ ou un groupe alkyle, alcoxy ou alkylalcoxy qui comporte de 1 à 15 atome(s) de C ;

étant entendu que plus d'un des radicaux $R^1$ dans le composé des formules (89-1) et/ou (89-2) sont égaux à F.

**2.** Dispositif selon la revendication 1, **caractérisé en ce que** le composé à l'intérieur de la couche d'émission présente la formule générale (89-1),

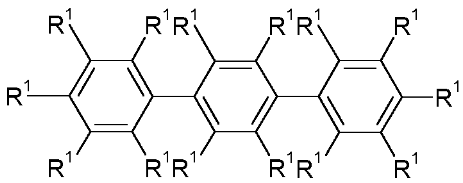

formule (89-1)

dans laquelle, de préférence, un maximum de 4 des radicaux $R^1$ ne sont pas égaux à H.

**3.** Dispositif selon la revendication 1, **caractérisé en ce que** le composé à l'intérieur de la couche d'émission est sélectionné parmi les composés

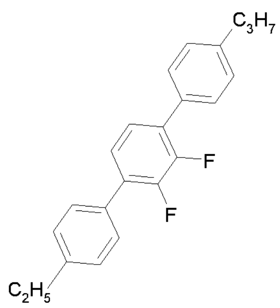

E1

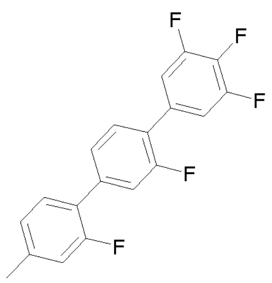

formule (91)

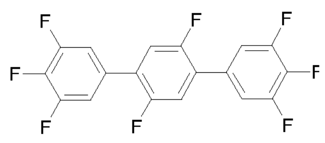

formule (93)

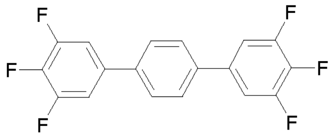

formule (96)

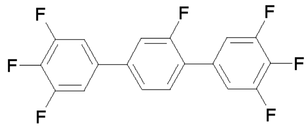

formule (97)

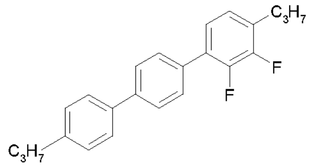

formule (119)

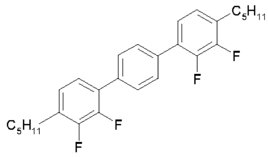

formule (121)

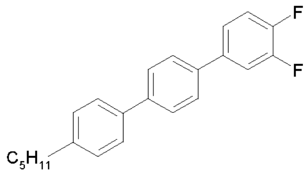

formule (122)

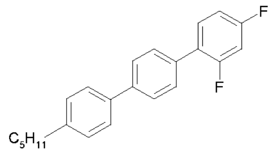

formule (123)

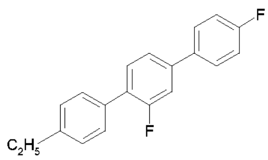

formule (124)

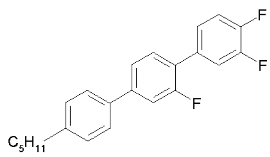

formule (127)

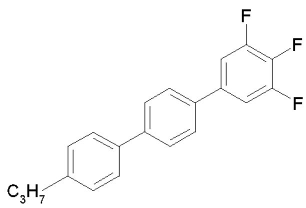

formule (128)

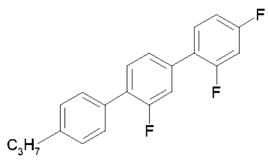

formule (129)

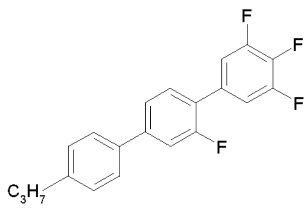

formule (131)

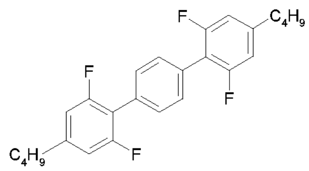

formule (132)

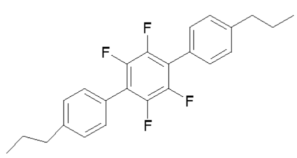

formule (134)

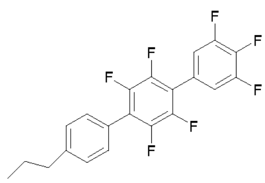

formule (135)

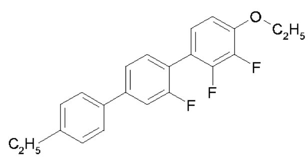

formule (137)

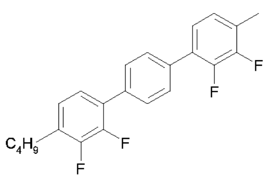

formule (138)

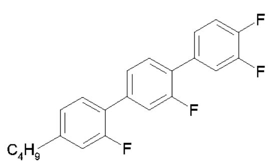

formule (139)

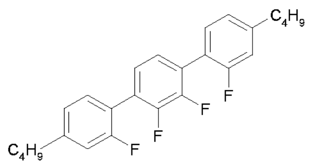

formule (140)

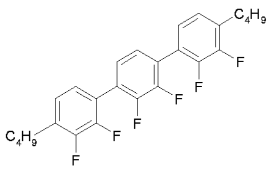

formule (141)

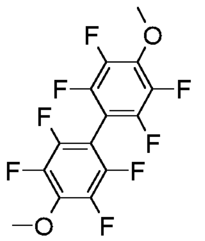

formule (147)

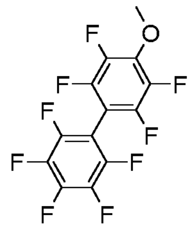

formule (148)

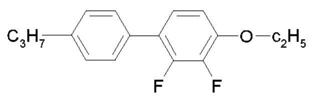

formule (151)

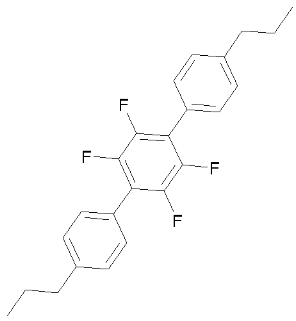

formule (152)

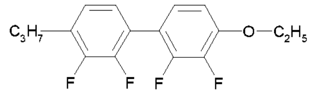

formule (155)

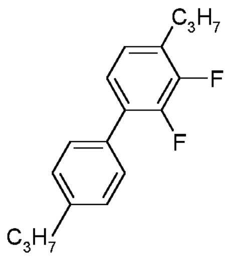

formule (159).

4. Dispositif selon une ou plusieurs des revendications 1 à 3, **caractérisé en ce que** le composé organique à l'intérieur de la couche d'émission est utilisé en tant qu'émetteur et/ou en tant qu'hôte.

5. Dispositif selon la revendication 1, **caractérisé en ce que** l'hôte est constitué par au moins 2 composés différents, où au moins un hôte est conforme aux formules (89-1) et/ou (89-2).

6. Dispositif selon la revendication 1, **caractérisé en ce que** le dispositif contient au moins une couche de barrière entre la couche d'émission de lumière et l'une des électrodes, où la couche de barrière supplémentaire est sélectionnée parmi les couches de barrière qui bloquent les excitons, les électrons ou les trous.

7. Dispositif selon une ou plusieurs des revendications 1 à 6, **caractérisé en ce que** le dispositif est un dispositif pris parmi le groupe qui est constitué par les diodes à émission de lumière organiques (OLED), les diodes à émission de lumière polymériques (PLED), les cellules électrochimiques à émission de lumière organiques (OLEC), les transistors à émission de lumière organiques (O-LET) et les transistors électrochimiques à émission de lumière organiques.

8. Composition comprenant au moins l'un des composés des formules (89-1) et/ou (89-2) et au moins un matériau organique ou un semiconducteur organique qui est sélectionné parmi le groupe constitué par les émetteurs, les matériaux hôtes, les matériaux de matrice, les matériaux de transport d'électrons (ETM), les matériaux d'injection d'électrons (EIM), les matériaux de transport de trous (HTM), les matériaux d'injection de trous (HIM), les matériaux de blocage d'électrons (EBM), les matériaux de blocage de trous (HBM) et les matériaux de blocage d'excitons (ExBM),

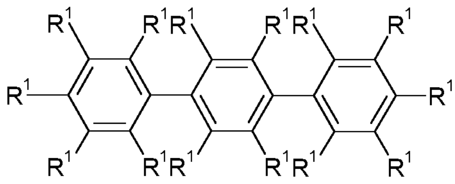

formule (89-1)

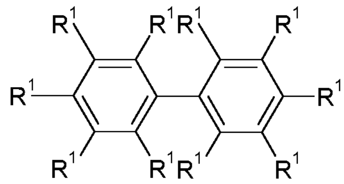

formule (89-2)

dans lesquelles ce qui suit s'applique aux symboles et aux indices qui sont utilisés :

$R^1$ est, de manière identique ou différente pour chaque occurrence, H, F, CN, $CF_3$, $CHF_2$ ou un groupe alkyle, alcoxy ou alkylalcoxy qui comporte de 1 à 15 atome(s) de C ;

étant entendu que plus d'un des radicaux $R^1$ dans le composé des formules (89-1) et/ou (89-2) sont égaux à F.

**9.** Formulation comprenant la composition selon la revendication 8 et au moins un solvant.

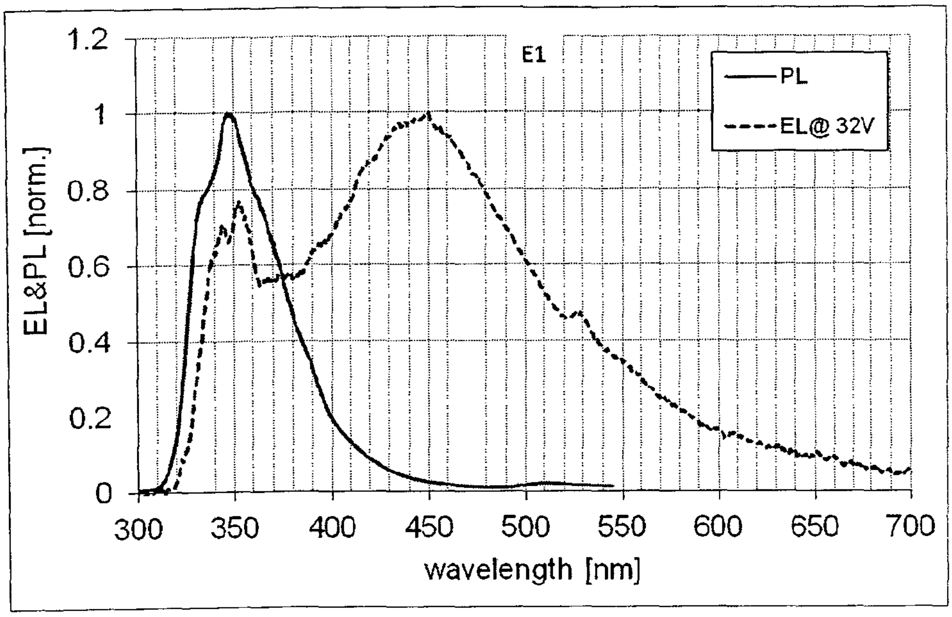


Abbildung 1

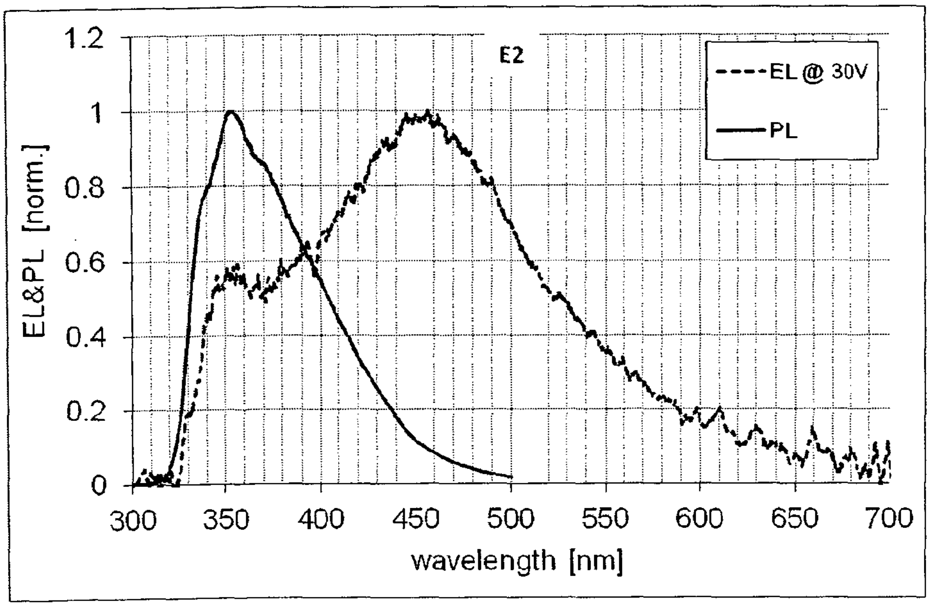


Abbildung 2

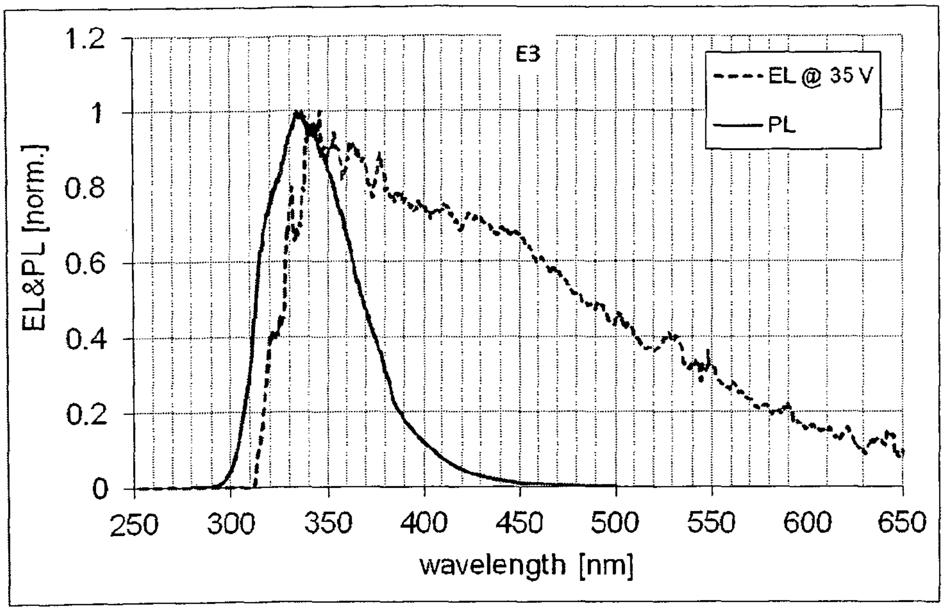


Abbildung 3

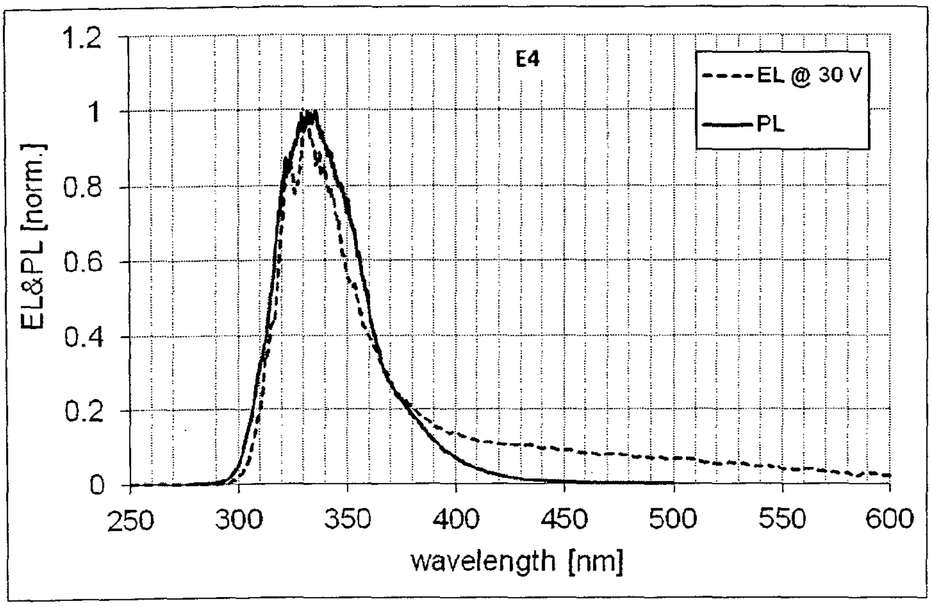


Abbildung 4

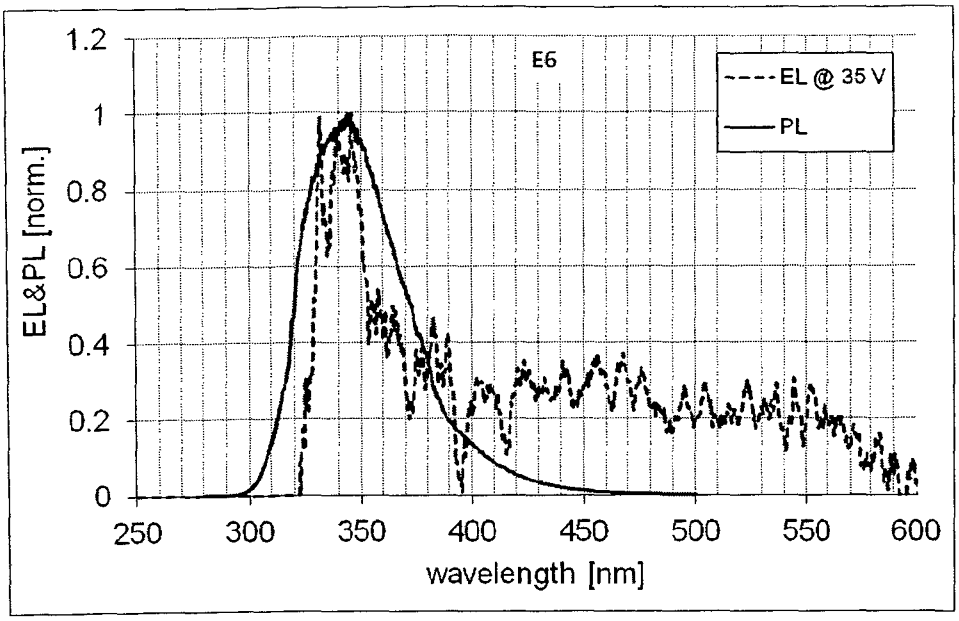


Abbildung 6

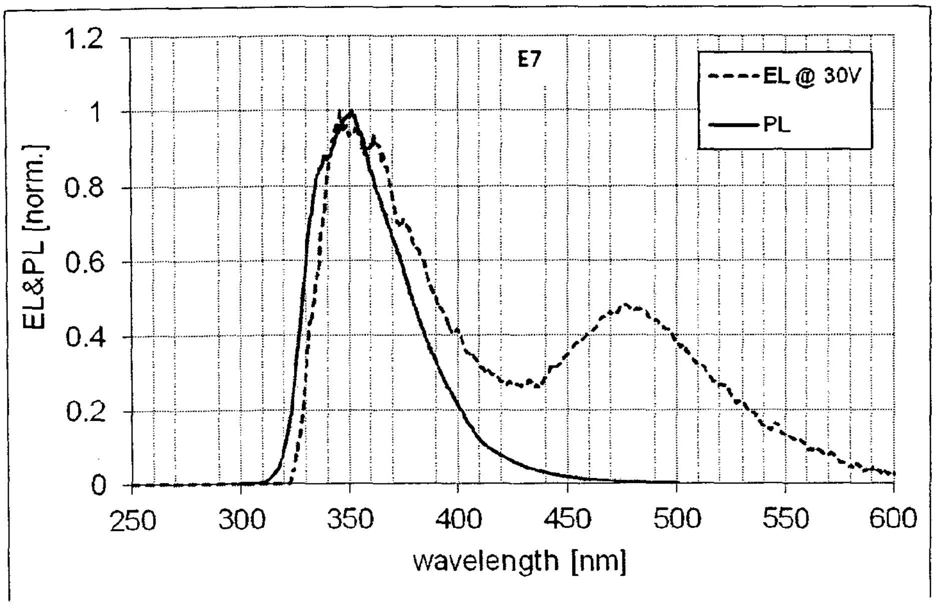


Abbildung 7

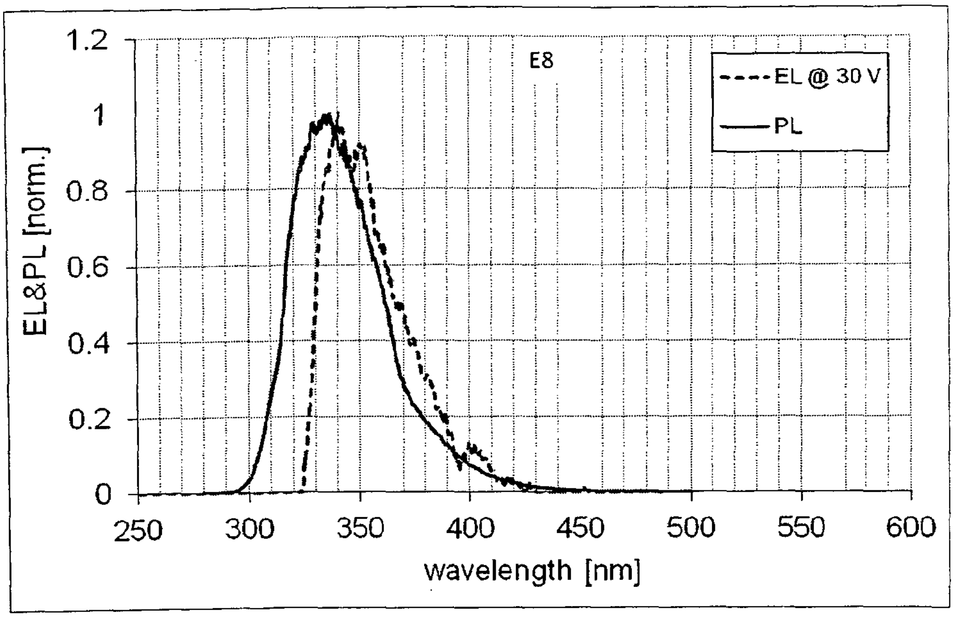


Abbildung 8

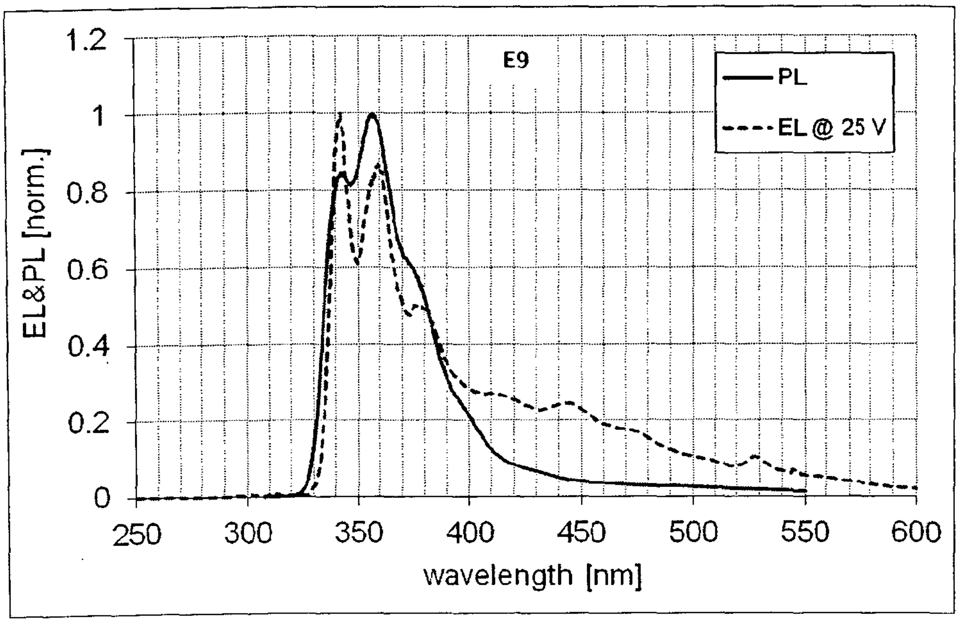


Abbildung 9

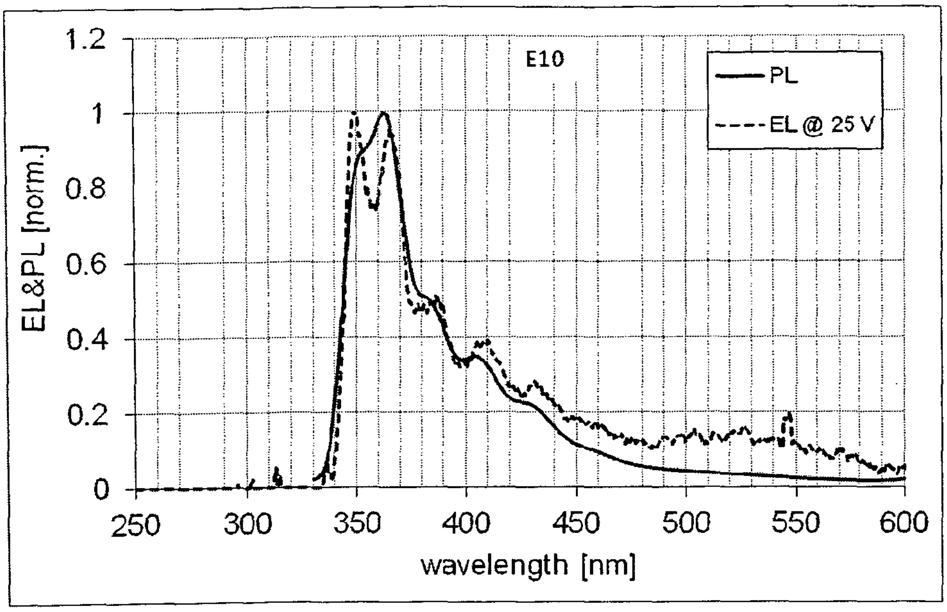


Abbildung 10

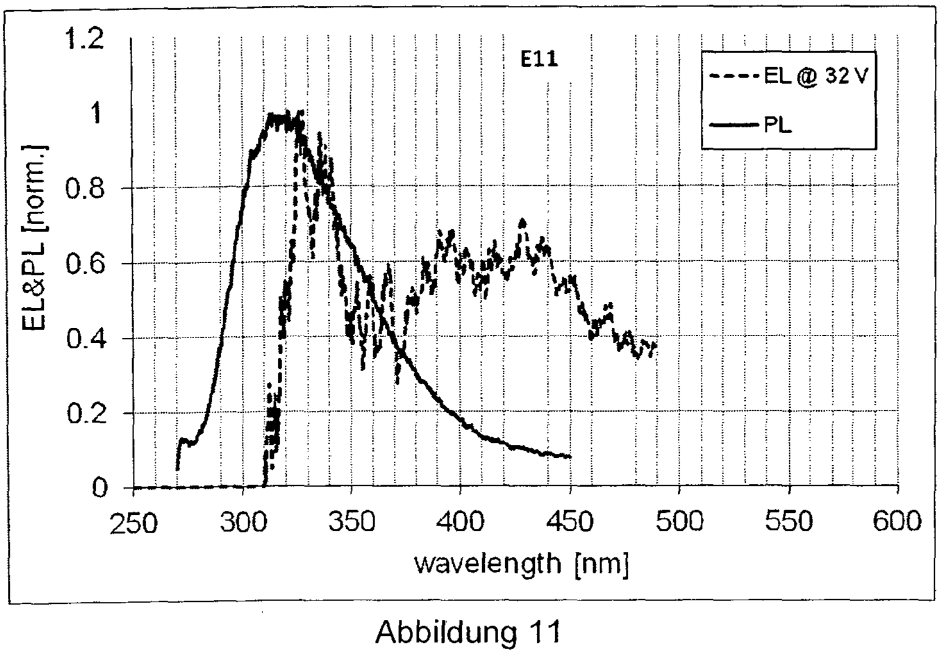


Abbildung 11

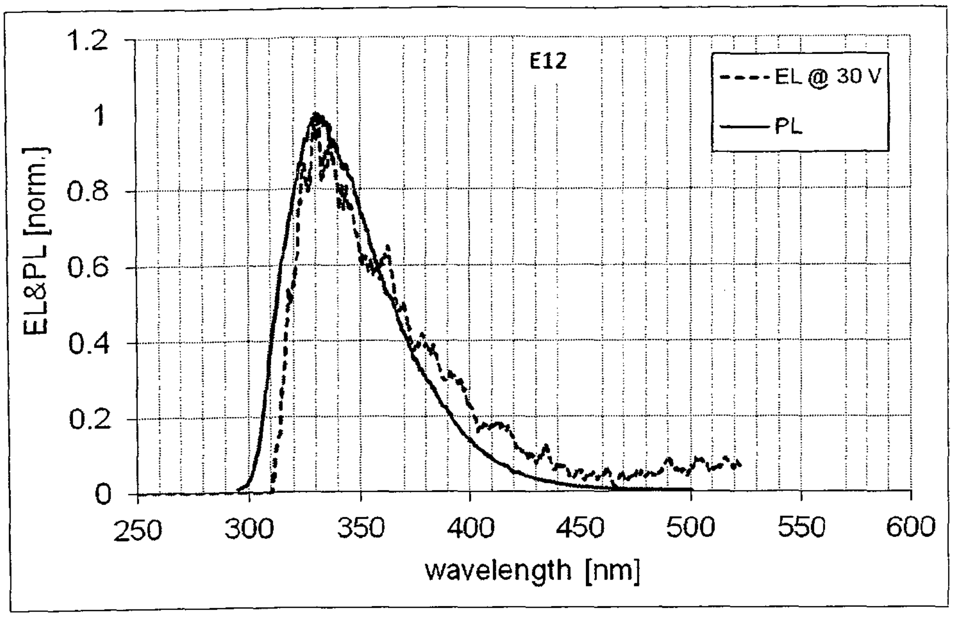


Abbildung 12

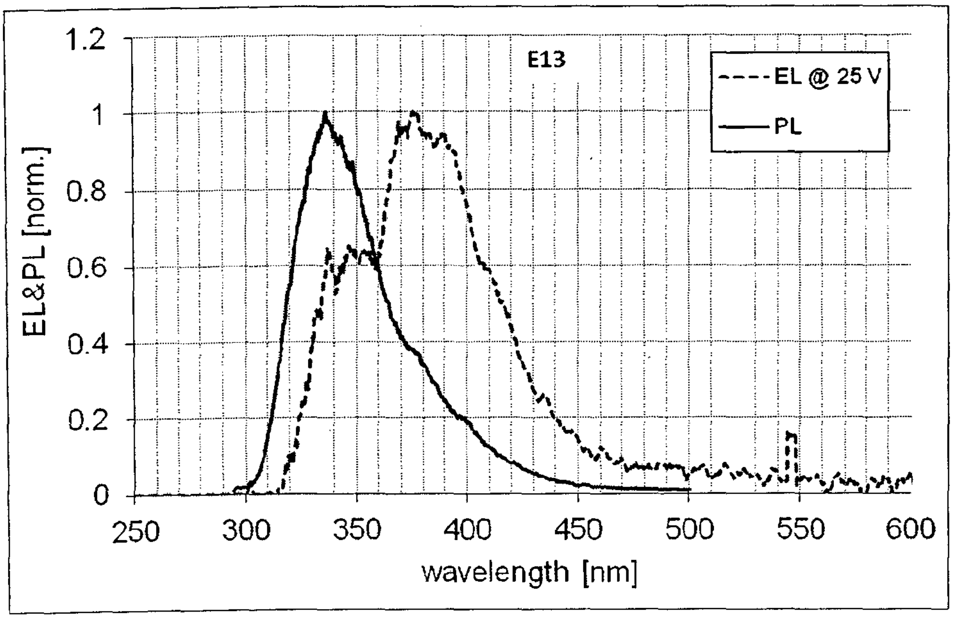


Abbildung 13

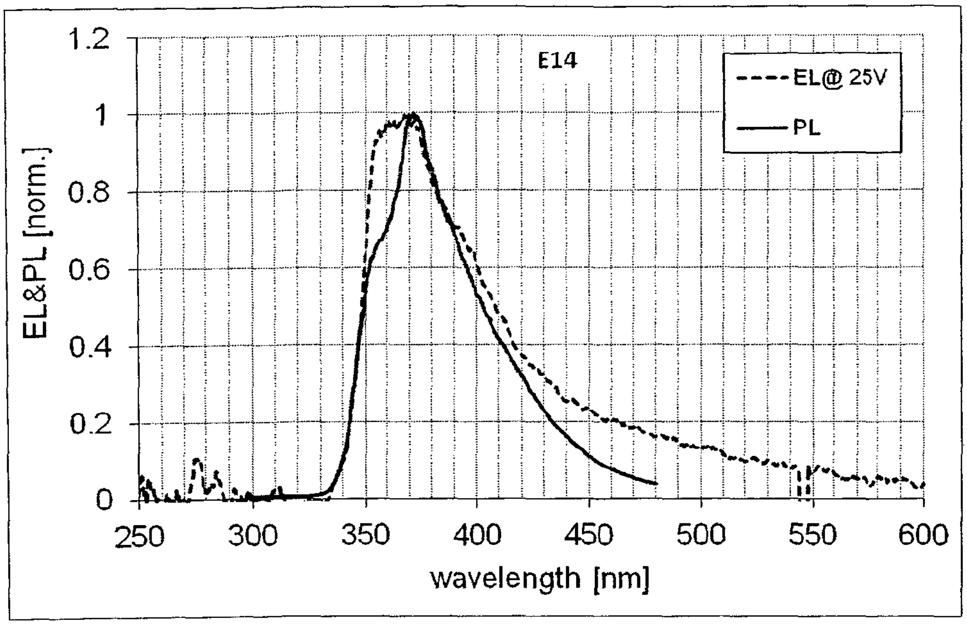


Abbildung 14

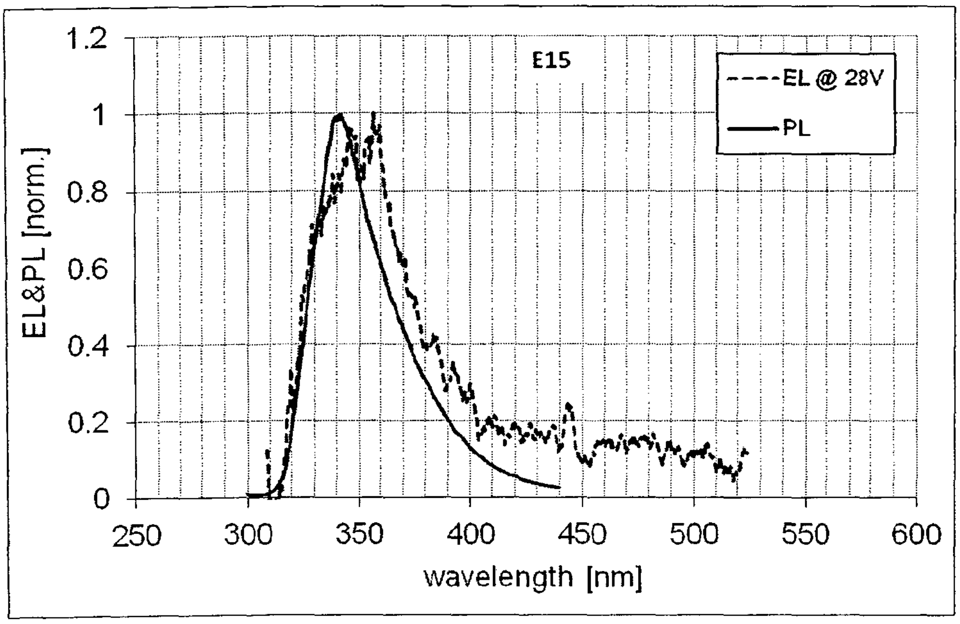


Abbildung 15

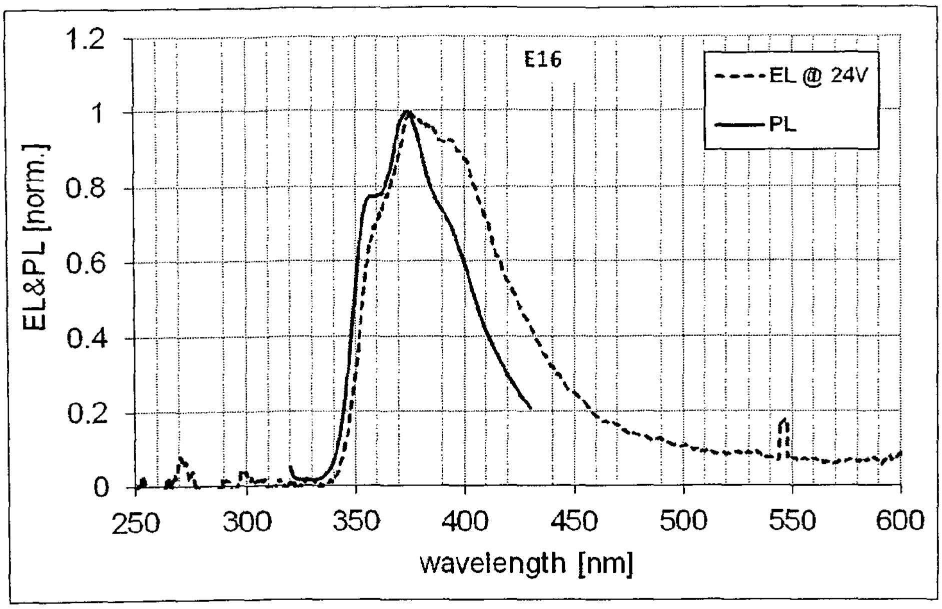


Abbildung 16

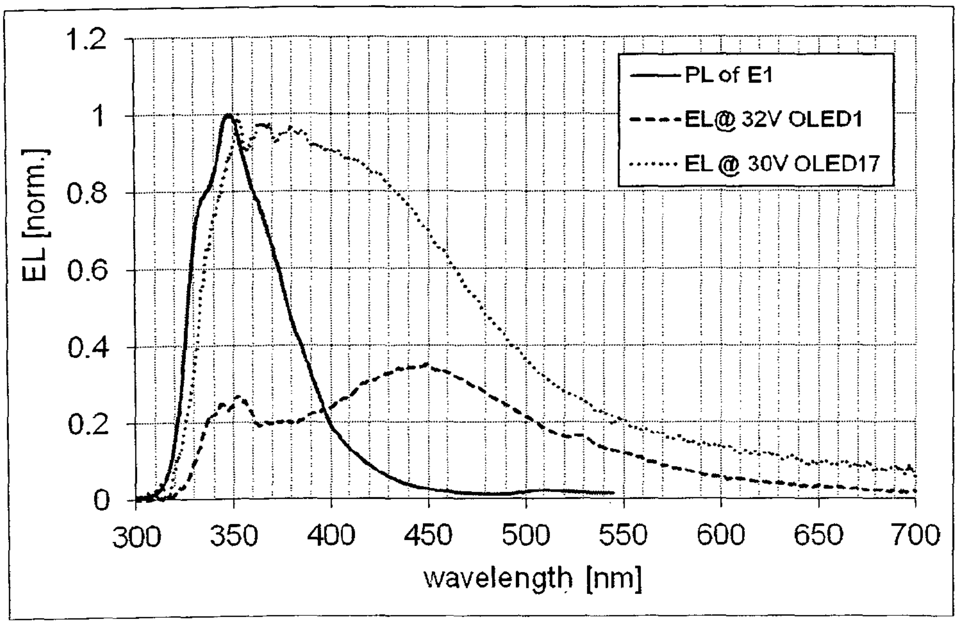


Abbildung 17

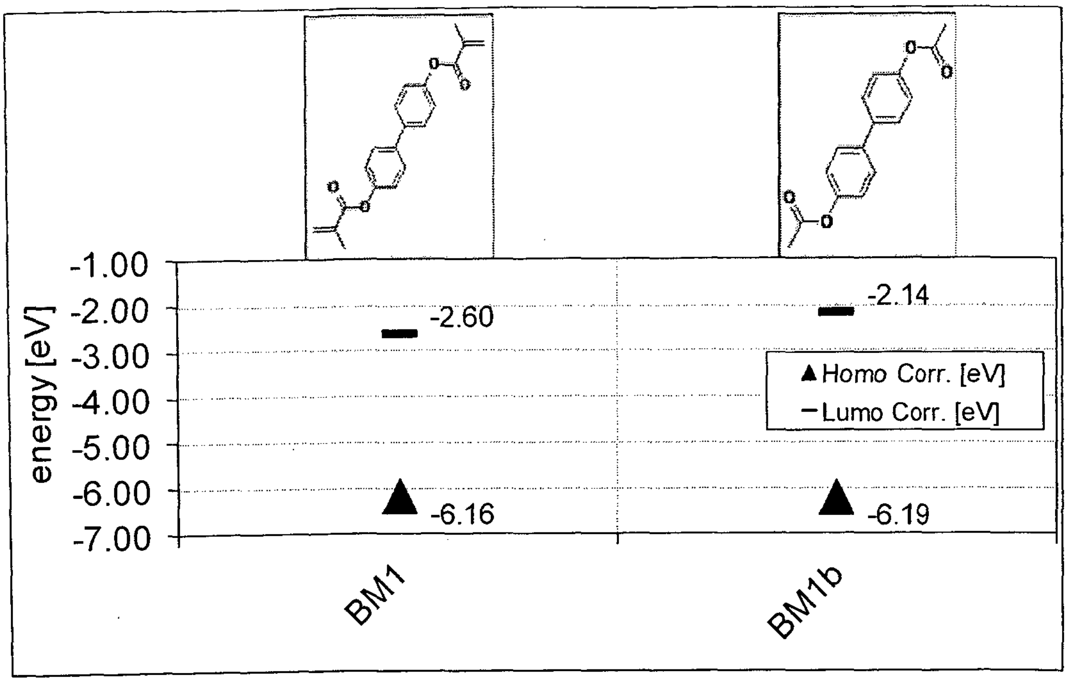


Abbildung 18

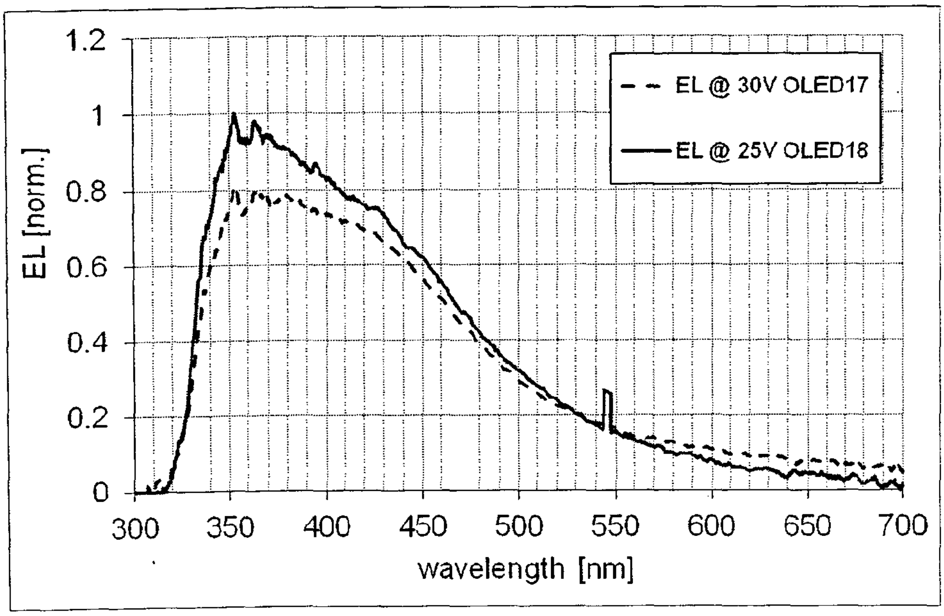


Abbildung 19

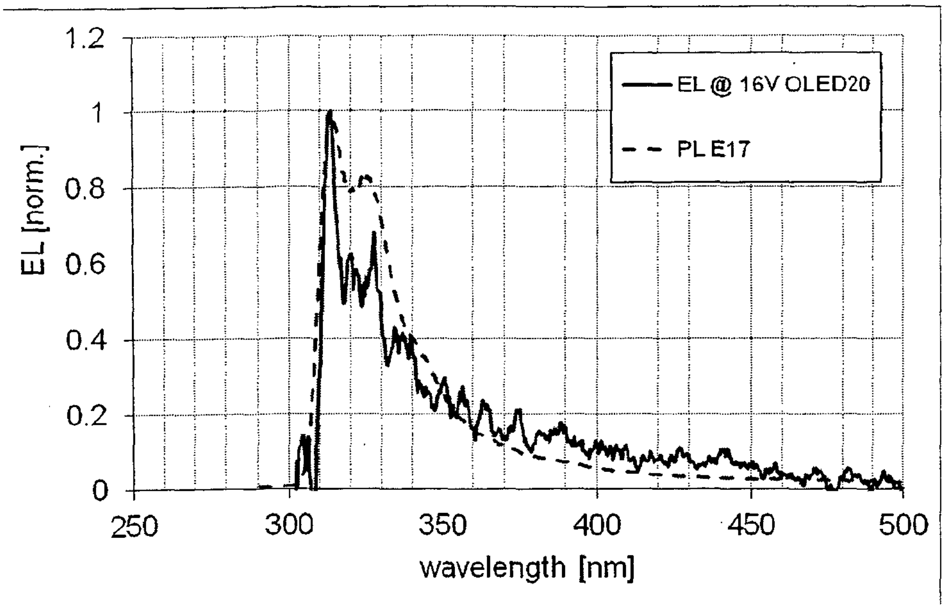


Abbildung 20

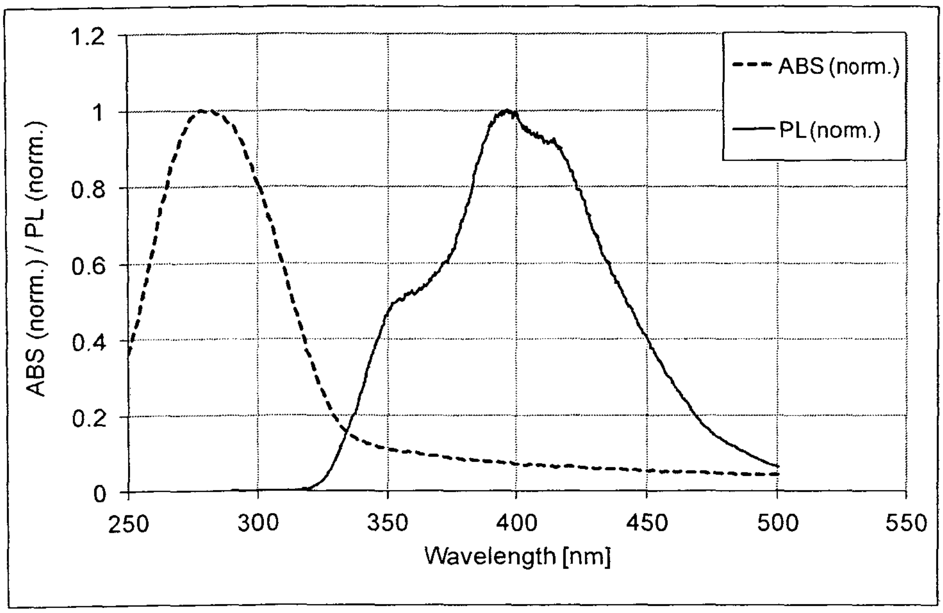


Abbildung 21

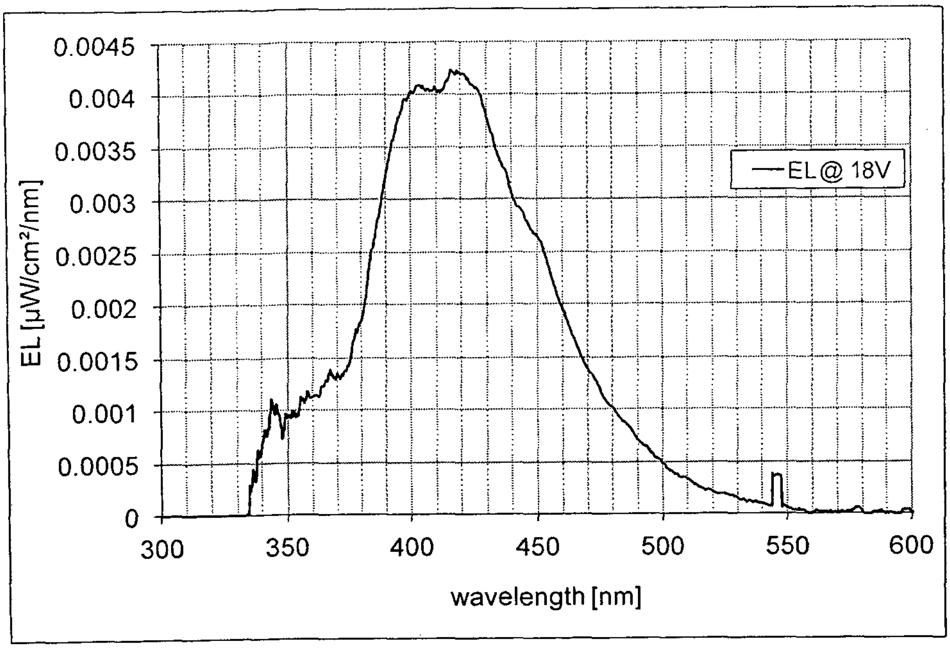


Abbildung 22

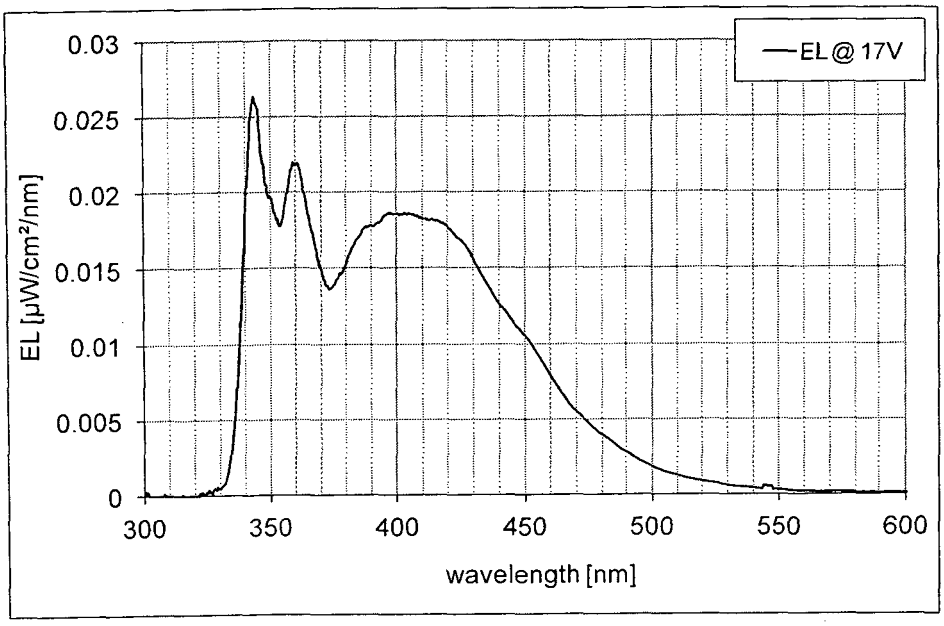


Abbildung 23

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- JP 403162484 B
- US 20100308313 A
- WO 2006043087 A
- WO 2010133278 A
- US 7807068 B
- WO 2011015265 A
- WO 2004041901 A
- WO 2004113412 A
- DE 19549741
- EP 1223209 A
- DE 10200040223914
- US 7297379 B
- EP 1223210 A
- EP 2033707 A
- EP 329752 A
- EP 4419932 B1
- WO 9103450 A1
- EP 1053578 A
- EP 440082 A
- DE 3878450
- EP 334911 A
- JP H06264059 A
- EP 441932 A
- DE 19927627


### In der Beschreibung aufgeführte Nicht-Patentliteratur


- **M. A. BALDO et al.** *Appl. Phys. Lett.,* 1999, vol. 75, 4-6
- **CHAO et al.** *Adv.Mater.,* 2005, vol. 17 (8), 992-996
- **WONG et al.** *Org.Lett.,* 2005, vol. 7 (23), 5131-5134
- **ZHOU et al.** *Macromolecules,* 2007, vol. 40 (9), 3015-3020
- **SHINAR et al.** *Applied Surface Science,* 2007, vol. 254 (3), 749-756
- **BURROWS.** *Applied Physics Letters,* 2006, vol. 88 (18), 183503
- **SHARMA et al.** *Applied Physics Letters,* 2006, vol. 88 (14), 143511-143513
- **BURROWS et al.** *Appl. Phys. Lett.,* 2006, vol. 88, 183503
- **VECCHI et al.** *Organic Letters,* 2006, vol. 8, 4211
- **ZHANG et al.** *J. Phys. Chem. B,* 2004, vol. 108, 9571
- **HOSHINO et al.** *J. Appl. Phys.,* 2000, vol. 88, 2892
- **ZOU et al.** *Appl. Phys. Lett.,* 2001, vol. 79, 2282
- **SHARMA et al.** *Appl. Phys. Lett.,* 2006, vol. 88, 143511
- **ZHOU et al.** *Macromolecules,* 2007, vol. 40, 3015
- **WONG et al.** *Organic Letters,* 2005, vol. 7, 5131
- **CHAO et al.** *Adv. Mater.,* 2005, vol. 17, 992
- **REN et al.** *Chem. Mater.,* 2004, vol. 16, 4743
- **PADMAPERUMA et al.** *Chem. Mater.,* 2006, vol. 18, 2389
- **ETORI et al.** *Jpn. J. Appl. Phys.,* 2007, vol. 46, 5071
- **SPILIOPOULOS et al.** *Macromolecules,* 2002, vol. 35, 7254
- **ICHIKAWA et al.** *Thin Solid Films,* 2007, vol. 515, 3932
- **NIU et al.** *RSC Adv.,* 2011, vol. 1, 415
- **SCHELER et al.** *In Macromol. Symp..,* 2007, vol. 254, 203-209
- **MUELLER et al.** *In Nature,* 2003, vol. 421, 829-833
- **M. S. ARNOLD et al.** *Appl. Phys. Lett.,* 2008, vol. 92, 053301
- **SETAYESH et al.** *Macromolecules,* 2000, vol. 33, 2016
- **WESSLAU.** *Makromolekulare Chemie,* 1966, vol. 93, 55
- **YOUN et al.** *J. Electrochem. Soc.,* 2011, vol. 158, J321